(19) Europäisches Patentamt
European Patent Office
Office européen des brevets

(11) Publication number: **0 496 608 B1**

# (12) EUROPEAN PATENT SPECIFICATION

(45) Date of publication of patent specification: **20.09.95**

(51) Int. Cl.[6]: **C07D 401/12**, A01N 47/36, C07D 213/76

(21) Application number: **92300564.9**

(22) Date of filing: **23.01.92**

The file contains technical information submitted after the application was filed and not included in this specification

## (54) Substituted pyridinesulfonamide compound or its salt, process for preparing the same, and herbicide containing the same.

(30) Priority: **24.01.91 JP 85718/91**
**12.07.91 JP 265553/91**

(43) Date of publication of application:
**29.07.92 Bulletin 92/31**

(45) Publication of the grant of the patent:
**20.09.95 Bulletin 95/38**

(84) Designated Contracting States:
**AT BE CH DE DK ES FR GB GR IT LI LU MC NL PT SE**

(56) References cited:
**EP-A- 0 116 518** **EP-A- 0 314 505**
**EP-A- 0 317 336** **EP-A- 0 402 316**
**EP-A- 0 451 468** **DE-A- 4 000 503**

(73) Proprietor: **ISHIHARA SANGYO KAISHA, Ltd.**
**3-22, Edobori 1-chome**
**Nishi-ku**
**Osaka-shi**
**Osaka-fu (JP)**

(72) Inventor: **Sakashita, Nobuyuki, c/o Ishihara Sangyo K. Ltd.**
**Central Laboratory,**
**3-1, Nishishibukawa 2-chome**
**Kusatsu-shi,**
**Shiga (JP)**
Inventor: **Nakajima, Toshio, ISK Mountain View Res. Cent. Inc**
**1195 West Fremont Avenue**
**Sunnyvale**
**CA 94087 (us)**
Inventor: **Murai, Shigeo, c/o Ishihara Sangyo K. Ltd.**
**Central Laboratory,**
**3-1, Nishishibukawa 2-chome**
**Kusatsu-shi,**
**Shiga (JP)**
Inventor: **Yoshida, Tsunezo, c/o Ishihara Sangyo K. Ltd.**
**Central Laboratory,**
**3-1, Nishishibukawa 2-chome**
**Kusatsu-shi,**
**Shiga (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 496 608 B1

Inventor: **Nakamura, Yugi, c/o Ishihara Sangyo K. Ltd.**
**Central Laboratory,**
**3-1, Nishishibukawa 2-chome**
**Kusatsu-shi,**
**Shiga (JP)**
Inventor: **Honzawa, Shooichi c/o Ishihara Sangyo K. Ltd.**
**Central Laboratory,**
**3-1, Nishishibukawa 2-chome**
**Kusatsu-shi,**
**Shiga (JP)**

(74) Representative: **Pearce, Anthony Richmond**
**MARKS & CLERK,**
**Alpha Tower,**
**Suffolk Street Queensway**
**Birmingham B1 1TT (GB)**

## Description

### FIELD OF THE INVENTION

The present invention relates to a novel substituted pyridinesulfonamide compound or its salt, a process for preparing the same, and a herbicide containing the same.

### BACKGROUND OF THE INVENTION

U.S. Patent No. 4,946,494 (EP-A-0314505) discloses a pyridinesulfonylurea derivative useful as an effective ingredient of a herbicidal composition, which is, however, different in the substituent at the 6-position of the pyridine ring in terms of chemical structure from the compound of the present invention.

EP-A-0116518 discloses herbicidal N-phenylsulfonyl-N'-pyrimidinyl and triazinyl compounds where the phenyl group may be substituted by a wide variety of substituents including alkylsulfonyl, but there is no suggestion to utilise a substituted sulphonylamino group as a substituent.

EP-A-0317336 discloses herbicidal N-pyridinylsulfonyl-N'-pyrimidinyl and triazinyl compounds where the pyridine ring is substituted by 3-methylcarbonyl, 3-ethylcarbonyl or 3-isopropylcarbonyl.

EP-A-0402316 also discloses herbicidal N-pyridinylsulfonyl-N'-pyrimidinyl and triazinyl compounds where the pyridine ring is substituted at the 3-position by a substituted sulfonyl group and also optionally further substituted by halogen, $C_1$-$C_4$ alkyl, $C_1$-$C_4$ alkylthio, $C_1$-$C_4$ haloalkoxy or $CF_3$.

### SUMMARY OF THE INVENTION

In accordance with one aspect of the present invention, there is provided a substituted pyridinesulfonamide compound or its salt represented by the following general formula (I):

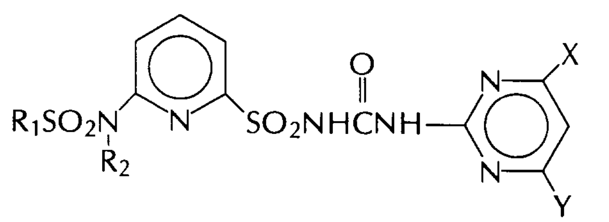

(I)

wherein $R_1$ and $R_2$ may be either each independently a member selected from the group consisting of $C_1$-$C_6$ alkyl optionally substituted by one or more substituents selected from the group consisting of halogen and $C_1$-$C_6$ alkoxy; $C_2$-$C_6$ alkenyl optionally substituted by one or more substituents selected from the group consisting of halogen and $C_1$-$C_6$ alkoxy; $C_3$-$C_6$ cycloalkyl optionally substituted by one or more substituents selected from the group consisting of halogen, $C_1$-$C_6$ alkyl and $C_1$-$C_6$ alkoxy; and phenyl optionally substituted by one or more substituents selected from the group consisting of halogen, $C_1$-$C_6$ alkyl, $C_1$-$C_6$ haloalkyl and a nitro group; or $R_1$ and $R_2$ are optionally combined with each other to form a $-(CH_2)_n-$ group wherein n is an integer of 2 to 5; and X and Y are each independently a member selected from the group consisting of $C_1$-$C_6$ alkyl and $C_1$-$C_6$ alkoxy.

In accordance with another aspect of the present invention, there is provided a process for preparing a substituted pyridinesulfonamide compound or its salt represented by the following general formula (I):

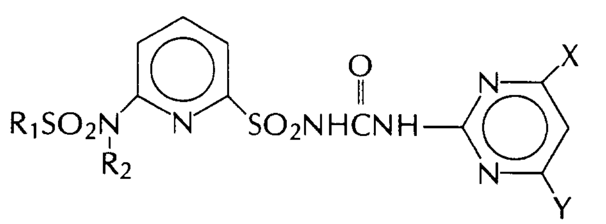

(I)

wherein $R_1$ and $R_2$ may be either each independently a member selected from the group consisting of $C_1$-$C_6$ alkyl optionally substituted by one or more substituents selected from the group consisting of halogen and $C_1$-$C_6$ alkoxy; $C_2$-$C_6$ alkenyl optionally substituted by one or more substituents selected from the group consisting of halogen and $C_1$-$C_6$ alkoxy; $C_3$-$C_6$ cycloalkyl optionally substituted by one or more substituents selected from the group consisting of halogen, $C_1$-$C_6$ alkyl and $C_1$-$C_6$ alkoxy; and phenyl optionally substituted by one or more substituents selected from the group consisting of halogen, $C_1$-$C_6$ alkyl, $C_1$-$C_6$ haloalkyl and a nitro group; or $R_1$ and $R_2$ are optionally combined with each other to form a $-(CH_2)_n-$ group wherein n is an integer of 2 to 5; and X and Y are each independently a member selected from the group consisting of $C_1$-$C_6$ alkyl and $C_1$-$C_6$ alkoxy,
which comprises reacting a substituted pyridine compound represented by the following general formula (II):

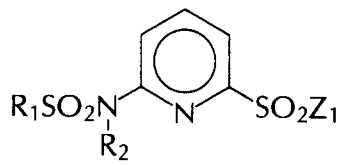

(II)

wherein $R_1$ and $R_2$ are the same as defined above; and $Z_1$ is a member selected from the group consisting of an $-NH_2$ group, an -NCO group, and an $-NHCO_2R_3$ group wherein $R_3$ is an alkyl or aryl group; with a pyrimidine compound represented by the following general formula (III):

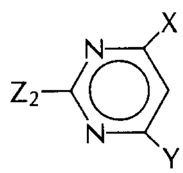

(III)

wherein X and Y are the same as defined above; and $Z_2$ is an $-NH_2$ group when $Z_1$ is an -NCO group or an $-NHCO_2R_3$ group, and is a member selected from the group consisting of an -NCO group and an $-NHCO_2R_3$ group wherein $R_3$ is the same as defined above, when $Z_1$ is an $-NH_2$ group.

In accordance with still another aspect of the present invention, there is provided a herbicide containing as the effective ingredient a substituted pyridinesulfonamide compound or its salt represented by the following general formula (I):

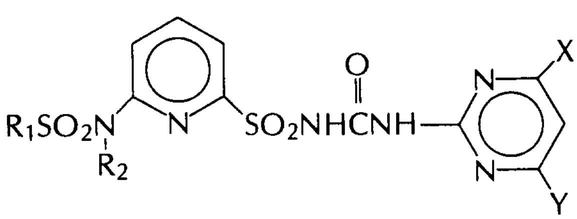

(I)

wherein $R_1$ and $R_2$ may be either each independently a member selected from the group consisting of $C_1$-$C_6$ alkyl optionally substituted by one or more substituents selected from the group consisting of halogen and $C_1$-$C_6$ alkoxy; $C_2$-$C_6$ alkenyl optionally substituted by one or more substituents selected from the group consisting of halogen and $C_1$-$C_6$; $C_3$-$C_6$ cycloalkyl optionally substituted by one or more substituents selected from the group consisting of halogen, $C_1$-$C_6$ alkyl and $C_1$-$C_6$alkoxy; and phenyl optionally substituted by one or more substituents selected from the group consisting of halogen, $C_1$-$C_6$ alkyl, $C_1$-$C_6$ haloalkyl and a nitro group; or $R_1$ and $R_2$ are optionally combined with each other to form a $-(CH_2)_n-$ group wherein n is an integer of 2 to 5; and X and Y are each independently a member selected from the group consisting of $C_1$-$C_6$ alkyl and $C_1$-$C_6$ alkoxy.

In accordance with a further aspect of the present invention, there is provided a substituted pyridine compound represented by the following general formula (II-1):

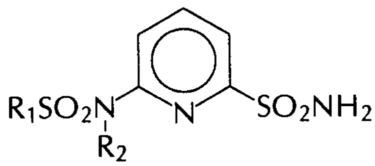

(II-1)

wherein $R_1$ and $R_2$ are either each independently a member selected from the group consisting of $C_1$-$C_6$ alkyl optionally substituted by one or more substituents selected from the group consisting of halogen and $C_1$-$C_6$ alkoxy; $C_2$-$C_6$ alkenyl optionally substituted; $C_3$-$C_6$ cycloalkyl optionally substituted by one or more substituents selected from the group consisting of halogen, $C_1$-$C_6$ alkyl and $C_1$-$C_6$ alkoxy; and phenyl optionally substituted by one or more substituents selected from the group consisting of halogen, $C_1$-$C_6$ alkyl, $C_1$-$C_6$ haloalkyl and a nitro group; or $R_1$ and $R_2$ are optionally combined with each other to form a -$(CH_2)_n$- group wherein n is an integer of 2 to 5.

## DETAILED DESCRIPTION OF THE INVENTION

The present invention will now be described in detail.

In the denotations of $R_1$ and $R_2$ in the general formula (I), the number of substituent(s) contained in such a substituted group may be either one, or two or more, in which case the substituents may be the same or different from each other. The same applies to a substituent(s) if further contained in such a substituent as mentioned above that can be contained in such a substituted group that can be denoted by $R_1$ and $R_2$.

In the general formula (I), examples of the $C_1$-$C_6$ alkyl group as well as alkyl moieties that may be included in the denotations of $R_1$, $R_2$, X and Y include methyl, ethyl, propyl, butyl, pentyl and hexyl groups that may each be linear or branched in terms of structural isomerism of the aliphatic chain. Examples of $C_2$-$C_6$ alkenyl groups that may be included in the denotations of $R_1$ and $R_2$ include vinyl, propenyl, butenyl, pentenyl and hexenyl groups that may each be linear or branched in terms of structural isomerism of the aliphatic chain. Examples of $C_3$-$C_6$ cycloalkyl groups that may be included in the denotations of $R_1$ and $R_2$ include cyclopropyl, cyclobutyl, cyclopentyl, and cyclohexyl groups. Halogen atoms that may be included in the denotations of $R_1$ and $R_2$ include fluorine, chlorine, bromine and iodine atoms.

Examples of the salt of the substituted pyridinesulfonamide compound represented by the general formula (I) include those of alkali metals such as sodium and potassium, those of alkaline earth metals such as magnesium and calcium, and those of amines such as dimethylamine and triethylamine.

Among the compounds of the formula (I), preferred are those wherein $R_1$ and $R_2$ are each independently a member selected from the group consisting of $C_1$-$C_6$ alkyl optionally substituted by one or more substituents selected from the group consisting of halogen and $C_1$-$C_6$ alkoxy; and $C_3$-$C_6$ cycloalkyl optionally substituted by one or more substituents selected from the group consisting of halogen, $C_1$-$C_6$ alkyl and $C_1$-$C_6$ alkoxy; more preferred are those wherein $R_1$ and $R_2$ are each independently a member selected from the group consisting of $C_1$-$C_6$ alkyl groups, $C_1$-$C_6$ haloalkyl groups, and $C_3$-$C_6$ cycloalkyl groups; and most preferred are 6-[(N-ethyl-N-methylsulfonyl)amino]-N-[[(4,6-dimethoxypyrimidin-2-yl)-amino]carbonyl]-2-pyridinesulfonamide (Compound No. 2 as described hereinafter), 6-[(N-ethyl-N-ethylsulfonyl)amino]-N-[[(4,6-dimethoxypyrimidin-2-yl)amino] carbonyl]-2-pyridinesulfonamide (Compound No. 18 as described hereinafter) and 6-[(N-ethyl-N-isopropylsulfonyl)amino]-N-[[(4,6-dimethoxypyrimidin-2-yl)amino]-carbonyl]-2-pyridinesulfonamide(Compound No. 22 as described hereinafter).

The substituted pyridinesulfonamide compound represented by the general formula (I) can be prepared, for example, according to the process of the present invention, embodiments of which may be represented by the following reaction formulae [A] to [D]:

Reaction Formula [A]

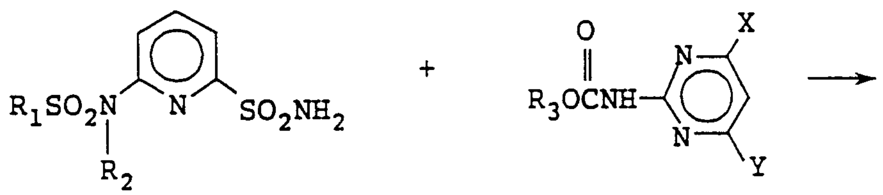

(I)

(II-1) (III-1)

Reaction Formula [B]

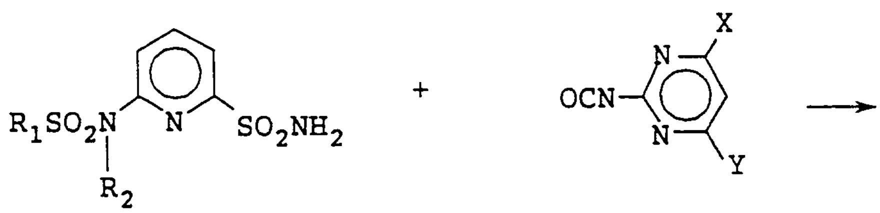

(I)

(II-1) (III-2)

Reaction Formula [C]

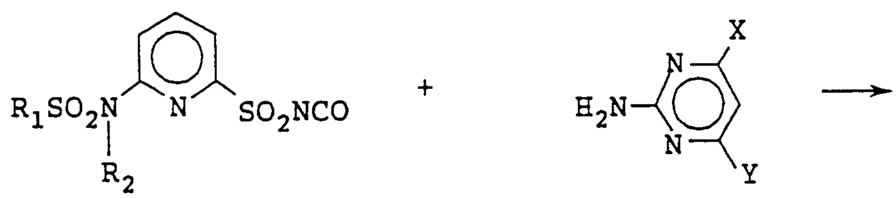

(I)

(II-2) (III-3)

Reaction Formula [D]

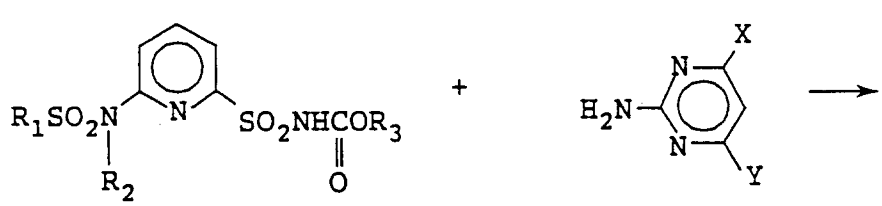

(I)

(II-3) (III-3)

In the reaction formulae [A] to [D], $R_1$, $R_2$, X and Y are the same as defined above, and $R_3$ is an alkyl group or an aryl group.

The alkyl group included in the denotation of $R_3$ may be any alkyl group as defined above in connection with the denotations of $R_1$, $R_2$, X and Y. As the aryl group included in the denotation of $R_3$, there can be mentioned a phenyl group, a phenyl group substituted with at least one chlorine atom, a phenyl group substituted with at least one methyl group, a naphthyl group, etc.

The reaction [A] is effected in the presence of a base, while the reactions [B], [C] and [D] may be effected in the presence of a base if desired. Examples of such a base include tertiary amines such as triethylamine, and 1,8-diazabicyclo[5.4.0]-7-undecene.

The reactions [A], [B], [C] and [D] may be effected in the presence of a solvent if necessary. Examples of the solvent include unsubstituted or substituted, aromatic hydrocarbons such as benzene, toluene, xylene, and chlorobenzene; unsubstituted or substituted, cyclic or acyclic, aliphatic hydrocarbons such as chloroform, carbon tetrachloride, methylene chloride, dichloroethane, trichloroethane, hexane, and cyclohexane; ethers such as diethyl ether, dioxane, and tetrahydrofuran; nitriles such as acetonitrile, propionitrile, and acrylonitrile; esters such as methyl acetate and ethyl acetate; aprotic polar solvents such as dimethyl sulfoxide and sulfolane; etc.

The reaction temperature of the reaction [A] is usually in the range of -20 to +100 °C, preferably in the range of 0 to 40 °C, while the reaction time of the reaction [A] is usually in the range of 0.01 to 24 hours, preferably in the range of 0.1 to 1.5 hours. The reaction temperature of the reaction [B] is usually in the range of 0 to 150 °C, while the reaction time of the reaction [B] is usually in the range of 0.1 to 24 hours. The reaction temperature of the reaction [C] is usually in the range of 0 to 150 °C, while the reaction time of the reaction [C] is usually in the range of 0.1 to 24 hours. The reaction temperature of the reaction [D] is usually in the range of -20 to +150 °C, while the reaction time of the reaction [D] is usually in the range of 0.1 to 24 hours.

A starting material compound represented by the general formula (II-1) in the reaction formulae [A] and [B] according to the present invention can be synthesized, for example, according to one of reaction formulae [E], [F] and [G]:

Reaction Formula [E]

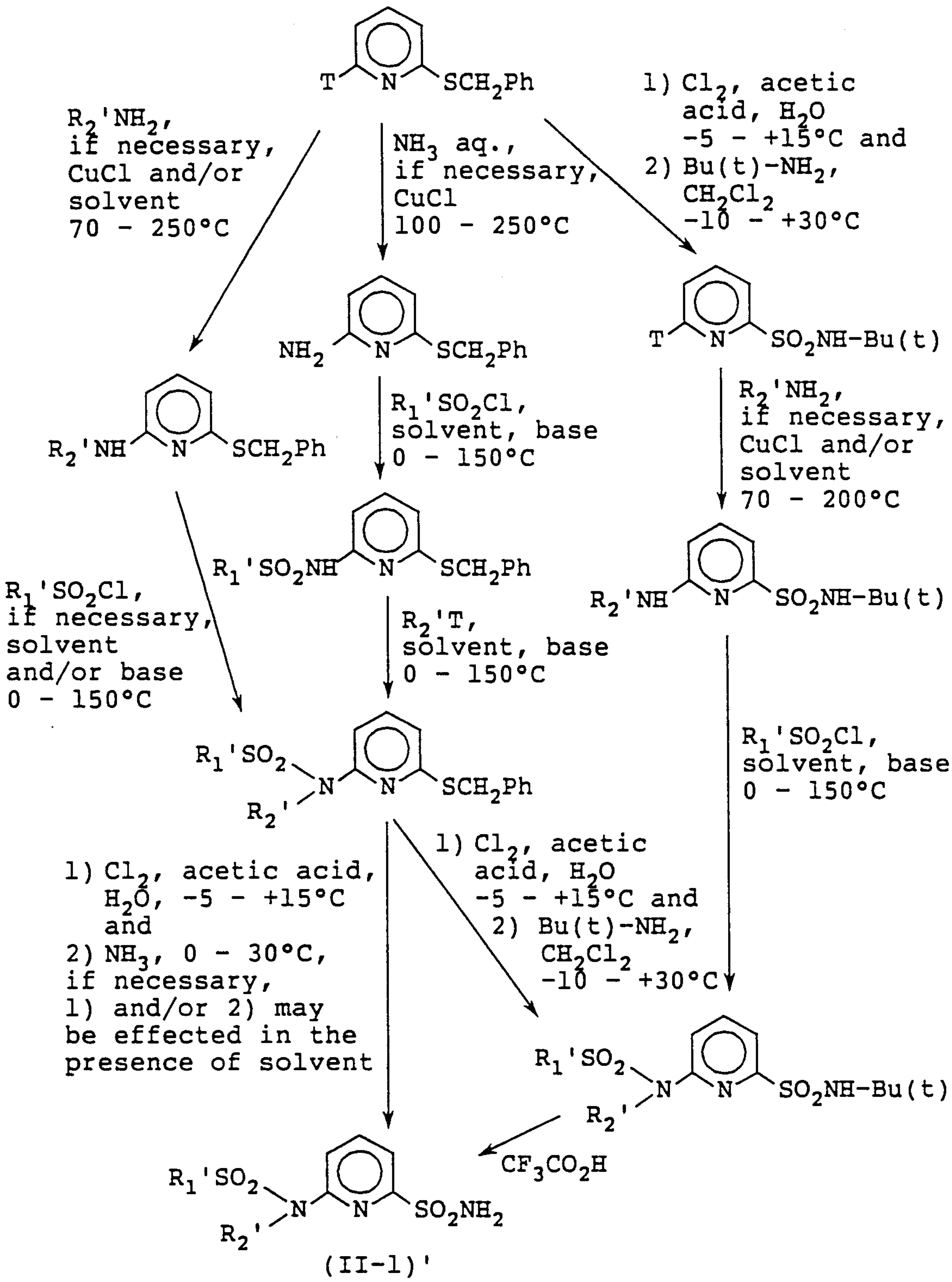


(II-1)'

Reaction Formula [F]

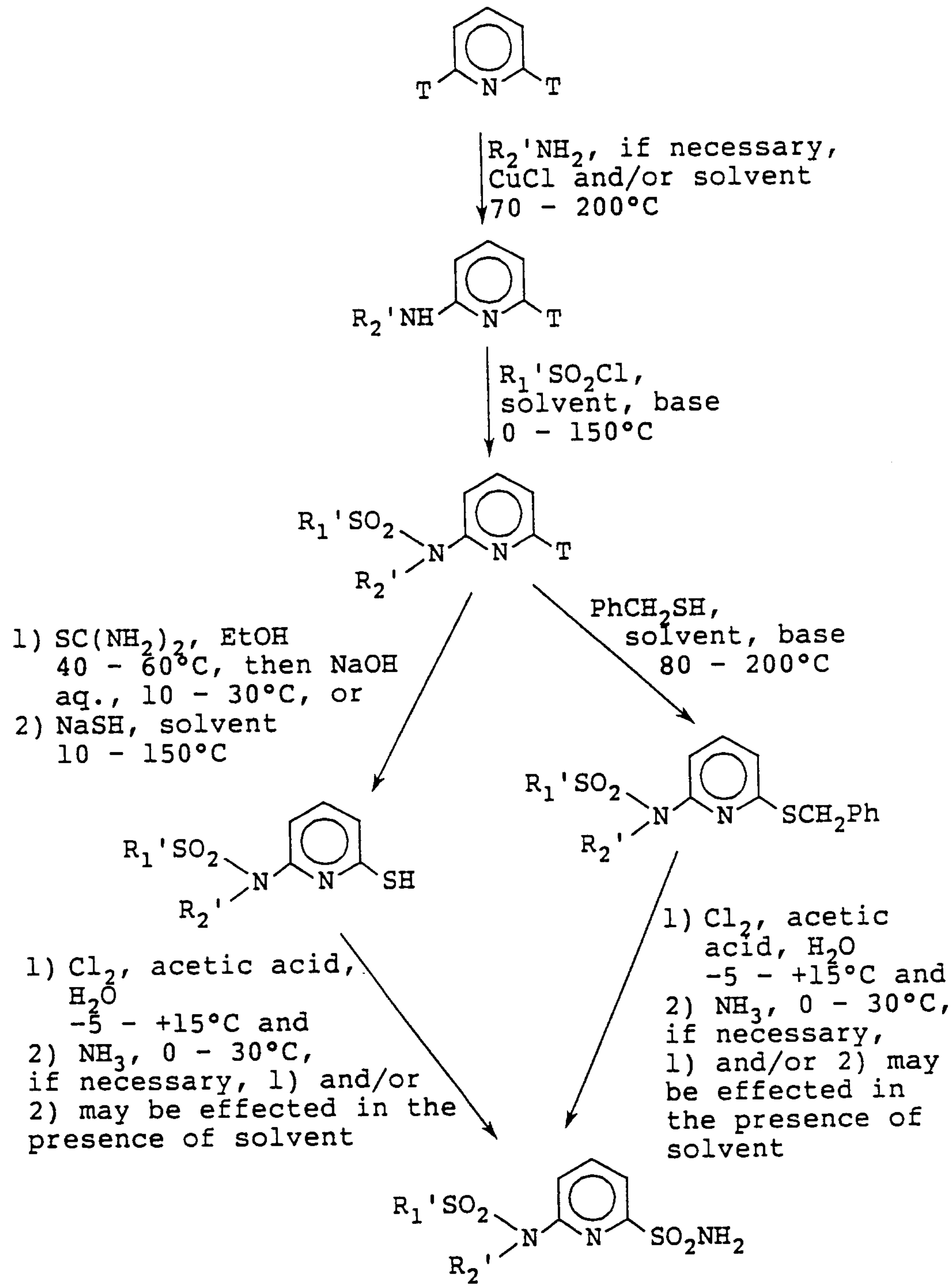


(II-1)'

Reaction Formula [G]

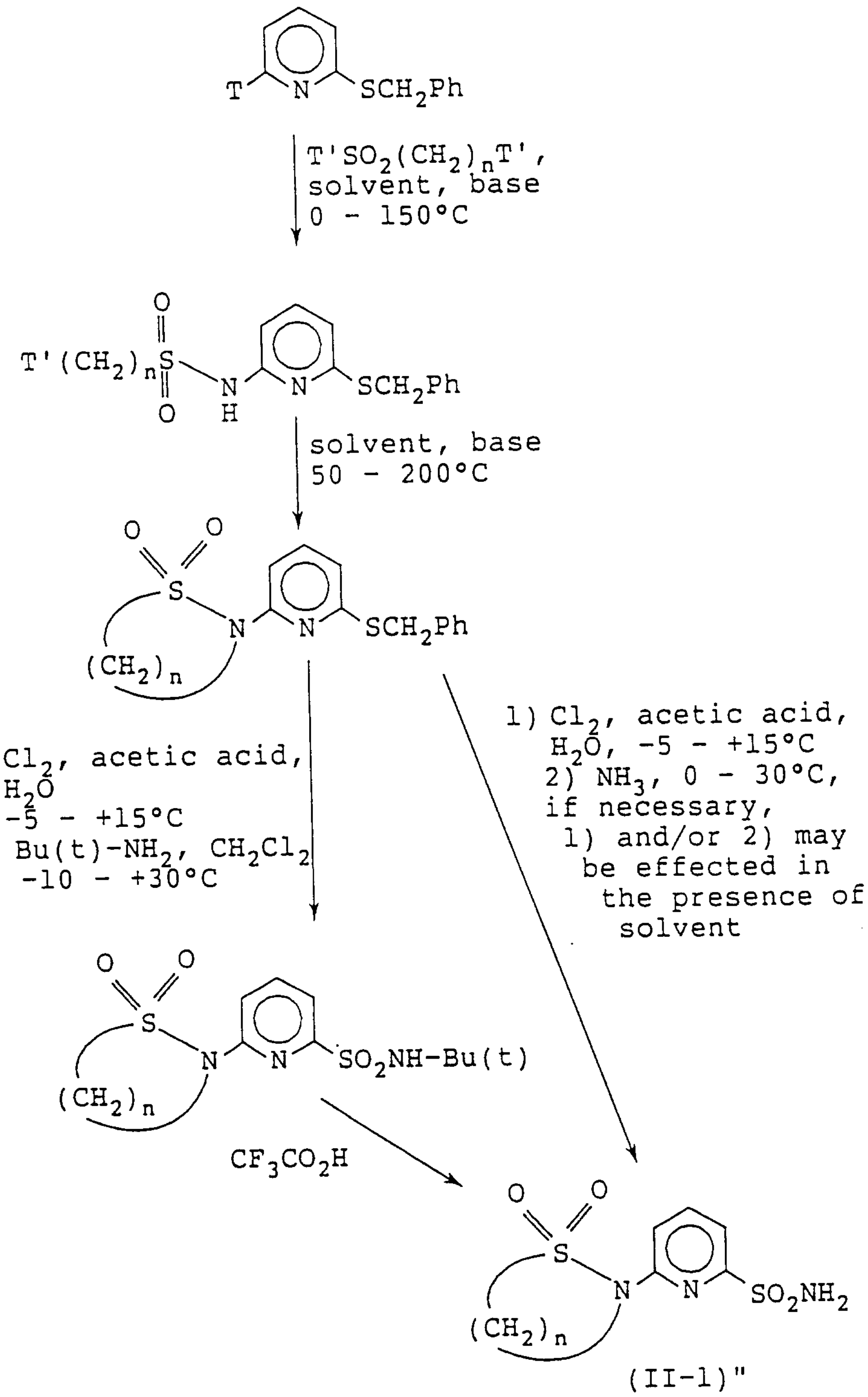


(II-1)"

In the reaction formulae [E], [F] and [G], $R_1$' and $R_2$' are each independently a member selected from the group consisting of unsubstituted or substituted alkyl groups, unsubstituted or substituted alkenyl groups, unsubstituted or substituted cycloalkyl groups, and unsubstituted or substituted phenyl groups; n is the same as defined above in connection with the general formula [I]; T and T' are each independently a member selected from the group consisting of a chlorine atom, a bromine atom, and an iodine atom; Ph stands for a phenyl group; Et stands for an ethyl group; Bu(t) stands for a tertiary butyl group; and aq. stands for an aqueous solution.

The substituted alkyl, alkenyl, cycloalkyl and phenyl groups included in the denotations of $R_1'$ and $R_2'$ are the same as defined above in connection with the denotations of $R_1$ and $R_2$.

A starting material compound represented by the formula (II-2) in the reaction formula [C] can be prepared, for example, according to the following reaction formula [H]:

Reaction Formula [H]

(1) $CH_3(CH_2)_3NCO$, $K_2CO_3$, $CH_3COCH_3$

(II-1) → (II-2)

(2) $COCl_2$, xylene

A starting material compound represented by the formula (II-3) in the reaction formula [D] can be prepared, for example, according to the following reaction formula [I]:

Reaction Formula [I]

$ClCO_2R_3$, NaH, tetrahydrofuran

(II-1) → (II-3)

$R_1$, $R_2$ and $R_3$ in the reaction formulae [H] and [I] are the same as defined hereinbefore.

The reaction conditions of the reactions [E] to [I], which involve the reaction temperature, the period of reaction time, the use or disuse as well as kind and amount of solvent (to be used if desired), and the kind and amount of base, can usually be appropriately chosen from the reaction conditions of similar reactions unless otherwise mentioned.

The salt of the aforementioned substituted pyridinesulfonamide compound can be easily prepared according to a usual method.

The following Examples will specifically illustrate the present invention in more detail, but should not be construed as limiting the scope of the invention.

## Synthesis Example 1

Synthesis of 6-[(N-ethyl-N-methylsulfonyl)amino]-N-[[(4,6-dimethoxypyrimidin-2-yl)amino]carbonyl]-2-pyridinesulfonamide (Compound No. 2 in Table 2 which will be given later)

1) 13 g of 2-benzylthio-6-bromopyridine, 80 mℓ of aqueous ammonia (about 40 wt.%), and a catalytic amount of cuprous chloride were mixed together and reacted in an autoclave at 120 °C for 4 hours.

After completion of the reaction, the reaction mixture was poured into water and subjected to extraction with methylene chloride. The extract was washed with water, dried over anhydrous sodium sulfate, and concentrated under reduced pressure. The resulting residue was purified through silica gel column chromatography with a developing solvent (ethyl acetate : hexane = 1:3) to obtain 5.7 g of 2-amino-6-benzylthiopyridine having a melting point of 65 to 66 °C.

2) 5.75 g of 2-amino-6-benzylthiopyridine and 86 mℓ of tetrahydrofuran was mixed together and further admixed with 1.67 g of powdery potassium hydroxide, followed by agitation thereof at room temperature. The resulting mixture was cooled with ice. 6.10 g of methanesulfonyl chloride was added dropwise to the ice-cooled mixture, followed by reaction under agitation at room temperature over one night.

After completion of the reaction, the reaction mixture was poured into water and subjected to extraction with methylene chloride. The exctract was dried over anhydrous sodium sulfate and concentrated under reduced pressure. The resulting residue was purified through silica gel column chromatography with a developing solvent (ethyl acetate : hexane = 1:1) to obtain 1.5 g of N-(6-benzylthiopyridin-2-yl)methanesulfonamide having a melting point of 123 to 125 °C.

3) 1.0 g of N-(6-benzylthiopyridin-2-yl)methanesulfonamide obtained in the above step 2) was mixed with 15 mℓ of tetrahydrofuran. The resulting mixture was cooled with ice and admixed with 0.15 g of 60 wt% sodium hydride, followed by agitation thereof at room temperature. Thereafter, 3.56 g of ethyl iodide was

added to the mixture. The resulting mixture was heated up and reacted under reflux over one night.

After completion of the reaction, the reaction mixture was poured into water, weakly acidified with hydrochloric acid, and subjected to extraction with methylene chloride. The extract was dried over anhydrous sodium sulfate and concentrated under reduced pressure. The resulting residue was purified through silica gel column chromatography with a developing solvent (ethyl acetate : hexane = 1:1) to obtain 0.8 g of white crystals of N-(6-benzylthiopyridin-2-yl)-N-ethylmethanesulfonamide.

4) 0.8 g of N-(6-benzylthiopyridin-2-yl)-N-ethylmethanesulfonamide obtained in the above step 3) was mixed with 10 mℓ of acetic acid and 10 mℓ of water. The resulting mixture was cooled to -5 to 0°C. Thereafter, chlorine gas was introduced into the cooled mixture to effect a reaction.

After completion of the reaction, the reaction mixture was poured into water and subjected to extraction with methylene chloride. The extract was washed with water and dried over anhydrous sodium sulfate, followed by introduction thereinto of ammonia gas to effect a reaction at room temperature for one hour.

After completion of the reaction, the reaction mixture was filtered. The filtrate was concentrated under reduced pressure and allowed to stand. Thereafter, the solidified residue was washed with ethyl acetate and n-hexane to obtain 0.62 g of 6-[(N-ethyl-N-methylsulfonyl)amino]-2-pyridinesulfonamide (Intermediate No. 2 in Table 1 which will be given later) having a melting point of 140 to 143°C.

5) 0.20 g of 6-[(N-ethyl-N-methylsulfonyl)amino]-2-pyridinesulfonamide obtained in the above step 4) was mixed with 0.20 g of phenyl (4,6-dimethoxypyrimidin-2-yl)carbamate and 7 mℓ of acetonitrile. The resulting mixture was further admixed and reacted with 0.11 g of 1,8-diazabicyclo[5.4.0]-7-undecene at room temperature for one hour.

After completion of the reaction, the reaction mixture was poured into water and weakly acidified with hydrochloric acid. The resulting solid substance was filtered off, washed with water, and dried to obtain 0.25 g of the desired product (Compound No. 2) having a melting point of 145 to 147°C.

Synthesis Example 2

Synthesis of 6-[(N-ethyl-N-ethylsulfonyl)amino]-N-[[(4,6-dimethoxypyrimidin-2-yl)amino]carbonyl]-2-pyridinesulfonamide (Compound No. 18 in Table 2 which will be given later)

1) 2.0 g of 2-amino-6-benzylthiopyridine and 30 mℓ of tetrahydrofuran was mixed together and further admixed with 0.52 g of powdery potassium hydroxide, followed by agitation thereof at room temperature. The resulting mixture was cooled with ice. 2.38 g of ethanesulfonyl chloride was added dropwise to the ice-cooled mixture, followed by reaction under agitation at room temperature for 30 minutes.

After completion of the reaction, the reaction mixture was poured into water and subjected to extraction with methylene chloride. The extract was dried over anhydrous sodium sulfate and concentrated under reduced pressure. The resulting residue was purified through silica gel column chromatography with a developing solvent (ethyl acetate : hexane = 1:1) to obtain 0.90 g of N-(6-benzylthiopyridin-2-yl)ethanesulfonamide having a melting point of 87 to 92°C.

2) 0.85 g of N-(6-benzylthiopyridin-2-yl)ethanesulfonamide obtained in the above step 1) was mixed with 15 mℓ of tetrahydrofuran. The resulting mixture was cooled with ice and admixed with 0.13 g of 60 wt% sodium hydride, followed by agitation thereof at room temperature. Thereafter, 2.0 g of ethyl iodide was added to the mixture. The resulting mixture was heated up and reacted under reflux for 1.5 hours.

After completion of the reaction, the reaction mixture was poured into water and subjected to extraction with methylene chloride. The extract was dried over anhydrous sodium sulfate and concentrated under reduced pressure. The resulting residue was purified through silica gel column chromatography with a developing solvent (ethyl acetate : hexane = 1:1) to obtain 0.75 g of oily N-(6-benzylthiopyridin-2-yl)-N-ethylethanesulfonamide.

3) 0.75 g of N-(6-benzylthiopyridin-2-yl)-N-ethylethanesulfonamide obtained in the above step 2) was mixed with 20 mℓ of acetic acid and 15 mℓ of water. The resulting mixture was cooled to -5 to 0°C. Thereafter, the chlorine gas was introduced into the cooled mixture to effect a reaction.

After completion of the reaction, the reaction mixture was poured into water and subjected to extraction with methylene chloride. The extract was washed with water and dried over anhydrous sodium sulfate, followed by introduction thereinto of ammonia gas to effect a reaction at room temperature for one hour.

After completion of the reaction, the reaction mixture was filtered. The filtrate was concentrated under reduced pressure and allowed to stand. Thereafter, the solidified residue was washed with ethyl acetate and n-hexane to obtain 0.60 g of 6-[(N-ethyl-N-ethylsulfonyl)amino]-2-pyridinesulfonamide (Intermediate No. 17 in Table 1 which will be given later) having a melting point of 115 to 118 °C.

4) 0.21 g of 6-[(N-ethyl-N-ethylsulfonyl)amino]-2-pyridinesulfonamideobtained in the above step 3) was mixed with 0.20 g of phenyl (4,6-dimethoxypyrimidin-2-yl)carbamate and 7 mℓ of acetonitrile. The resulting mixture was further admixed and reacted with 0.11 g of 1,8-diazabicyclo[5.4.0]-7-undecene at room temperature for 30 minutes.

After completion of the reaction, the reaction mixture was poured into water and weakly acidified with hydrochloric acid. The resulting solid substance was filtered off, washed with water, and dried to obtain 0.25 g of the desired product (Compound No. 18) having a melting point of 165 to 170 °C.

Synthesis Example 3

Synthesis of 6-[(N-ethyl-N-isopropylsulfonyl)amino]-N-[[(4,6-dimethoxypyrimidin-2-yl)amino]carbonyl]-2-pyridinesulfonamide (Compound No. 22 in Table 2 which will be given later)

1) 12 g of 2-benzylthio-6-bromopyridine, 70 mℓ of a 40 wt% aqueous solution of ethylamine, and a catalytic amount of cuprous chloride were mixed together and reacted in an autoclave at 180 °C for 4 hours.

After completion of the reaction, the reaction mixture was poured into water and subjected to extraction with methylene chloride. The extract was washed with water, dried over anhydrous sodium sulfate, and concentrated under reduced pressure. The resulting residue was purified through silica gel column chromatography with a developing solvent (ethyl acetate : hexane = 1:10) to obtain 5.5 g of oily 2-benzylthio-6-ethylaminopyridine.

2) 2.0 g of 2-benzylthio-6-ethylaminopyridine obtained in the above step 1) was mixed and reacted with 2.33 g of isopropylsulfonyl chloride under agitation at 80 to 100 °C for one night.

After completion of the reaction, methylene chloride was added to the reaction mixture. The resulting mixture was washed with water, dried over anhydrous sodium sulfate, and concentrated under reduced pressure. The resulting residue was purified through silica gel column chromatography with a developing solvent (ethyl acetate : hexane = 1:10) to obtain 0.20 g of oily N-(6-benzylthiopyridin-2-yl)-N-ethylisopropylsulfonamide.

3) 0.20 g of N-(6-benzylthiopyridin-2-yl)-N-ethylisopropylsulfonamide obtained in the above step 2) was mixed with 25 mℓ of acetic acid and 20 mℓ of water. The resulting mixture was cooled to -5 to 0 °C. Chlorine gas was introduced into the cooled mixture to effect a reaction.

After completion of the reaction, the reaction mixture was poured into water and subjected to extraction with methylene chloride. The extract was washed with water and dried over anhydrous sodium sulfate, followed by introduction thereinto of ammonia gas to effect a reaction at room temperature for 30 minutes.

After completion of the reaction, the reaction mixture was filtered. The filtrate was concentrated under reduced pressure. The resulting residue was purified through silica gel column chromatography with a developing solvent (ethyl acetate : hexane = 3:1) to obtain 0.12 g of oily 6-[(N-ethyl-N-isopropylsulfonyl)amino]-2-pyridinesulfonamide (Intermediate No. 21 in Table 1 which will be given later).

4) 0.12 g of 6-[(N-ethyl-N-isopropylsulfonyl)amino]-2-pyridinesulfonamide obtained in the above step 3) was mixed with 0.11 g of phenyl (4,6-dimethoxypyrimidin-2-yl)carbamate and 7 mℓ of acetonitrile and further admixed with 59 mg of 1,8-diazabicyclo[5.4.0]-7-undecene to effect a reaction at room temperature for 30 minutes.

After completion of the reaction, the reaction mixture was poured into water, weakly acidified with hydrochloric acid, and subjected to extraction with methylene chloride. The extract was dried over anhydrous sodium sulfate and concentrated under reduced pressure. The resulting residue was purified through silica gel column chromatography with a developing solvent (ethyl acetate : hexane = 4:1) to obtain 0.11 g of the desired product (Compound No. 22) having a melting point of 163 to 166 °C

Representative examples of the intermediate represented by the general formula (II-1) which were prepared in substantially the same manner as described above are listed in Table 1, while representative examples of the substituted pyridinesulfonamide compound of the present invention represented by the general formula (I) which were prepared in substantially the same manner as described above are listed in Table 2.

Table 1

| Intermediate No. | $R_1$ | $R_2$ | Physical Properties m.p. (°C) |
|---|---|---|---|
| 1 | $CH_3$ | $CH_3$ | 118-119 |
| 2 | $CH_3$ | $C_2H_5$ | 140-143 |
| 3 | $CH_3$ | $n-C_3H_7$ | 100-104 |
| 4 | $C_2H_5$ | $CH_3$ | |
| 5 | $CF_3$ | $CH_3$ | |
| 6 | $CF_3$ | $C_2H_5$ | 148-151 |
| 7 | $CF_3$ | $n-C_3H_7$ | 142-145 |
| 8 | $CF_3$ | $n-C_4H_9$ | 138-140 |
| 9 | $CH_3$ | $iso-C_3H_7$ | 179-181 |
| 10 | $CF_3$ | $iso-C_3H_7$ | |
| 11 | $iso-C_3H_7$ | $CH_3$ | 130-133 |
| 12 | cyclopropyl | $CH_3$ | 132-134 |
| 13 | $CH_3$ | cyclopropyl | |
| 14 | $-(CH_2)_3-$ | | 224-229 |
| 15 | $-(CH_2)_4-$ | | 175-176 |
| 16 | $-(CH_2)_5-$ | | |
| 17 | $C_2H_5$ | $C_2H_5$ | 115-118 |
| 18 | $C_2H_5$ | $n-C_3H_7$ | 111-115 |
| 19 | $n-C_3H_7$ | $CH_3$ | oily substance |
| 20 | $n-C_3H_7$ | $C_2H_5$ | 125-128 |
| 21 | $iso-C_3H_7$ | $C_2H_5$ | oily substance |

Table 1 (continued)

| Intermediate No. | $R_1$ | $R_2$ | Physical Properties m.p. (°C) |
|---|---|---|---|
| 22 | n-$C_4H_9$ | $C_2H_5$ | 70-73 |
| 23 | tert-$C_4H_9$ | $C_2H_5$ | |
| 24 | n-$C_6H_{13}$ | $C_2H_5$ | |
| 25 | $CH_2CF3$ | $C_2H_5$ | 108-109 |
| 26 | $(CH_2)_3Cl$ | $C_2H_5$ | oily substance |
| 27 | $(CH_2)_3CF_3$ | $C_2H_5$ | |
| 28 | $C_2H_5OC_2H_5$ | $C_2H_5$ | |
| 29 | $CH(CH_3)CH_2OC_2H_5$ | $C_2H_5$ | |
| 30 | $CH=CH_2$ | $CH_3$ | |
| 31 | $CH=CH_2$ | $C_2H_5$ | 75-79 |
| 32 | $CH=CHCF_3$ | $C_2H_5$ | |
| 33 | cyclohexyl | $C_2H_5$ | 123-124 |
| 34 | cyclopropyl | $C_2H_5$ | 137-139 |
| 35 | $C_2H_5$ | cyclopropyl | |
| 36 | cyclopentyl | $C_2H_5$ | oily substance |
| 37 | 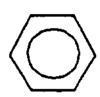 | $CH_3$ | 96-99 |
| 38 | 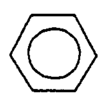 | $C_2H_5$ | oily substance |
| 39 | 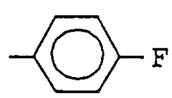 | $CH_3$ | oily substance |

Table 1 (continued)

| Intermediate No. | $R_1$ | $R_2$ | Physical Properties m.p. (°C) |
|---|---|---|---|
| 40 | 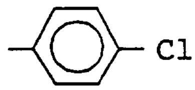 | $C_2H_5$ | oily substance |
| 41 | 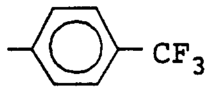 | $CH_3$ | oily substance |
| 42 | 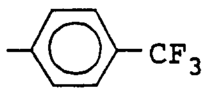 | $C_2H_5$ | 98-100 |
| 43 | 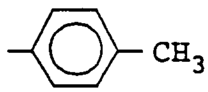 | $C_2H_5$ | |
| 44 | 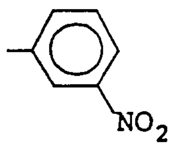 | $C_2H_5$ | 185-188 |
| 45 | sec-$C_4H_9$ | $CH_3$ | 95-96 |
| 46 | sec-$C_4H_9$ | $C_2H_5$ | oily substance |
| 47 | sec-$C_4H_9$ | n-$C_3H_7$ | |
| 48 | sec-$C_4H_9$ | iso-$C_3H_7$ | |
| 49 | iso-$C_3H_7$ | n-$C_3H_7$ | oily substance |
| 50 | iso-$C_4H_9$ | $C_2H_5$ | oily substance |
| 51 | n-$C_4H_9$ | $CH_3$ | oily substance |
| 52 | $(CH_2)_2CH(CH_3)_2$ | $C_2H_5$ | oily substance |
| 53 | $(CH_2)_2CH(CH_3)_2$ | $CH_3$ | oily substance |
| 54 | $CH_3$ | $CHF_2$ | 126-128 |
| 55 | $C_2H_5$ | $CHF_2$ | 106-109 |
| 56 | cyclohexyl | $CH_3$ | oily substance |

Table 1 (continued)

| Intermediate No. | $R_1$ | $R_2$ | Physical Properties m.p. (°C) |
|---|---|---|---|
| 57 | tert-$C_4H_9$ | $CH_3$ | |
| 58 | $CH_3$ | $CH_2CF_3$ | |
| 59 | $C_2H_5$ | $CH_2CF_3$ | |
| 60 | iso-$C_3H_7$ | $CH_2CF_3$ | |
| 61 | $C_2H_5$ | iso-$C_3H_7$ | 183-185 |
| 62 | n-$C_3H_7$ | iso-$C_3H_7$ | |
| 63 | cyclopropyl | n-$C_3H_7$ | |
| 64 | n-$C_3H_7$ | cyclopropyl | |
| 65 | $CH_3$ | n-$C_4H_9$ | |
| 66 | $C_2H_5$ | n-$C_4H_9$ | |
| 67 | n-$C_3H_7$ | n-$C_4H_9$ | |
| 68 | iso-$C_3H_7$ | n-$C_4H_9$ | |
| 69 | $CH_3$ | sec-$C_4H_9$ | |
| 70 | $C_2H_5$ | sec-$C_4H_9$ | |
| 71 | n-$C_3H_7$ | sec-$C_4H_9$ | |
| 72 | iso-$C_3H_7$ | sec-$C_4H_9$ | |
| 73 | $CH_3$ | iso-$C_4H_9$ | |
| 74 | $C_2H_5$ | iso-$C_4H_9$ | |
| 75 | n-$C_3H_7$ | iso-$C_4H_9$ | |
| 76 | iso-$C_3H_7$ | iso-$C_4H_9$ | |
| 77 | n-$C_3H_7$ | n-$C_3H_7$ | |
| 78 | iso-$C_3H_7$ | iso-$C_3H_7$ | |

Table 1 (continued)

| Intermediate No. | $R_1$ | $R_2$ | Physical Properties m.p. (°C) |
|---|---|---|---|
| 79 | cyclopropyl | iso-$C_3H_7$ | |
| 80 | iso-$C_4H_9$ | $CH_3$ | |
| 81 | cyclobutyl | $CH_3$ | 142-144 |
| 82 | cyclobutyl | $C_2H_5$ | oily substance |
| 83 | cyclobutyl | n-$C_3H_7$ | |
| 84 | cyclobutyl | iso-$C_3H_7$ | |
| 85 | iso-$C_4H_9$ | n-$C_3H_7$ | |
| 86 | iso-$C_4H_9$ | iso-$C_3H_7$ | |
| 87 | n-$C_4H_9$ | n-$C_3H_7$ | |
| 88 | n-$C_4H_9$ | iso-$C_3H_7$ | |

Table 2

| Compound No. | $R_1$ | $R_2$ | X | Y | Physical Properties m.p. (°C) |
|---|---|---|---|---|---|
| 1 | $CH_3$ | $CH_3$ | $OCH_3$ | $OCH_3$ | 176-177 |
| 2 | $CH_3$ | $C_2H_5$ | $OCH_3$ | $OCH_3$ | 145-147 |
| 3 | $CH_3$ | n-$C_3H_7$ | $OCH_3$ | $OCH_3$ | 85-88 |
| 4 | $C_2H_5$ | $CH_3$ | $OCH_3$ | $OCH_3$ | 161-163 |
| 5 | $CF_3$ | $CH_3$ | $OCH_3$ | $OCH_3$ | viscous substance |
| 6 | $CF_3$ | $C_2H_5$ | $OCH_3$ | $OCH_3$ | 179-180 |
| 7 | $CF_3$ | n-$C_3H_7$ | $OCH_3$ | $OCH_3$ | 159-161 |
| 8 | $CF_3$ | n-$C_4H_9$ | $OCH_3$ | $OCH_3$ | 116-118 |
| 9 | $CH_3$ | iso-$C_3H_7$ | $OCH_3$ | $OCH_3$ | 132-138 |
| 10 | $CF_3$ | iso-$C_3H_7$ | $OCH_3$ | $OCH_3$ | |
| 11 | iso-$C_3H_7$ | $CH_3$ | $OCH_3$ | $OCH_3$ | 143-146 |
| 12 | cyclopropyl | $CH_3$ | $OCH_3$ | $OCH_3$ | 164-168 |
| 13 | $CH_3$ | cyclopropyl | $OCH_3$ | $OCH_3$ | |
| 14 | $CH_3$ | $C_2H_5$ | $CH_3$ | $OCH_3$ | |
| 15 | $-(CH_2)_3-$ | | $OCH_3$ | $OCH_3$ | 191-194 |
| 16 | $-(CH_2)_4-$ | | $OCH_3$ | $OCH_3$ | 182-185 |
| 17 | $-(CH_2)_5-$ | | $OCH_3$ | $OCH_3$ | |
| 18 | $C_2H_5$ | $C_2H_5$ | $OCH_3$ | $OCH_3$ | 165-170 |
| 19 | $C_2H_5$ | n-$C_3H_7$ | $OCH_3$ | $OCH_3$ | 140-143 |

Table 2 (continued)

| Compound No. | $R_1$ | $R_2$ | X | Y | Physical Properties m.p. (°C) |
|---|---|---|---|---|---|
| 20 | n-$C_3H_7$ | $CH_3$ | $OCH_3$ | $OCH_3$ | 175-178 |
| 21 | n-$C_3H_7$ | $C_2H_5$ | $OCH_3$ | $OCH_3$ | 151-154 |
| 22 | iso-$C_3H_7$ | $C_2H_5$ | $OCH_3$ | $OCH_3$ | 163-166 |
| 23 | n-$C_4H_9$ | $C_2H_5$ | $OCH_3$ | $OCH_3$ | 155-158 |
| 24 | tert-$C_4H_9$ | $C_2H_5$ | $OCH_3$ | $OCH_3$ | |
| 25 | n-$C_6H_{13}$ | $C_2H_5$ | $OCH_3$ | $OCH_3$ | |
| 26 | $CH_2CF_3$ | $C_2H_5$ | $OCH_3$ | $OCH_3$ | 184-185 |
| 27 | $(CH_2)_3Cl$ | $C_2H_5$ | $OCH_3$ | $OCH_3$ | 156-159 |
| 28 | $(CH_2)_3CF_3$ | $C_2H_5$ | $OCH_3$ | $OCH_3$ | |
| 29 | $C_2H_5OC_2H_5$ | $C_2H_5$ | $OCH_3$ | $OCH_3$ | |
| 30 | $CH(CH_3)CH_2OC_2H_5$ | $C_2H_5$ | $OCH_3$ | $OCH_3$ | |
| 31 | $CH=CH_2$ | $CH_3$ | $OCH_3$ | $OCH_3$ | 150-152 |
| 32 | $CH=CH_2$ | $C_2H_5$ | $OCH_3$ | $OCH_3$ | 145-148 |
| 33 | $CH=CHCF_3$ | $C_2H_5$ | $OCH_3$ | $OCH_3$ | |
| 34 | cyclohexyl | $C_2H_5$ | $OCH_3$ | $OCH_3$ | 148-149 |
| 35 | cyclopropyl | $C_2H_5$ | $OCH_3$ | $OCH_3$ | 169-172 |
| 36 | $C_2H_5$ | cyclopropyl | $OCH_3$ | $OCH_3$ | |
| 37 | cyclopentyl | $C_2H_5$ | $OCH_3$ | $OCH_3$ | 135-138 |
| 38 | 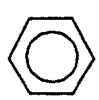 | $CH_3$ | $OCH_3$ | $OCH_3$ | 170-174 |
| 39 | 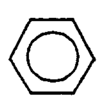 | $C_2H_5$ | $OCH_3$ | $OCH_3$ | 142-145 |

Table 2 (continued)

| Compound No. | $R_1$ | $R_2$ | X | Y | Physical Properties m.p. (°C) |
|---|---|---|---|---|---|
| 40 | 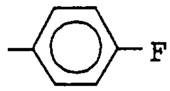 | $CH_3$ | $OCH_3$ | $OCH_3$ | 193-197 |
| 41 | 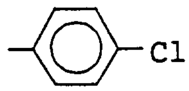 | $C_2H_5$ | $OCH_3$ | $OCH_3$ | 166-169 |
| 42 | 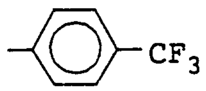 | $CH_3$ | $OCH_3$ | $OCH_3$ | 122-124 |
| 43 | 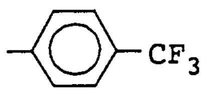 | $C_2H_5$ | $OCH_3$ | $OCH_3$ | 157-160 |
| 44 | 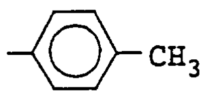 | $C_2H_5$ | $OCH_3$ | $OCH_3$ | |
| 45 | 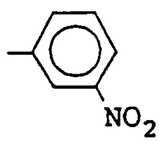 | $C_2H_5$ | $OCH_3$ | $OCH_3$ | 161-164 |
| 46 | sec-$C_4H_9$ | $CH_3$ | $OCH_3$ | $OCH_3$ | 153-154 |
| 47 | sec-$C_4H_9$ | $C_2H_5$ | $OCH_3$ | $OCH_3$ | 136-137 |
| 48 | sec-$C_4H_9$ | n-$C_3H_7$ | $OCH_3$ | $OCH_3$ | |
| 49 | sec-$C_4H_9$ | iso-$C_3H_7$ | $OCH_3$ | $OCH_3$ | |
| 50 | iso-$C_3H_7$ | n-$C_3H_7$ | $OCH_3$ | $OCH_3$ | 163-166 |
| 51 | iso-$C_4H_9$ | $C_2H_5$ | $OCH_3$ | $OCH_3$ | 150-153 |
| 52 | n-$C_4H_9$ | $CH_3$ | $OCH_3$ | $OCH_3$ | 175-177 |
| 53 | $(CH_2)_2CH(CH_3)_2$ | $C_2H_5$ | $OCH_3$ | $OCH_3$ | 139-140 |
| 54 | $(CH_2)_2CH(CH_3)_2$ | $CH_3$ | $OCH_3$ | $OCH_3$ | 158-159 |

Table 2 (continued)

| Compound No. | $R_1$ | $R_2$ | X | Y | Physical Properties m.p. (°C) |
|---|---|---|---|---|---|
| 55 | $CH_3$ | $CHF_2$ | $OCH_3$ | $OCH_3$ | 155-157 |
| 56 | $C_2H_5$ | $CHF_2$ | $OCH_3$ | $OCH_3$ | 157-159 |
| 57 | cyclohexyl | $CH_3$ | $OCH_3$ | $OCH_3$ | 164-165 |
| 58 | 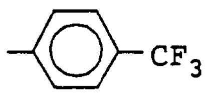 | $CH_3$ | $CH_3$ | $CH_3$ | 93-95 |
| 59 | tert-$C_4H_9$ | $CH_3$ | $OCH_3$ | $OCH_3$ | |
| 60 | $CH_3$ | $CH_2CF_3$ | $OCH_3$ | $OCH_3$ | |
| 61 | $C_2H_5$ | $CH_2CF_3$ | $OCH_3$ | $OCH_3$ | |
| 62 | iso-$C_3H_7$ | $CH_2CF_3$ | $OCH_3$ | $OCH_3$ | |
| 63 | $C_2H_5$ | iso-$C_3H_7$ | $OCH_3$ | $OCH_3$ | 125-129 |
| 64 | n-$C_3H_7$ | iso-$C_3H_7$ | $OCH_3$ | $OCH_3$ | |
| 65 | cyclopropyl | n-$C_3H_7$ | $OCH_3$ | $OCH_3$ | |
| 66 | n-$C_3H_7$ | cyclopropyl | $OCH_3$ | $OCH_3$ | |
| 67 | $CH_3$ | n-$C_4H_9$ | $OCH_3$ | $OCH_3$ | |
| 68 | $C_2H_5$ | n-$C_4H_9$ | $OCH_3$ | $OCH_3$ | |
| 69 | n-$C_3H_7$ | n-$C_4H_9$ | $OCH_3$ | $OCH_3$ | |
| 70 | iso-$C_3H_7$ | n-$C_4H_9$ | $OCH_3$ | $OCH_3$ | |
| 71 | $CH_3$ | sec-$C_4H_9$ | $OCH_3$ | $OCH_3$ | |
| 72 | $C_2H_5$ | sec-$C_4H_9$ | $OCH_3$ | $OCH_3$ | |
| 73 | n-$C_3H_7$ | sec-$C_4H_9$ | $OCH_3$ | $OCH_3$ | |
| 74 | iso-$C_3H_7$ | sec-$C_4H_9$ | $OCH_3$ | $OCH_3$ | |

Table 2 (continued)

| Compound No. | $R_1$ | $R_2$ | X | Y | Physical Properties m.p. (°C) |
|---|---|---|---|---|---|
| 75 | $CH_3$ | iso-$C_4H_9$ | $OCH_3$ | $OCH_3$ | |
| 76 | $C_2H_5$ | iso-$C_4H_9$ | $OCH_3$ | $OCH_3$ | |
| 77 | n-$C_3H_7$ | iso-$C_4H_9$ | $OCH_3$ | $OCH_3$ | |
| 78 | iso-$C_3H_7$ | iso-$C_4H_9$ | $OCH_3$ | $OCH_3$ | |
| 79 | n-$C_3H_7$ | n-$C_3H_7$ | $OCH_3$ | $OCH_3$ | |
| 80 | iso-$C_3H_7$ | iso-$C_3H_7$ | $OCH_3$ | $OCH_3$ | |
| 81 | cyclopropyl | iso-$C_3H_7$ | $OCH_3$ | $OCH_3$ | |
| 82 | iso-$C_4H_9$ | $CH_3$ | $OCH_3$ | $OCH_3$ | |
| 83 | cyclobutyl | $CH_3$ | $OCH_3$ | $OCH_3$ | 167-169 |
| 84 | cyclobutyl | $C_2H_5$ | $OCH_3$ | $OCH_3$ | 169-170 |
| 85 | cyclobutyl | n-$C_3H_7$ | $OCH_3$ | $OCH_3$ | |
| 86 | cyclobutyl | iso-$C_3H_7$ | $OCH_3$ | $OCH_3$ | |
| 87 | iso-$C_4H_9$ | n-$C_3H_7$ | $OCH_3$ | $OCH_3$ | |
| 88 | iso-$C_4H_9$ | iso-$C_3H_7$ | $OCH_3$ | $OCH_3$ | |
| 89 | n-$C_4H_9$ | n-$C_3H_7$ | $OCH_3$ | $OCH_3$ | |
| 90 | n-$C_4H_9$ | iso-$C_3H_7$ | $OCH_3$ | $OCH_3$ | |
| 91 | $C_2H_5$ | $C_2H_5$ | $CH_3$ | $OCH_3$ | |
| 92 | $C_2H_5$ | $C_2H_5$ | $CH_3$ | $CH_3$ | |
| 93 | $C_2H_5$ | n-$C_3H_7$ | $CH_3$ | $OCH_3$ | |
| 94 | $C_2H_5$ | n-$C_3H_7$ | $CH_3$ | $CH_3$ | |
| 95 | iso-$C_3H_7$ | $C_2H_5$ | $CH_3$ | $OCH_3$ | |
| 96 | iso-$C_3H_7$ | $C_2H_5$ | $CH_3$ | $CH_3$ | |

Table 2 (continued)

| Compound No. | $R_1$ | $R_2$ | X | Y | Physical Properties m.p. (°C) |
|---|---|---|---|---|---|
| 97 | cyclopropyl | $C_2H_5$ | $CH_3$ | $OCH_3$ | |
| 98 | cyclopropyl | $C_2H_5$ | $CH_3$ | $CH_3$ | |
| 99 | sec-$C_4H_9$ | $C_2H_5$ | $CH_3$ | $OCH_3$ | |
| 100 | sec-$C_4H_9$ | $C_2H_5$ | $CH_3$ | $CH_3$ | |
| 101 | $C_2H_5$ | iso-$C_3H_7$ | $CH_3$ | $OCH_3$ | |
| 102 | $C_2H_5$ | iso-$C_3H_7$ | $CH_3$ | $CH_3$ | |

As the effective ingredient of the herbicide, the substituted pyridinesulfonamide compound or its salt of the present invention, as will be evident from the test examples given hereinafter, exhibits a wide herbicidal spectrum at a low dosage while it shows safety on soybean, wheat, cotton, etc.

The herbicide of the present invention can be applied to a wide variety of sites including agricultural fields such as upland fields, orchards and mulberry fields, and non-agricultural fields such as forests, farm roads, playgrounds and factory sites. The method of application of the herbicide of the present invention can be arbitrarily chosen from a soil treatment application and a foliage treatment application. The herbicide of the present invention may be applied in the form of a formulation such as a dust, granules, water-dispersible granules, a wettable powder, an emulsifiable concentrate, a soluble concentrate, and a suspension concentrate, which is prepared by blending the substituted pyridinesulfonamide compound or its salt of the present invention as the effective ingredient usually with a carrier and, if necessary, further with various other adjuvants selected from among diluents, solvents, emulsifiers, spreaders, surfactants, etc. The suitable blending weight ratio of the effective ingredient to the agricultural adjuvants may be in the range of 1:99 to 90:10, preferably in the range of 5:95 to 80:20. The suitable amount of the effective ingredient to be used cannot be unequivocally determined because it varies depending on weather conditions, soil conditions, the form of the above-mentioned formulation, the kinds of object weeds, and the application season, However, the amount of the effective ingredient to be applied is generally in the range of 0.005 g to 50 g/a (are), preferably in the range of 0.01 to 10 g/a, more preferably in the range of 0.05 to 5 g/a.

The herbicide of the present invention may be used in mixture or combination with at least one ingredient or component selected from among other agricultural chemicals, agricultural adjuvants, phytotoxicity-reducing agents, etc. to sometimes exhibit improvements in effect and functions. The herbicide of the present invention may be used in mixture or combination with another herbicide, in which case a synergistic effect can sometimes be attained.

Examples of another herbicide are as follows: Phenoxypropionate compounds such as

* ethyl (±)-2-[4-[(6-chloro-2-quinoxalinyl)oxy]phenoxy]propionate (common name: quizalofop-ethyl)
* ethyl (±)-2-[4-[(6-chloro-2-benzoxazolyl)oxy]phenoxy]propionate (common name: fenoxaprop-ethyl)
* butyl (±)-2-[4-[[5-(trifluoromethyl)-2-pyridinyl]oxy]phenoxy]propionate (common name: fluazifop-butyl)
* methyl 2-[4-[[3-chloro-5-(trifluoromethyl)-2-pyridinyl]oxy]phenoxy]propionate (common name: haloxyfop-methyl)
* 2-ethoxyethyl 2-[4-[[3-chloro-5-(trifluoromethyl)-2-pyridinyl]oxy]phenoxy]propionate (common name: haloxyfop-etotyl)
* (R)-2-[[(1-methylethylidene)amino]oxy]ethyl 2-[4-[6-chloro-2-quinoxalinyl)oxy]phenoxy]propanoate (common name: propaquizafop);

Diphenyl ether compounds such as

* sodium 5-[2-chloro-4-(trifluoromethyl)phenoxy]-2-nitrobenzoate (common name: acifluorfen-sodium)
* (±)-2-ethoxy-1-methyl-2-oxoethyl-5-[2-chloro-4-(trifluoromethyl)phenoxy]-2-nitrobenzoate (common name: lactofen)

* 5-[2-chloro-4-(trifluoromethyl)phenoxy]-N-(methylsulfonyl)-2-nitrobenzamide (common name: fomesafen);

Haloacetamide compounds such as

* 2-chloro-N-(2-ethyl-6-methylphenyl)-N-(2-methoxy-1-methylethyl)acetamide (common name: metolachlor)
* 2-chloro-N-(2,6-diethylphenyl)-N-(methoxymethyl)acetamide (common name: alachlor);

Imidazolinone compounds such as

* (±)-2-[4,5-dihydro-4-methyl-4-(1-methylethyl)-5-oxo-1H-imidazol-2-yl]-5-ethyl-3-pyridinecarboxylic acid (common name: imazethapyr)
* 2-[4,5-dihydro-4-methyl-4-(1-methylethyl)-5-oxo-1H-imidazol-2-yl]-3-quinolinecarboxylic acid (common name: imazaquin);

Dinitroaniline compounds such as

* 2,6-dinitro-N,N-dipropyl-4-(trifluoromethyl)aniline (common name: trifluralin)
* N-(1-ethylpropyl)-3,4-dimethyl-2,6-dinitroaniline (common name: pendimethalin);

Carbamate compounds such as

* 3-[(methoxycarbonyl)amino]phenyl (3-methylphenyl)carbamate (common name: phenmedipham)
* ethyl [3-[[(phenylamino)carbonyl]oxy]phenyl]carbamate (common name: desmedipham);

Cyclohexane dione compounds such as

* 2-[1-(ethoxyimino)butyl]-5-[2-(ethylthio)propyl]-3-hydroxy-2-cyclohexen-1-one (common name: sethoxydim); and

Other compounds such as

* 3-(1-methylethyl)-(1H)-2,1,3-benzothiadiazin-4(3H)-one-2,2-dioxide (common name: bentazon)
* ethyl 2-[[[[(4-chloro-6-methoxy-2-pyrimidinyl)amino]carbonyl]amino]sulfonyl]benzoate (common name: chlorimuron-ethyl)
* 4-(2,4-dichlorophenoxy)butanoic acid (common name: 2,4-DB)
* 3-(3,4-dichlorophenyl)-1-methoxy-1-methylurea (common name: linuron)
* 4-amino-6-(1,1-dimethylethyl)-3-(methylthio)-1,2,4-triazin-5(4H)-one (common name: metribuzin)
* 7-oxabicyclo[2.2.1]heptane-2,3-dicarboxylic acid (common name: endothal)
* S-ethyl dipropylcarbamothioate (common name: EPTC)
* (±)-2-ethoxy-2,3-dihydro-3,3-dimethyl-5-benzofuranyl methanesulfonate (common name: ethofumesate)
* 5-amino-4-chloro-2-phenyl-3(2H)-pyridazinone (Common name: chloridazon)

The substituted pyridinesulfonamide compound or its salt as the effective ingredient of the herbicide of the present invention may be used in an amount of 0.05 to 5 g/a in combination with above-exemplified another compound in an amount of 0.01 to 50 g/a.

In particular, the phenoxypropionate compounds, the imidazolinone compounds and the cyclohexane dione compounds may be used in an amount of 0.2 to 5 g/a, and the haloacetamide compounds and the dinitroaniline compounds may be used in an amount of 5 to 30 g/a in combination with the substituted pyridinesulfonamide compound or its salt of the present invention.

Further, the following compounds may be also used as the another herbicide in mixture or combination with the herbicide of the present invention, where a synergistic effect can be attained, too.

Sulfonylurea compounds such as

* methyl 2-[[[[(4-methoxy-6-methyl-1,3,5-triazin-2-yl)amino]carbonyl]amino]sulfonyl]benzoate (common name: metsulfuron-methyl)
* methyl 2-[[[[N-(4-methoxy-6-methyl-1,3,5-triazin-2-yl)methylamino]carbonyl]amino]sulfonyl]benzoate (common name: tribenuron-methyl)
* 2-chloro-N-[[(4-methoxy-6-methyl-1,3,5-triazin-2-yl]amino]carbonyl]benzenesulfonamide (common name: chlorsulfuron)
* methyl 3-[[[[(4-methoxy-6-methyl-1,3,5-triazin-2-yl)amino]carbonyl]amino]sulfonyl]-2-thiophenecarboxylate (common name: thiameturon-methyl);

Phenoxypropionate compounds such as

* methyl (±)-2-[4-(2,4-dichlorophenoxy)phenoxy]propionate (common name: diclofop-methyl);

Imidazolinone compounds such as

* mixture of methyl 6-(4-isopropyl-4-methyl-5-oxo-2-imidazolin-2-yl)-m-toluate and methyl 2-(4-isopropyl-4-methyl-5-oxo-2-imidazolin-2-yl)-p-toluate (common name: imazamethabenz); and

Other compounds such as

* 1,2-dimethyl-3,5-diphenyl-1H-pyrazolium methylsulfate (common name: difenzoquat methylsulfate)
* 3,5-dibromo-4-hydroxybenzonitrile (common name: bromoxynil)

* 4-hydroxy-3,5-diiodobenzonitrile (common name: ioxynil)
* 2,4-dichlorophenoxyacetic acid (common name: 2,4-D)
* S-(2,3,3-trichloro-2-propenyl)-bis(1-methylethyl)carbamothioate (common name: triallate)
* 4-chloro-2-methylphenoxyacetic acid (common name: MCPA)
* 3,6-dichloro-2-pyridinecarboxylic acid (common name: clopyralid)
* O-(6-chloro-3-phenyl-4-pyridazinyl)-S-octyl thiocarbonothioate (common name: pyridate)
* 3,6-dichloro-2-methoxybenzoic acid (common name: dicamba)
* N'-(3,4-dichlorophenyl)-N,N-dimethylurea (common name: diuron)
* 4-amino-3,5,6-trichloro-2-pyridinecarboxylic acid (common name: picloram)
* N,N-dimethyl N'-[3-(trifluoromethyl)phenyl]urea (common name: fluometuron)
* 2-[[4-chloro-6-(ethylamino)-1,3,5-triazin-2-yl]amino]-2-methylpropionitrile (common name: cyanazine)
* N,N'-bis(1-methylethyl)-6-(methylthio)-1,3,5-triazine-2,4-diamine (common name: prometryn)
* N-(3,4-dichlorophenyl)propanamide (common name: propanil)
* disodium salt of methylarsonic acid (common name: DSMA)
* monosodium salt of methylarsonic acid (common name: MSMA)
* 2-chloro-1-(3-ethoxy-4-nitrophenoxy)-4-(trifluoromethyl)benzene (common name: oxyfluorfen)
* O,O-bis(1-methylethyl) S-[2-[(phenylsulfonyl)amino]ethyl]phosphorodithioate (common name: bensulide)
* 2-(3,4-dichlorophenyl)-4-methyl-1,2,4-oxadiazolidine-3,5-dione (common name: methazole)
* 4-chloro-5-(methylamino)-2-[3-(trifluoromethyl)phenyl]-3(2H)-pyridazinone (common name: norflurazon)
* 2-chloro-6-(4,6-dimethoxypyrimidin-2-ylthio)benzoate (JP-A-1-230561) (the term "JP-A" as used herein means an "unexamined published Japanese patent application")
* ethyl 2-chloro-6-(4,6-dimethoxypyrimidin-2-ylthio)benzoate (JP-A-1-230561)
* sodium 2-chloro-6-(4,6-dimethoxypyrimidin-2-ylthio)benzoate (JP-A-1-230561)

The substituted pyridinesulfonamide compound or its salt as the effective ingredient of the herbicide of the present invention may be used in an amount of 0.05 to 5 g/a in combination with above-exemplified another compound in an amount of 0.01 to 50 g/a.

Test Examples, using the herbicide of the present invention, will now be described.

## Test Example 1

1/1,500 are [are (a) = 100 $m^2$] pots were filled with upland soil, which in the pots was then sown with a variety of plant seeds. The plants grown will be enumerated below.

| Name | Abbreviation |
|---|---|
| rice (Oryza sativa) | OR |
| soybean (Glycine max) | GL |
| corn (Zea mays) | ZE |
| cotton (Gossypium) | GO |
| wheat (Triticum) | TR |
| cocklebur (Xanthium strumarium) | XA |
| morning glory (Ipomoea purpurea) | IP |
| prickly sida (Sida spinosa) | SI |
| slender amaranth (Amaranthus viridis) | AM |
| barnyard grass (Echinochloa crus-galli) | EC |
| large crabgrass (Digitaria adscendens) | DI |

When each plant reached a given growth stage (a 1.3- to 3.0-leaf stage for rice, primary leaf to a 1.2-leaf stage for soybean, 2.3- to 4.1-leaf stage for corn, cotyledon to 1.1-leaf stage for cotton, 2.2- to 3.0-leaf stage for wheat, 1.0- to 3.2-leaf stage for cocklebur, 0.3- to 2.0-leaf stage for morning glory, 0.1- to 1.8-leaf stage for prickly sida, 0.3- to 2.4-leaf stage for slender amaranth, 1.7- to 3.3-leaf stage for barnyard glass, and 1.4- to 3.1-leaf stage for large crabgrass), a predetermined amount of the herbicide of the present invention in the form of a wettable powder, weighed out, diluted with 5 ℓ/a of water and admixed with 0.2 vol%, based on the resulting composition, of an agricultural spreader, was foliarly applied to the plant with a small spray gun. The progress of growth of the plant was visually observed 17 to 29 days after the foliar application to evaluate the degree of growth control according to 10 ratings (1: the same as in an untreated

plot - 10: perfect growth control). The results are listed in Table 3.

Table 3

| Compound No. | Amount of Effective Ingredient (g/a) | OR | GL | ZE | GO | TR | XA | IP | SI | AM | EC | DI | Day* |
|---|---|---|---|---|---|---|---|---|---|---|---|---|---|
| 1 | 1.25 | 8 | 7 | 10 | 8 | | 9 | 7 | 7 | 7 | 5 | | 18 |
| 2 | 1.25 | 8 | 3 | 10 | | 5 | 10 | 8 | 5 | 6 | 9 | 9 | 29 |
| | 0.31 | 7 | 2 | 9 | | 1 | 6 | 5 | 3 | 3 | 7 | 2 | |
| 3 | 1.25 | 7 | 3 | 10 | | 8 | 9 | 7 | 6 | 9 | 9 | 8 | 22 |
| | 0.31 | 5 | 3 | 8 | | 5 | 4 | 5 | 5 | 8 | 10 | 7 | |
| 4 | 0.31 | 8 | 4 | 9 | | 4 | 8 | 8 | 8 | 9 | 8 | 10 | 22 |
| 5 | 1.25 | 8 | 10 | 10 | 9 | | 10 | 9 | 6 | 10 | 7 | | 24 |
| | 0.31 | 8 | 10 | 10 | 8 | | 10 | 9 | 6 | 9 | 5 | | |
| 6 | 1.25 | 9 | 9 | 9 | 9 | | 10 | 9 | 8 | 10 | 9 | | 20 |
| | 0.31 | 7 | 10 | 8 | 9 | | 10 | 9 | 7 | 10 | 7 | | |
| 7 | 1.25 | 7 | 9 | 9 | 9 | | 10 | 10 | 8 | 10 | 7 | | 20 |
| | 0.31 | 6 | 9 | 8 | 8 | | 10 | 7 | 6 | 9 | 7 | | |
| 8 | 1.25 | 6 | 7 | 9 | 7 | | 10 | 10 | 5 | 9 | 7 | | 20 |
| | 0.31 | 6 | 5 | 8 | 5 | | 10 | 3 | 4 | 7 | | | |
| 11 | 1.25 | 8 | 7 | 10 | | | 10 | 10 | 6 | 10 | 10 | 3 | 21 |
| | 0.31 | 7 | 6 | 10 | | | 10 | 10 | 5 | 10 | 10 | 3 | |
| 15 | 1.25 | 2 | 3 | 3 | | 4 | 9 | 6 | 6 | 9 | 4 | 4 | 29 |
| | 0.31 | 2 | 2 | 2 | | 3 | 7 | 5 | 6 | 8 | 3 | 3 | |

Table 3 (continued)

| Compound No. | Amount of Effective Ingredient (g/a) | OR | GL | ZE | GO | TR | XA | IP | SI | AM | EC | DI | Day* |
|---|---|---|---|---|---|---|---|---|---|---|---|---|---|
| 18 | 1.25 | 9 | 3 | 10 | | 5 | 9 | 9 | 5 | | 9 | 10 | 29 |
| | 0.31 | 7 | 2 | 10 | | 4 | 9 | 7 | 3 | 7 | 7 | 9 | |
| 19 | 1.25 | 6 | 5 | 10 | | 4 | 10 | 7 | 6 | 9 | 10 | 9 | 23 |
| | 0.31 | 5 | 5 | 10 | | 2 | 9 | 6 | 3 | 5 | 9 | 9 | |
| 20 | 1.25 | 7 | 4 | 7 | | 2 | 9 | 8 | 5 | 5 | 1 | 5 | 21 |
| | 0.31 | 7 | 1 | 7 | | 1 | 8 | 7 | 3 | 3 | 1 | 1 | |
| 21 | 1.25 | 6 | 7 | 10 | | 2 | 10 | 7 | 3 | 7 | 6 | 4 | 23 |
| | 0.31 | 5 | 7 | 9 | | 1 | 9 | 6 | 2 | 6 | 4 | 3 | |
| 22 | 1.25 | 6 | 3 | 10 | | 6 | 10 | 9 | 8 | 10 | 8 | 5 | 20 |
| | 0.31 | 6 | 2 | 10 | | 5 | 10 | 8 | 6 | 10 | 8 | 3 | |
| 23 | 1.25 | 1 | 4 | 3 | | 2 | 7 | 5 | 6 | 9 | 2 | 2 | 20 |
| 26 | 1.25 | 2 | 3 | 3 | | 3 | 5 | 6 | 7 | 6 | 3 | 3 | 20 |
| | 0.31 | 2 | 2 | 1 | | 3 | 4 | 5 | 5 | 5 | 2 | 2 | |
| 27 | 1.25 | 2 | 1 | 3 | | 3 | 9 | 3 | 4 | 7 | 4 | 3 | 20 |
| | 0.31 | 1 | 1 | 2 | | 3 | 8 | 1 | 2 | 6 | 3 | 3 | |
| 31 | 1.25 | 7 | 7 | 10 | | 4 | 9 | 10 | 6 | 9 | 5 | 3 | 18 |
| | 0.31 | 6 | 7 | 9 | | 2 | 9 | 9 | 3 | 9 | 3 | 2 | |
| 32 | 1.25 | 3 | 6 | 4 | | 4 | 9 | 9 | 7 | 10 | 3 | 3 | 18 |
| | 0.31 | 1 | 6 | 4 | | 3 | 9 | 9 | 6 | 10 | 1 | 3 | |

Table 3 (continued)

| Compound No. | Amount of Effective Ingredient (g/a) | OR | GL | ZE | GO | TR | XA | IP | SI | AM | EC | DI | Day* |
|---|---|---|---|---|---|---|---|---|---|---|---|---|---|
| 34 | 1.25 | 8 | 6 | 10 | 9 | | 7 | 9 | 7 | 9 | 10 | 4 | 24 |
| | 0.31 | 6 | 5 | 10 | 4 | | 6 | 7 | 6 | 9 | 9 | 4 | |
| 37 | 1.25 | 8 | 9 | 10 | 6 | | 10 | 7 | 10 | 10 | 10 | 6 | 21 |
| | 0.31 | 7 | 9 | 10 | 5 | | 10 | 6 | 10 | 10 | 10 | 5 | |
| 38 | 1.25 | 1 | 4 | 3 | | 3 | 10 | 8 | 3 | 5 | 1 | 2 | 18 |
| | 0.31 | 1 | 3 | 1 | | 2 | 8 | 7 | 1 | 5 | 1 | 2 | |
| 39 | 1.25 | 1 | 6 | 1 | | 1 | 9 | 4 | 6 | 9 | 4 | 1 | 20 |
| | 0.31 | 1 | 3 | 1 | | 1 | 9 | 3 | 5 | 8 | 2 | 1 | |
| 40 | 1.25 | 5 | 4 | 1 | | 3 | 10 | 10 | 5 | 10 | 1 | 5 | 22 |
| | 0.31 | 4 | 1 | 1 | | 2 | 7 | 7 | 2 | 7 | 1 | 4 | |
| 41 | 1.25 | 1 | 3 | 1 | | 2 | 9 | 6 | 6 | 8 | 1 | 2 | 20 |
| | 0.31 | 1 | 2 | 1 | | 1 | 6 | 4 | 4 | 6 | 1 | 1 | |
| 42 | 1.25 | 7 | 10 | 10 | | 7 | 10 | 9 | 6 | 10 | 1 | 2 | 22 |
| | 0.31 | 6 | 10 | 6 | | 4 | 10 | 9 | 4 | 10 | 1 | 2 | |
| 43 | 1.25 | 4 | 9 | 6 | | 3 | 10 | 9 | 7 | 10 | 4 | 4 | 22 |
| | 0.31 | 4 | 9 | 4 | | 2 | 9 | 6 | 6 | 10 | 4 | 2 | |
| 45 | 1.25 | 3 | 3 | 3 | | 3 | 8 | 7 | 3 | 7 | 3 | 3 | 23 |
| 46 | 1.25 | 5 | 5 | 10 | 3 | | 10 | 10 | 6 | 10 | 10 | 3 | 26 |
| | 0.31 | 4 | 4 | 10 | 2 | | 9 | 8 | 4 | 10 | 10 | 1 | |

Table 3 (continued)

| Compound No. | Amount of Effective Ingredient (g/a) | OR | GL | ZE | GO | TR | XA | IP | SI | AM | EC | DI | Day* |
|---|---|---|---|---|---|---|---|---|---|---|---|---|---|
| 47 | 1.25 | 5 | 8 | 10 | 4 | | 9 | 7 | 6 | 9 | 9 | 5 | 17 |
| | 0.31 | 5 | 6 | 10 | 3 | | 9 | 6 | 5 | 9 | 8 | 4 | |
| 50 | 1.25 | 7 | 4 | 10 | 6 | | 10 | 10 | 6 | 10 | 10 | 4 | 21 |
| | 0.31 | 6 | 3 | 10 | 5 | | 10 | 10 | 5 | 10 | 10 | 3 | |
| 52 | 1.25 | 5 | 1 | 3 | 4 | | 7 | 6 | 6 | 9 | 4 | 6 | 21 |
| | 0.31 | 4 | 1 | 1 | 3 | | 6 | 3 | 5 | 7 | 3 | 5 | |
| 53 | 1.25 | 2 | 4 | 4 | 3 | | 10 | 4 | 3 | 6 | 4 | 2 | 26 |
| 55 | 1.25 | 4 | 3 | 3 | 4 | | 7 | 3 | 4 | 7 | 3 | 4 | 17 |
| 56 | 1.25 | 6 | 7 | 9 | 6 | | 10 | 4 | 6 | 9 | 6 | 6 | 17 |
| | 0.31 | 5 | 7 | 10 | 6 | | 9 | 3 | 5 | 7 | 5 | 6 | |
| 57 | 1.25 | 5 | 8 | 10 | 5 | | 7 | 6 | 4 | 9 | 8 | 3 | 21 |
| | 0.31 | 4 | 6 | 10 | 4 | | 7 | 5 | 4 | 8 | 7 | 2 | |
| 58 | 1.25 | 7 | 6 | 4 | | 6 | 6 | 6 | 2 | 6 | 3 | 6 | 22 |

* observation day after foliar application

Test Example 2

1/10,000 are (a) pots were filled with upland soil, which in the pots was then sown with a variety of plant seeds. The plants grown will be enumerated below.

| Name | Abbreviation |
|---|---|
| soybean (Glycine max)<br>indian mallow (Abutilon avicennae)<br>oriental senna (Cassia obtusifolia) | GL<br>AB<br>CA |

When each plant reached a given growth stage (a 0.1-leaf stage for soybean, a 1.1-leaf stage for indian mallow, and 0.1-leaf stage for oriental senna), a predetermined amount of the herbicide of the present invention in the form of a wettable powder, weighed out, diluted with 5 ℓ/a of water and admixed with 0.2 vol%, based on the resulting composition, of an agricultural spreader, was foliarly applied to the plant with a small spray gun. The progress of growth of the plant was visually observed 28 days after the foliar application to evaluate the degree of growth control according to the same 10 ratings as in Test Example 1. The results are listed in Table 4.

Table 4

| Compound No. | Amount of Effective Ingredient (g/a) | GL | AB | CA |
|---|---|---|---|---|
| 2 | 5<br>1.25 | 4<br>3 | 10<br>9 | 9-10<br>8 |

Test Example 3

1/3,000 are (a) pot and 1/10,000 are (a) pots were filled with upland soil, which in the pots was then sown with a variety of plant seeds. The plants grown will be enumerated below.

| pot | Name | Abbreviation |
|---|---|---|
| 1/3,000 a<br>1/10,000 a<br>1/10,000 a<br>1/10,000 a<br>1/10,000 a<br>1/10,000 a | soybean (Glycine max)<br>cocklebur (Xanthium strumarium)<br>morning glory (Ipomoea purpurea)<br>slender amaranth (Amaranthus viridis)<br>indian mallow (Abutilon avicennae)<br>oriental senna (Cassia obtusifolia) | GL<br>XA<br>IP<br>AM<br>AB<br>CA |

When each plant reached a given growth stage (a 0.5-leaf stage soybean, a 1.0-leaf stage for cocklebur, a 1.1-leaf stage for morning glory, a 1.4-leaf stage for slender amaranth, a 1.2-leaf stage for indian mallow, and a 0.2-leaf stage for oriental senna), a predetermined amount of the herbicide of the present invention in the form of a wettable powder, weighed out, diluted with 5 ℓ/a of water and admixed with 0.2 vol%, based on the resulting composition, of an agricultural spreader, was foliarly applied to the plant with a small spray gun. Visual observations on the soybean and the other plants were made on 18 days and 24 days, respectively after the foliar application to evaluate the degree of growth control according to the same 10 ratings as in Test Example 1. The results are listed in Table 5.

Table 5

| Compound No. | Amount of Effective Ingredient (g/a) | GL | XA | IP | AM | AB | CA |
|---|---|---|---|---|---|---|---|
| 18 | 1.25 | 2 | 9 | 8 | 6 | 9 | 6 |

Test Example 4

1/3,000 are (a) pot and 1/10,000 are (a)pots were filled with upland soil, which in the pots was then sown with a variety of plant seeds. The plants grown will be enumerated below.

| pot | Name | Abbreviation |
|---|---|---|
| 1/3,000 a | soybean (Glycine max) | GL |
| 1/10,000 a | cocklebur (Xanthium strumarium) | XA |
| 1/10,000 a | morning glory (Ipomoea purpurea) | IP |
| 1/10,000 a | slender amaranth (Amaranthus viridis) | AM |
| 1/10,000 a | indian mallow (Abutilon avicennae) | AB |
| 1/10,000 a | oriental senna (Cassia obtusifolia) | CA |

When each plant reached a given growth stage (a primary-leaf stage for soybean, a 2.0-leaf stage for cocklebur, a 1.2-leaf stage for morning glory,a 1.2-leaf stage for slender amaranth, a 1.3-leaf stage for indian mallow, and a 0.2-leaf stage for oriental senna), a predetermined amount of the herbicide of the present invention in the form of a wettable powder, weighed out diluted with 5 ℓ/a of water and admixed with 0.2 vol%, based on the resulting composition, of an agricultural spreader, was foliarly applied to the plant with a small spray gun. Visual observations on the soybean and the other plants were made on 20 days and 25 days, respectively after the foliar application to evaluate the degree of growth control according to the same 10 ratings as in Test Example 1. The results are listed in Table 6.

Table 6

| Compound No. | Amount of Effective Ingredient (g/a) | GL | XA | IP | AM | AB | CA |
|---|---|---|---|---|---|---|---|
| 22 | 1.25 | 3 | 9-10 | 9-10 | 7 | 10 | 6-7 |

Test Example 5

1/10,000 are (a) pots were filled with upland soil, which in the pots was then sown with a variety of plant seeds. The plants grown will be enumerated below.

| Name | Abbreviation |
|---|---|
| wheat (Triticum) | TR |
| wild oat (Avena fatua) | AV |

When each plant reached a given growth stage (a 2.1-leaf stage for wheat and a 1.0-leaf stage for wild oat), a predetermined amount of the herbicide of the present invention in the form of a wettable powder, weighed out, diluted with 5 ℓ/a of water and admixed with 0.2 vol%, based on the resulting composition, of an agricultural spreader, was foliarly applied to the plant with a small spray gun. The progress of growth of the plant was visually observed 19 days after the foliar application to evaluate the degree of growth control according to the same 10 ratings as in Test Example 1. The results are listed in Table 7.

Table 7

| Compound No. | Amount of Effective Ingredient (g/a) | TR | AV |
|---|---|---|---|
| 47 | 1.25 | 3 | 8-9 |
| | 0.31 | 2-3 | 8 |
| | 0.08 | 2 | 7 |

Test Example 6

1/10,000 are (a) pots were filled with upland soil, which in the pots was then sown with a variety of plant seeds. The plants grown will be enumerated below.

| Name | Abbreviation |
|---|---|
| cotton (Gossypium) | GO |
| oriental senna (Cassia obtusifolia) | CA |
| large crabgrass (Digitaria adscendens) | DI |

When each plant reached a given growth stage (a cotyledon stage for cotton, a 0.2-leaf stage for oriental senna, and a 2.9-leaf stage for large crab-grass), a predetermined amount of the herbicide of the present invention in the form of a wettable powder, weighed out, diluted with 5 ℓ/a of water and admixed with 0.2 vol%, based on the resulting composition, of an agricultural spreader, was foliarly applied to the plant with a small spray gun. The progress of growth of the plant was visually observed 28 days after the foliar application to evaluate the degree of growth control according to the same 10 ratings as in Test Example 1. The results are listed in Table 8.

Table 8

| Compound No. | Amount of Effective Ingredient (g/a) | GO | CA | DI |
|---|---|---|---|---|
| 19 | 1.25 | 3 | 8-9 | 8-9 |
| | 0.31 | 2 | 7-8 | 7-8 |
| | 0.08 | 1 | 6 | 6-7 |

Formulation Examples of the herbicide of the present invention will now be described.

Formulation Example 1

| | |
|---|---|
| (1) clay (trade name: Newlite) | 97 parts by weight |
| (2) a polyoxyethylene octylphenyl ether premixed with white carbon (trade name: Dikssol W-92) | 2 parts by weight |
| (3) Compound No. 18 | 1 part by weight |

The above-mentioned components are mixed together and pulverized to form a dust.

Formulation Example 2

| | |
|---|---|
| (1) Compound No.22 | 75 parts by weight |
| (2) a polycarboxylic acid type polymer (trade name: Demol EP powder) | 13.5 parts by weight |
| (3) NaCl | 10 parts by weight |
| (4) dextrin | 0.5 part by weight |
| (5) an alkyl sulfonate (trade name: TP-89121) | 1 part by weight |

The above-mentioned components are placed in a high-speed mixing granulator, admixed with 20 wt% of water, granulated, and dried to form water-dispersible granules.

Formulation Example 3

| | |
|---|---|
| (1) kaolin | 78 parts by weight |
| (2) a condensate of sodium naphthalenesulfonate and formalin (trade name: Lavelin FAN) | 2 parts by weight |
| (3) a sodium polyoxyethylene alkylaryl ether sulfate premixed with white carbon (Trade name: Sorpol 5039) | 5 parts by weight |
| (4) white carbon (trade name: Carplex) | 15 parts by weight |

A mixture of the above-mentioned components (1) to (4) is mixed with Compound No. 2 at a weight ratio of 9:1 to form a wettable powder.

Formulation Example 4

| | |
|---|---|
| (1) diatomaceous earth | 63 parts by weight |
| (2) a polyoxyethylene alkylphenyl ether sulfate ammonium salt premixed with white carbon (trade name: Dikssol W-66) | 5 parts by weight |
| (3) a dialkyl sulfosuccinate premixed with white carbon (trade name: Dikssol W-09B) | 2 parts by weight |
| (4) Compound No. 18 | 30 parts by weight |

The above-mentioned components are mixed together to form a wettable powder.

Formulation Example 5

| | |
|---|---|
| (1) talc micropowder (trade name: Hi-Filler No. 10) | 33 parts by weight |
| (2) a dialkyl sulfosuccinate premixed with white carbon (trade name: Sorpol 5050) | 3 parts by weight |
| (3) a mixture of a polyoxyethylene alkylarylether sulfate and a polyoxyethylene monomethyl ether carbonate, premixed with white carbon (trade name: Sorpol 5073) | 4 parts by weight |
| (4) Compound No. 22 | 60 parts by weight |

The above-mentioned components are mixed together to form a wettable powder.

Formulation Example 6

| | |
|---|---|
| (1) Compound No. 2 | 4 parts by weight |
| (2) corn oil | 79 parts by weight |
| (3) a mixture of a dialkyl sulfosuccinate, polyoxyethylene nonylphenyl ether, polyoxyethylene hydrogenated castor oil and polyglycerol esters of fatty acid (trade name: Sorpol 3815) | 15 parts by weight |
| (4) a bentonite-alkylamino complex | 2 parts by weight |

The above-mentioned components are uniformly mixed together and pulverized with a Dyno mill (manufactured by Willey et Barhofen Co.) to form a suspension concentrate.

While the invention has been described in detail and with reference to specific embodiments thereof, it will be apparent to one skilled in the art that various changes and modifications can be made therein without departing from the spirit and scope thereof.

## Claims

### Claims for the following Contracting States : AT, BE, CH, DE, DK, FR, GB, GR, IT, LI, LU, NL, MC, PT, SE

1. A substituted pyridinesulfonamide compound or its salt represented by the following general formula (I):

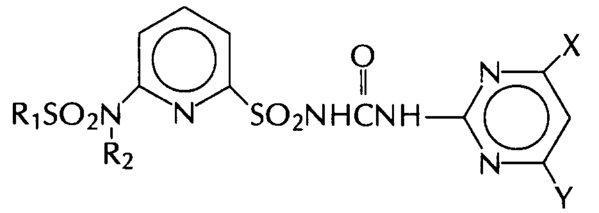

(I)

wherein $R_1$ and $R_2$ may be either each independently a member selected from the group consisting of $C_1$-$C_6$ alkyl optionally substituted by one or more substituents selected from the group consisting of halogen and $C_1$-$C_6$ alkoxy; $C_2$-$C_6$ alkenyl optionally substituted by one or more substituents selected from the group consisting of halogen and $C_1$-$C_6$ alkoxy; $C_3$-$C_6$ cycloalkyl optionally substituted by one or more substituents selected from the group consisting of halogen, $C_1$-$C_6$ alkyl and $C_1$-$C_6$ alkoxy; and phenyl optionally substituted by one or more substituents selected from the group consisting of halogen, $C_1$-$C_6$ alkyl, $C_1$-$C_6$ haloalkyl and a nitro group; or $R_1$ and $R_2$ are optionally combined with each other to form a $-(CH_2)_n-$ group wherein n is an integer of 2 to 5; and X and Y are each independently a member selected from the group consisting of $C_1$-$C_6$ alkyl and $C_1$-$C_6$ alkoxy.

2. A compound according to claim 1, wherein $R_1$ and $R_2$ are each independently a member selected from the group consisting of $C_1$-$C_6$ alkyl optionally substituted by one or more substituents selected from the group consisting of halogen and $C_1$-$C_6$ alkoxy; and $C_3$-$C_6$ cycloalkyl optionally substituted by one or more substituents selected from the group consisting of halogen, $C_1$-$C_6$ alkyl and $C_1$-$C_6$ alkoxy.

3. A compound according to claim 1, wherein $R_1$ and $R_2$ are each independently a member selected from the group consisting of $C_1$-$C_6$ alkyl, $C_1$-$C_6$ haloalkyl, and $C_3$-$C_6$ cycloalkyl.

4. 6-[(N-ethyl-N-methylsulfonyl)amino]-N-[[(4,6-dimethoxypyrimidin-2-yl)amino]carbonyl]-2-pyridinesulfonamide.

5. 6-[(N-ethyl-N-ethylsulfonyl)amino]-N-[[(4,6-dimethoxypyrimidin-2-yl)amino]carbonyl]-2-pyridinesulfonamide.

6. 6-[(N-ethyl-N-isopropylsulfonyl)amino]-N-[[(4,6-dimethoxypyrimidin-2-yl)amino]carbonyl]-2-pyridinesulfonamide.

7. A herbicide containing as the effective ingredient a substituted pyridinesulfonamide compound or its salt represented by the following general formula (I):

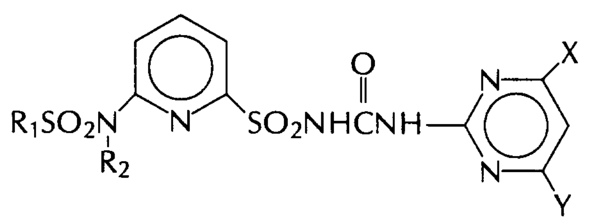

(I)

wherein $R_1$ and $R_2$ may be either each independently a member selected from the group consisting of $C_1$-$C_6$ alkyl optionally substituted by one or more substituents selected from the group consisting of halogen and $C_1$-$C_6$ alkoxy; $C_2$-$C_6$ alkenyl optionally substituted by one or more substituents selected from the group consisting of halogen and $C_1$-$C_6$ alkoxy; $C_3$-$C_6$ cycloalkyl optionally substituted by one

or more substituents selected from the group consisting of halogen, $C_1$-$C_6$ alkyl and $C_1$-$C_6$ alkoxy; and phenyl optionally substituted by one or more substituents selected from the group consisting of halogen, $C_1$-$C_6$ alkyl, $C_1$-$C_6$ haloalkyl and a nitro group; or $R_1$ and $R_2$ are optionally combined with each other to form a $-(CH_2)_n-$ group wherein n is an integer of 2 to 5; and X and Y are each independently a member selected from the group consisting of $C_1$-$C_6$ alkyl and $C_1$-$C_6$ alkoxy.

**8.** The herbicide according to claim 7, wherein $R_1$ and $R_2$ are each independently a member selected from the group consisting of $C_1$-$C_6$ alkyl optionally substituted by one or more substituents selected from the group consisting of halogen and $C_1$-$C_6$ alkoxy; and $C_3$-$C_6$ cycloalkyl optionally substituted by one or more substituents selected from the group consisting of halogen, $C_1$-$C_6$ alkyl and $C_1$-$C_6$ alkoxy.

**9.** The herbicide according to claim 7, wherein $R_1$ and $R_2$ are each independently a member selected from the group consisting of $C_1$-$C_6$ alkyl, $C_1$-$C_6$ haloalkyl and $C_3$-$C_6$ cycloalkyl.

**10.** The herbicide according to claim 7, wherein said effective ingredient is 6-[(N-ethyl-N-methylsulfonyl)-amino]-N-[[(4,6-dimethoxypyrimidin-2-yl)amino]-carbonyl]-2-pyridinesulfonamide.

**11.** The herbicide according to claim 7, wherein said effective ingredient is 6-[(N-ethyl-N-ethylsulfonyl)-amino]-N-[[(4,6-dimethoxypyrimidin-2-yl)amino]-carbonyl]-2-pyridinesulfonamide.

**12.** The herbicide according to claim 7, wherein said effective ingredient is 6-[(N-ethyl-N-isopropylsulfonyl)-amino]-N-[[(4,6-dimethoxypyrimidin-2-yl)amino]-carbonyl]-2-pyridinesulfonamide.

**13.** A herbicidal composition which comprises: a herbicidally effective amount of at least one compound selected from the group consisting of substituted pyridinesulfonamide compounds or their salts represented by the following general formula (I):

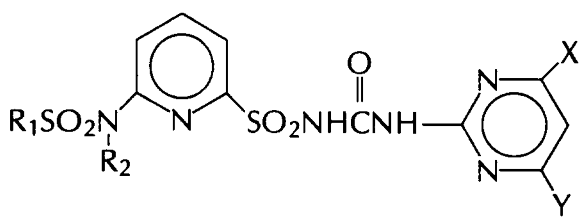

(I)

wherein $R_1$ and $R_2$ may be either each independently a member selected from the group consisting of $C_1$-$C_6$ alkyl optionally substituted by one or more substituents selected from the group consisting of halogen and $C_1$-$C_6$ alkoxy; $C_2$-$C_6$ alkenyl optionally substituted by one or more substituents selected from the group consisting of halogen and $C_1$-$C_6$ alkoxy; $C_3$-$C_6$ cycloalkyl optionally substituted by one or more substituents selected from the group consisting of halogen, $C_1$-$C_6$ alkyl and $C_1$-$C_6$ alkoxy; and phenyl optionally substituted by one or more substituents selected from the group consisting of halogen, $C_1$-$C_6$ alkyl, $C_1$-$C_6$ haloalkyl and a nitro group; or $R_1$ and $R_2$ are optionally combined with each other to form a $-(CH_2)_n-$ group wherein n is an integer of 2 to 5; and X and Y are each independently a member selected from the group consisting of $C_1$-$C_6$ alkyl and $C_1$-$C_6$ alkoxy;

a herbicidally effective amount of at least one compound selected from the group consisting of ethyl (±)-2-[4-[(6-chloro-2-quinoxalinyl)oxy]phenoxy]propionate, ethyl (±)-(-2-[4-[(6-chloro-2-benzoxazolyl)oxy]-phenoxy]propionate, butyl (±)-2-[4-[[5-(trifluoromethyl)-2-pyridinyl]oxy]phenoxy]propionate, methyl 2-[4-[[3-chloro-5-(trifluoromethyl)-2-pyridinyl]oxy]phenoxy]-propionate, 2-ethoxyethyl 2-[4-[[3-chloro-5-(trifluoromethyl)-2-pyridinyl]oxy]phenoxy]propionate, (R)-2-[[(1-methylethylidene)amino]oxy]ethyl 2-[4-[6-chloro-2-quinoxalinyl)oxy]phenoxy]propanoate, sodium 5-[2-chloro-4-(trifluoromethyl)phenoxy]-2-nitrobenzoate, (±)-2-ethoxy-1-methyl-2-oxoethyl-5-[2-chloro-4-(trifluoromethyl)phenoxy]-2-nitrobenzoate, 5-[2-chloro-4-(trifluoromethyl)phenoxy]-N-(methylsulfonyl)-2-nitrobenzamide, 2-chloro-N-(2-ethyl-6-methylphenyl)-N-(2-methoxy-1-methylethyl)acetamide, 2-chloro-N-(2,6-diethylphenyl)-N-(methoxymethyl)acetamide, (±)-2-[4,5-dihydro-4-methyl-4-(1-methylethyl)-5-oxo-1H-imidazol-2-yl]-5-ethyl-3-pyridinecarboxylic acid, 2-[4,5-dihydro-4-methyl-4-(1-methylethyl)-5-oxo-1H-imidazol-2-yl]-3-quinolinecarboxylic acid, 2,6-dinitro-N,N-dipropyl-4-(trifluoromethyl)aniline, N-(1-ethylpropyl)-3,4-dimethyl-2,6-dinitroaniline, 3-[(methoxycarbonyl)amino]phenyl (3-methylphenyl) carbamate, ethyl [3-[[-(phenylamino)carbonyl]oxy]phenyl]carbamate, 2-[1-(ethoxyimino)butyl]-5-[2-(ethylthio)propyl]-3-hydroxy-2-cyclohexen-1-one, 3-(1-methylethyl)-(1H)-2,1,3-benzothiadiazin-4(3H)-one-2,2-dioxide, ethyl 2-[[[[(4-chloro-6-methoxy-2-pyrimidinyl)amino]carbonyl]amino]sulfonyl]benzoate, 4-(2,4-dichlorophenoxy)-butanoic acid, 3-(3,4-dichlorophenyl)-1-methoxy-1-methylurea, 4-amino-6-(1,1-dimethylethyl)-3-(methylthio)-1,2,4-triazin-5(4H)-one, 7-oxabicyclo[2.2.1]heptane-2,3-dicarboxylic acid, S-ethyl dipropylcarbamothioate, (±)-2-ethoxy-2,3-dihydro-3,3-dimethyl-5-benzofuranyl methanesulfonate and 5-amino-4-chloro-2-phenyl-3(2H)-pyridazinone;
and one or more agricultural adjuvants.

**14.** A herbicidal composition which comprises:
a herbicidally effective amount of at least one compound selected from the group consisting of substituted pyridinesulfonamide compounds or their salts represented by the following general formula (I):

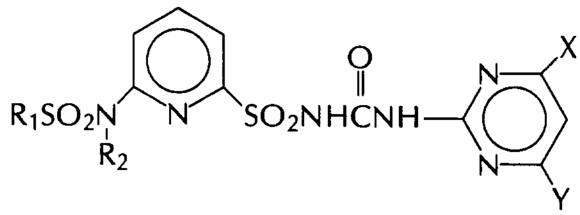

(I)

wherein $R_1$ and $R_2$ may be either each independently a member selected from the group consisting of $C_1$-$C_6$ alkyl optionally substituted by one or more substituents selected from the group consisting of halogen and $C_1$-$C_6$ alkoxy; $C_2$-$C_6$ alkenyl optionally substituted by one or more substituents selected from the group consisting of halogen and $C_1$-$C_6$ alkoxy; $C_3$-$C_6$ cycloalkyl optionally substituted by one or more substituents selected from the group consisting of halogen, $C_1$-$C_6$ alkyl and $C_1$-$C_6$ alkoxy; and phenyl optionally substituted by one or more substituents selected from the group consisting of halogen, $C_1$-$C_6$ alkyl, $C_1$-$C_6$ haloalkyl and a nitro group; or $R_1$ and $R_2$ are optionally combined with each other to form a $-(CH_2)_n-$ group wherein n is an integer of 2 to 5; and X and Y are each independently a member selected from the group consisting of $C_1$-$C_6$ alkyl and $C_1$-$C_6$ alkoxy;
a herbicidally effective amount of at least one compound selected from the group consisting of methyl 2-[[[[(4-methoxy-6-methyl-1,3,5-triazin-2-yl)amino]carbonyl]amino]sulfonyl]benzoate, methyl 2-[[-[[N-(4-methoxy-6-methyl-1,3,5-triazin-2-yl)methylamino]carbonyl]amino]sulfonyl]benzoate, 2-chloro-N-[[-(4-methoxy-6-methyl-1,3,5-triazin-2-yl]amino]carbonyl]benzenesulfonamide, methyl 3-[[[[(4-methoxy-6-methyl-1,3,5-triazin-2-yl]amino]carbonyl]amino]sulfonyl]-2-thiophenecarboxylate, methyl (±)-2-[4-(2,4-dichlorophenoxy)phenoxy]propionate, methyl 6-(4-isopropyl-4-methyl-5-oxo-2-imidazolin-2-yl)-m-toluate, methyl 2-(4-isopropyl-4-methyl-5-oxo-2-imidazolin-2-yl)-p-toluate, 1,2-dimethyl-3,5-diphenyl-1H-pyrazolium methylsulfate, 3,5-dibromo-4-hydroxybenzonitrile, 4-hydroxy-3,5-diiodobenzonitrile, 2,4-dichlorophenoxyacetic acid, S-(2,3,3-trichloro-2-propenyl)-bis(1-methylethyl)carbamothioate, 4-chloro-2-methylphenoxyacetic acid, 3,6-dichloro-2-pyridinecarboxylic acid, O-(6-chloro-3-phenyl-4-pyridazinyl)-S-octyl thiocarbonothioate, 3,6-dichloro-2-methoxybenzoic acid, N'-(3,4-dichlorophenyl)-N,N-dimethylurea and 4-amino-3,5,6-trichloro-2-pyridinecarboxylic acid; and
one or more agricultural adjuvants.

**15.** A herbicidal composition which comprises:
a herbicidally effective amount of at least one compound selected from the group consisting of substituted pyridinesulfonamide compounds or their salts represented by the following general formula (I):

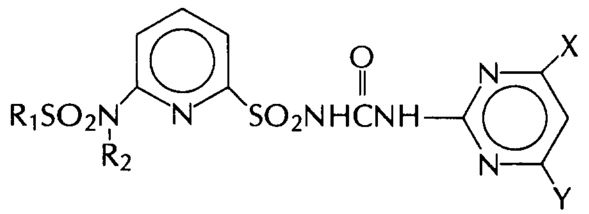

(I)

wherein $R_1$ and $R_2$ may be either each independently a member selected from the group consisting of $C_1$-$C_6$ alkyl optionally substituted by one or more substituents selected from the group consisting of halogen and $C_1$-$C_6$ alkoxy; $C_2$-$C_6$ alkenyl optionally substituted by one or more substituents selected from the group consisting of halogen and $C_1$-$C_6$ alkoxy; $C_3$-$C_6$ cycloalkyl optionally substituted by one or more substituents selected from the group consisting of halogen, $C_1$-$C_6$ alkyl and $C_1$-$C_6$ alkoxy; and phenyl optionally substituted by one or more substituents selected from the group consisting of halogen, $C_1$-$C_6$ alkyl, $C_1$-$C_6$ haloalkyl and a nitro group; or $R_1$ and $R_2$ are optionally combined with each other to form a $-(CH_2)_n-$ group wherein n is an integer of 2 to 5; and X and Y are each independently a member selected from the group consisting of $C_1$-$C_6$ alkyl and $C_1$-$C_6$ alkoxy;

a herbicidally effective amount of at least one compound selected from the group consisting of N,N-dimethyl N'-[3-(trifluoromethyl)phenyl]urea, 2-[[4-chloro-6-(ethylamino)-1,3,5-triazin-2-yl]amino]-2-methylpropionitrile, N,N'-bis(1-methylethyl)-6-(methylthio)-1,3,5-triazine-2,4-diamine, N-(3,4-dichlorophenyl)propanamide, disodium salt of methylarsonic acid, monosodium salt of methylarsonic acid, 2-chloro-1-(3-ethoxy-4-nitrophenoxy)-4-(trifluoromethyl)benzene, O,O-bis(1-methylethyl) S-[2-[-(phenylsulfonyl)amino]ethyl]phosphorodithioate, 2-(3,4-dichlorophenyl)-4-methyl-1,2,4-oxadiazolidine-3,5-dione, 4-chloro-5-(methyl-amino)-2-[3-(trifluoromethyl)phenyl]-3(2H)-pyridazinone, 2-chloro-6-(4,6-dimethoxypyrimidin-2-ylthio) benzoate, ethyl 2-chloro-6-(4,6-dimethoxypyrimidin-2-ylthio) benzoate and sodium 2-chloro-6-(4,6-dimethoxypyrimidin-2-ythio) benzoate; and

one or more agricultural adjuvants.

**16.** A herbicidal method which comprises applying to plants a herbicidally effective amount of a substituted pyridinesulfonamide compound or its salt represented by the following general formula (I):

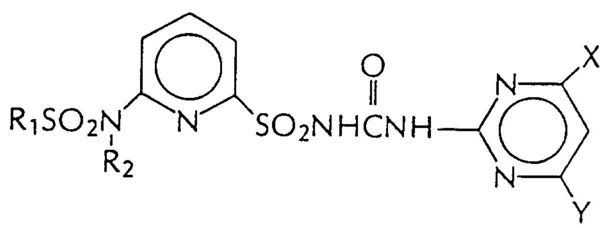

(I)

wherein $R_1$ and $R_2$ may be either each independently a member selected from the group consisting of $C_1$-$C_6$ alkyl optionally substituted by one or more substituents selected from the group consisting of halogen and $C_1$-$C_6$ alkoxy; $C_2$-$C_6$ alkenyl optionally substituted by one or more substituents selected from the group consisting of halogen and $C_{1-6}$ alkoxy; $C_3$-$C_6$ cycloalkyl optionally substituted by one or more substituents selected from the group consisting of halogen, $C_1$-$C_6$ alkyl and $C_1$-$C_6$ alkoxy; and phenyl optionally substituted by one or more substituents selected from the group consisting of halogen, $C_1$-$C_6$ alkyl, $C_1$-$C_6$ haloalkyl and a nitro group; or $R_1$ and $R_2$ are optionally combined with each other to form a - $(CH_2)_n$- group wherein n is an integer of 2 to 5; and X and Y are each independently a member selected from the group consisting of $C_1$-$C_6$ alkyl and $C_1$-$C_6$ alkoxy.

**17.** A process for preparing a substituted pyridinesulfonamide compound or its salt represented by the following general formula (I):

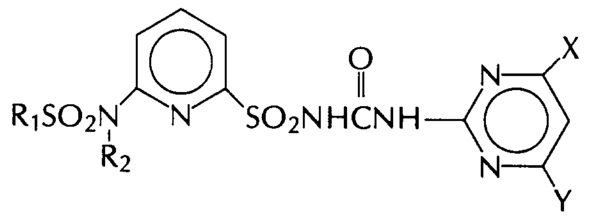

(I)

wherein $R_1$ and $R_2$ may be either each independently a member selected from the group consisting of $C_1$-$C_6$ alkyl optionally substituted by one or more substituents selected from the group consisting of halogen and $C_1$-$C_6$ alkoxy; $C_2$-$C_6$ alkenyl optionally substituted by one or more substituents selected from the group consisting of halogen and $C_1$-$C_6$ alkoxy; $C_3$-$C_6$ cycloalkyl optionally substituted by one or more substituents selected from the group consisting of halogen, $C_1$-$C_6$ alkyl and $C_1$-$C_6$ alkoxy; and phenyl optionally substituted by one or more substituents selected from the group consisting of halogen, $C_1$-$C_6$ alkyl, $C_1$-$C_6$ haloalkyl and a nitro group; or $R_1$ and $R_2$ are optionally combined with each other to form a $-(CH_2)_n-$ group wherein n is an integer of 2 to 5; and X and Y are each independently a member selected from the group consisting of $C_1$-$C_6$ alkyl and $C_1$-$C_6$ alkoxy,
which comprises reacting a substituted pyridine compound represented by the following general formula (II):

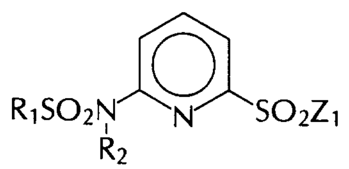

(II)

wherein $R_1$ and $R_2$ are the same as defined above; and $Z_1$ is a member selected from the group consisting of an $-NH_2$ group, an -NCO group, and an $-NHCO_2R_3$ group wherein $R_3$ is an alkyl or aryl group; with a pyrimidine compound represented by the following general formula (III):

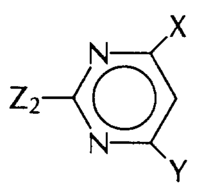

(III)

wherein X and Y are the same as defined above; and $Z_2$ is an $-NH_2$ group when $Z_1$ is an -NCO group or an $-NHCO_2R_3$ group, and is a member selected from the group consisting of an -NCO group and an $-NHCO_2R_3$ group wherein $R_3$ is the same as defined above, when $Z_1$ is an $-NH_2$ group.

**18.** A substituted pyridine compound represented by the following general formula (II-1):

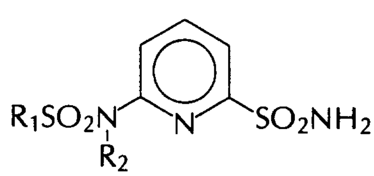

(II-1)

wherein $R_1$ and $R_2$ are either each independently a member selected from the group consisting of $C_1$-$C_6$ alkyl optionally substituted by one or more substituents selected from the group consisting of halogen and $C_1$-$C_6$ alkoxy; $C_2$-$C_6$ alkenyl optionally substituted by one or more substituents selected

from the group consisting of halogen and $C_1$-$C_6$ alkoxy; $C_3$-$C_6$ cycloalkyl optionally substituted by one or more substituents selected from the group consisting of halogen, $C_1$-$C_6$ alkyl and $C_1$-$C_6$ alkoxy; and phenyl optionally substituted by one or more substituents selected from the group consisting of halogen, $C_1$-$C_6$ alkyl, $C_1$-$C_6$ haloalkyl and a nitro group; or $R_1$ and $R_2$ are optionally combined with each other to form a $-(CH_2)_n-$ group wherein n is an integer of 2 to 5.

**Claims for the following Contracting State : ES**

**1.** A process for preparing a substituted pyridinesulfonamide compound or its salt represented by the following general formula (I):

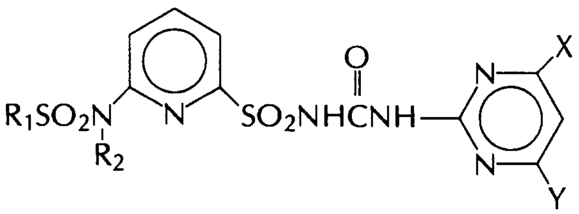

(I)

wherein $R_1$ and $R_2$ may be either each independently a member selected from the group consisting of $C_1$-$C_6$ alkyl optionally substituted by one or more substituents selected from the group consisting of halogen and $C_1$-$C_6$ alkoxy; $C_2$-$C_6$ alkenyl optionally substituted by one or more substituents selected from the group consisting of halogen and $C_1$-$C_6$ alkoxy; $C_3$-$C_6$ cycloalkyl optionally substituted by one or more substituents selected from the group consisting of halogen, $C_1$-$C_6$ alkyl and $C_1$-$C_6$ alkoxy; and phenyl optionally substituted by one or more substituents selected from the group consisting of halogen, $C_1$-$C_6$ alkyl, $C_1$-$C_6$ haloalkyl and a nitro group; or $R_1$ and $R_2$ are optionally combined with each other to form a $-(CH_2)_n-$ group wherein n is an integer of 2 to 5; and X and Y are each independently a member selected from the group consisting of $C_1$-$C_6$ alkyl and $C_1$-$C_6$ alkoxy,
which comprises reacting a substituted pyridine compound represented by the following general formula (II):

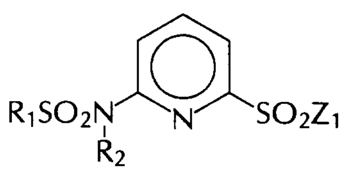

(II)

wherein $R_1$ and $R_2$ are the same as defined above; and $Z_1$ is a member selected from the group consisting of an $-NH_2$ group, an -NCO group and an $-NHCO_2R_3$ group wherein $R_3$ is an alkyl or aryl group;
with a pyrimidine compound represented by the following general formula (III):

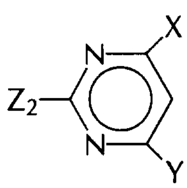

(III)

wherein X and Y are the same as defined above; and $Z_2$ is an $-NH_2$ group when $Z_1$ is an -NCO group or an $-NHCO_2R_3$ group, and is a member selected from the group consisting of an -NCO group and an $-NHCO_2R_3$ group wherein $R_3$ is the same as defined above, when $Z_1$ is an $-NH_2$ group.

2. A process according to claim 1, wherein $R_1$ and $R_2$ are each independently a member selected from the group consisting of $C_1$-$C_6$ alkyl optionally substituted by one or more substituents selected from the group consisting of halogen and $C_1$-$C_6$ alkoxy; and $C_3$-$C_6$ cycloalkyl optionally substituted by one or more substituents selected from the group consisting of halogen, $C_1$-$C_6$ alkyl and $C_1$-$C_6$ alkoxy.

3. A process according to claim 1, wherein $R_1$ and $R_2$ are each independently a member selected from the group consisting of $C_1$-$C_6$ alkyl, $C_1$-$C_6$ haloalkyl, and $C_3$-$C_6$ cycloalkyl.

4. A process according to claim 1, wherein the compound of the formula (I) is 6-[(N-ethyl-N-methylsulfonyl)amino]-N-[[(4,6-dimethoxypyrimidin-2-yl)amino]carbonyl]-2-pyridinesulfonamide.

5. A process according to claim 1, wherein the compound of the formula (I) is 6-[(N-ethyl-N-ethylsulfonyl)-amino]-N-[[(4,6-dimethoxypyrimidin-2-yl)amino]carbonyl]-2-pyridinesulfonamide.

6. A process according to claim 1, wherein the compound of the formula (I) is 6-[(N-ethyl-N-isopropylsulfonyl)amino]-N-[[(4,6-dimethoxypyrimidin-2-yl)amino]carbonyl]-2-pyridinesulfonamide.

7. A method of producing a herbicidal composition comprising mixing a substituted pyridinesulfonamide compound or its salt represented by the general formula (I) as defined in claim 1, as an active ingredient, with one or more agricultural adjuvants.

8. A method according to claim 7, wherein $R_1$ and $R_2$ in the formula (I) are each independently a member selected from the group consisting of $C_1$-$C_6$ alkyl optionally substituted by one or more substituents selected from the group consisting of halogen and $C_1$-$C_6$ alkoxy; and $C_3$-$C_6$ cycloalkyl optionally substituted by one or more substituents selected from the group consisting of halogen, $C_1$-$C_6$ alkyl and $C_1$-$C_6$ alkoxy.

9. A method according to claim 7, wherein $R_1$ and $R_2$ are each independently a member selected from the group consisting of $C_1$-$C_6$ alkyl, $C_1$-$C_6$ haloalkyl and $C_3$-$C_6$ cycloalkyl.

10. A method according to claim 7, wherein said effective ingredient is 6-[(N-ethyl-N-methylsulfonyl)amino]-N-[[(4,6-dimethoxypyrimidin-2-yl)amino]-carbonyl]-2-pyridinesulfonamide.

11. A method according to claim 7, wherein said effective ingredient is 6-[(N-ethyl-N-ethylsulfonyl)amino]-N-[[(4,6-dimethoxypyrimidin-2-yl)amino]-carbonyl]-2-pyridinesulfonamide.

12. A method according to claim 7, wherein said effective ingredient is 6-[(N-ethyl-N-isopropylsulfonyl)-amino]-N-[[(4,6-dimethoxypyrimidin-2-yl)amino]-carbonyl]-2-pyridinesulfonamide.

13. A method according to claim 7, wherein the herbicidal composition further comprises a herbicidally effective amount of at least one compound selected from the group consisting of ethyl (±)-2-[4-[(6-chloro-2-quinoxalinyl)oxy]phenoxy]propionate, ethyl (±)-(-2-[4-[(6-chloro-2-benzoxazolyl)oxy]-phenoxy]-propionate, butyl (±)-2-[4-[[5-(trifluoromethyl)-2-pyridinyl]oxy]phenoxy]propionate, methyl 2-[4-[[3-chloro-5-(trifluoromethyl)-2-pyridinyl]oxy]phenoxy]-propionate, 2-ethoxyethyl 2-[4-[[3-chloro-5-(trifluoromethyl)-2-pyridinyl]oxy]phenoxy]propionate, (R)-2-[[(1-methylethylidene)amino]oxy]ethyl 2-[4-[6-chloro-2-quinoxalinyl)oxy]phenoxy]propanoate, sodium 5-[2-chloro-4-(trifluoromethyl)phenoxy]-2-nitrobenzoate, (±)-2-ethoxy-1-methyl-2-oxoethyl-5-[2-chloro-4-(trifluoromethyl)phenoxy]-2-nitrobenzoate, 5-[2-chloro-4-(trifluoromethyl)phenoxy]-N-(methylsulfonyl)-2-nitrobenzamide, 2-chloro-N-(2-ethyl-6-methylphenyl)-N-(2-methoxy-1-methylethyl)acetamide, 2-chloro-N-(2,6-diethylphenyl)-N-(methoxymethyl)acetamide, (±)-2-[4,5-dihydro-4-methyl-4-(1-methylethyl)-5-oxo-1H-imidazol-2-yl]-5-ethyl-3-pyridinecarboxylic acid, 2-[4,5-dihydro-4-methyl-4-(1-methylethyl)-5-oxo-1H-imidazol-2-yl]-3-quinolinecarboxylic acid, 2,6-dinitro-N,N-dipropyl-4-(trifluoromethyl)aniline, N-(1-ethylpropyl)-3,4-dimethyl-2,6-dinitroaniline, 3-[-(methoxycarbonyl)amino]phenyl (3-methylphenyl)carbamate, ethyl [3-[[(phenylamino)carbonyl]oxy]-phenyl]carbamate, 2-[1-(ethoxyimino)butyl]-5-[2-(ethylthio)propyl]-3-hydroxy-2-cyclohexen-1-one, 3-(1-methylethyl)-(1H)-2,1,3-benzothiadiazin-4(3H)-one-2,2-dioxide, ethyl 2-[[[[(4-chloro-6-methoxy-2-pyrimidinyl)amino]carbonyl]amino]sulfonyl]benzoate, 4-(2,4-dichlorophenoxy)butanoic acid, 3-(3,4-dichlorophenyl)-1-methoxy-1-methylurea, 4-amino-6-(1,1-dimethylethyl)-3-(methylthio)-1,2,4-triazin-5(4H)-one, 7-oxabicyclo[2.2.1]heptane-2,3-dicarboxylic acid, S-ethyl dipropylcarbamothioate, (±)-2-ethoxy-2,3-dihydro-3,3-dimethyl-5-benzofuranyl methanesulfonate and 5-amino-4-chloro-2-phenyl-3(2H)-pyridazinone.

**14.** A method of producing a herbicidal composition which comprises mixing a herbicidally effective amount of at least one compound selected from the group consisting of substituted pyridinesulfonamide compounds or their salts represented by the following general formula (I) as defined in claim 1 with one or more agricultural adjuvants and

a herbicidally effective amount of at least one compound selected from the group consisting of methyl 2-[[[[(4-methoxy-6-methyl-1,3,5-triazin-2-yl)amino]carbonyl]amino]sulfonyl]benzoate, methyl 2-[[-[[N-(4-methoxy-6-methyl-1,3,5-triazin-2-yl)methylamino]carbonyl]amino]sulfonyl]benzoate, 2-chloro-N-[[-(4-methoxy-6-methyl-1,3,5-triazin-2-yl]amino]carbonyl]benzenesulfonamide, methyl 3-[[[[(4-methoxy-6-methyl-1,3,5-triazin-2-yl]amino]carbonyl]amino]sulfonyl]-2-thiophenecarboxylate, methyl (±)-2-[4-(2,4-dichlorophenoxy)phenoxy]propionate, methyl 6-(4-isopropyl-4-methyl-5-oxo-2-imidazolin-2-yl)-m-toluate, methyl 2-(4-isopropyl-4-methyl-5-oxo-2-imidazolin-2-yl)p-toluate, 1,2-dimethyl-3,5-diphenyl-1H-pyrazolium methylsulfate, 3,5-dibromo-4-hydroxybenzonitrile, 4-hydroxy-3,5-diiodobenzonitrile, 2,4-dichlorophenoxyacetic acid, S-(2,3,3-trichloro-2-propenyl)-bis(1-methylethyl)carbamothioate, 4-chloro-2-methylphenoxyacetic acid, 3,6-dichloro-2-pyridinecarboxylic acid, O-(6-chloro-3-phenyl-4-pyridazinyl)-S-octyl thiocarbonothioate, 3,6-dichloro-2-methoxybenzoic acid, N'-(3,4-dichlorophenyl)-N,N-dimethylurea and 4-amino-3,5,6-trichloro-2-pyridinecarboxylic acid; and

one or more agricultural adjuvants.

**15.** A method according to claim 7, wherein the composition further contains a herbicidally effective amount of at least one compound selected from the group consisting of N,N-dimethyl N'-[3-(trifluoromethyl)-phenyl]urea, 2-[[4-chloro-6-(ethylamino)-1,3,5-triazin-2-yl]amino]-2-methylpropionitrile, N,N'-bis(1-methylethyl)-6-(methylthio)-1,3,5-triazine-2,4-diamine, N-(3,4-dichlorophenyl)propanamide, disodium salt of methylarsonic acid, monosodium salt of methylarsonic acid, 2-chloro-1-(3-ethoxy-4-nitrophenoxy)-4-(trifluoromethyl)benzene, O,O-bis(1-methylethyl) S-[2-[(phenylsulfonyl)amino]ethyl]phosphorodithioate, 2-(3,4-dichlorophenyl)-4-methyl-1,2,4-oxadiazolidine-3,5-dione, 4-chloro-5-(methyl-amino)-2-[3-(trifluoromethyl)phenyl]-3(2H)-pyridazinone, 2-chloro-6-(4,6-dimethoxypyrimidin-2-ylthio) benzoate, ethyl 2-chloro-6-(4,6-dimethoxypyrimidin-2-ylthio) benzoate and sodium 2-chloro-6-(4,6-dimethoxypyrimidin-2-ylthio) benzoate; and

one or more agricultural adjuvants.

**16.** A herbicidal method which comprises applying to plants a herbicidally effective amount of a substituted pyridinesulfonamide compound or its salt represented by the general formula (I) as defined in claim 1.

**Patentansprüche**

**Patentansprüche für folgende Vertragsstaaten : AT, BE, CH, DE, DK, FR, GB, GR, IT, LI, LU, NL, MC, PT, SE**

**1.** Substituierte Pyridinsulfonamidverbindungen oder deren Salze, dargestellt durch die folgende allgemeine Formel (I):

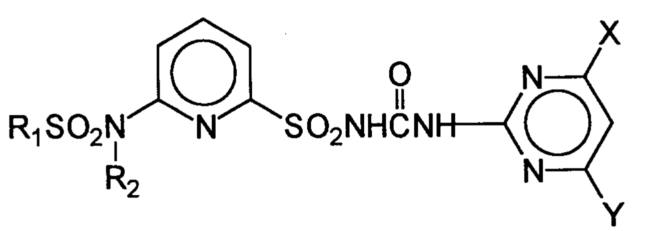

(I)

worin $R_1$ und $R_2$ entweder jeweils unabhängig ein Vertreter, ausgewählt aus der Gruppe, bestehend aus $C_{1-6}$-Alkyl, gegebenenfalls substituiert mit einem oder mehreren Substituenten aus der Gruppe, bestehend aus Halogen und $C_{1-6}$-Alkoxy; $C_{2-6}$-Alkenyl, gegebenenfalls substituiert mit einem oder

mehreren Substituenten aus der Gruppe, bestehend aus Halogen und $C_{1-6}$-Alkoxy; $C_{3-6}$-Cycloalkyl, gegebenenfalls substituiert mit einem oder mehreren Substituenten aus der Gruppe, bestehend aus Halogen, $C_{1-6}$-Alkyl und $C_{1-6}$-Alkoxy; und Phenyl, gegebenenfalls substituiert mit einem oder mehreren Substituenten aus der Gruppe, bestehend aus Halogen, $C_{1-6}$-Alkyl, $C_{1-6}$-Haloalkyl und einer Nitrogruppe, sind; oder $R_1$ und $R_2$ gegebenenfalls miteinander unter Bildung einer $-(CH_2)_n$-Gruppe kombiniert sind, wobei n eine ganze Zahl von 2 bis 5 ist; und X und Y jeweils unabhängig ein Vertreter aus der Gruppe, bestehend aus $C_{1-6}$-Alkyl und $C_{1-6}$-Alkoxy, sind.

**2.** Verbindung gemäss Anspruch 1, worin $R_1$ und $R_2$ jeweils unabhängig ein Vertreter aus der Gruppe, bestehend aus $C_{1-6}$-Alkyl, gegebenenfalls substituiert mit einem oder mehreren Substituenten aus der Gruppe, bestehend aus Halogen und $C_{1-6}$-Alkoxy; und $C_{3-6}$-Cycloalkyl, gegebenenfalls substituiert mit einem oder mehreren Substituenten aus der Gruppe, bestehend aus Halogen, $C_{1-6}$-Alkyl und $C_{1-6}$-Alkoxy, sind.

**3.** Verbindung gemäss Anspruch 1, worin $R_1$ und $R_2$ jeweils unabhängig ein Vertreter aus der Gruppe, bestehend aus $C_{1-6}$-Alkyl, $C_{1-6}$-Haloalkyl und $C_{3-6}$-Cycloalkyl sind.

**4.** 6-[(N-Ethyl-N-methylsulfonyl)amino]-N-[[(4,6-dimethoxypyrimidin-2-yl)amino]carbonyl]-2-pyridinsulfonamid.

**5.** 6-[(N-Ethyl-N-ethylsulfonyl)amino]-N-[[(4,6-dimethoxypyrimidin-2-yl)amino]carbonyl]-2-pyridinsulfonamid.

**6.** 6-[(N-Ethyl-N-isopropylsulfonyl)amino-]-N-[[(4,6-dimethoxypyrimidin-2-yl)amino]carbonyl]-2-pyridinsulfonamid.

**7.** Herbizid, enthaltend als wirksamen Bestandteil eine substituierte Pyridinsulfonamidverbindung oder deren Salz mit der folgenden allgemeinen Formel (I)

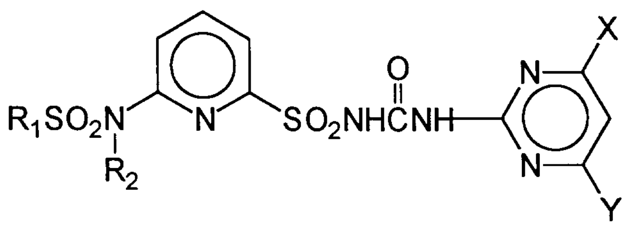

(I)

worin $R_1$ und $R_2$ entweder jeweils unabhängig ein Vertreter, ausgewählt aus der Gruppe, bestehend aus $C_{1-6}$-Alkyl, gegebenenfalls substituiert mit einem oder mehreren Substituenten aus der Gruppe, bestehend aus Halogen und $C_{1-6}$-Alkoxy; $C_{2-6}$-Alkenyl, gegebenenfalls substituiert mit einem oder mehreren Substituenten aus der Gruppe, bestehend aus Halogen und $C_{1-6}$-Alkoxy; $C_{3-6}$-Cycloalkyl, gegebenenfalls substituiert mit einem oder mehreren Substituenten aus der Gruppe, bestehend aus Halogen, $C_{1-6}$-Alkyl und $C_{1-6}$-Alkoxy; und Phenyl, gegebenenfalls substituiert mit einem oder mehreren Substituenten aus der Gruppe, bestehend aus Halogen, $C_{1-6}$-Alkyl, $C_{1-6}$-Haloalkyl und einer Nitrogruppe, sind; oder $R_1$ und $R_2$ gegebenenfalls miteinander unter Bildung einer $-(CH_2)_n$-Gruppe kombiniert sind, wobei n eine ganze Zahl von 2 bis 5 ist; und X und Y jeweils unabhängig ein Vertreter aus der Gruppe, bestehend aus $C_{1-6}$-Alkyl und $C_{1-6}$-Alkoxy, sind.

**8.** Herbizid nach Anspruch 7, worin $R_1$ und $R_2$ jeweils unabhängig ein Vertreter aus der Gruppe, bestehend aus $C_{1-6}$-Alkyl, gegebenenfalls substituiert mit einem oder mehreren Substituenten aus der Gruppe, bestehend aus Halogen und $C_{1-6}$-Alkoxy; und $C_{3-6}$-Cycloalkyl, gegebenenfalls substituiert mit einem oder mehreren Substituenten aus der Gruppe, bestehend aus Halogen, $C_{1-6}$-Alkyl und $C_{1-6}$-Alkoxy, sind.

**9.** Herbizid nach Anspruch 7, wobei $R_1$ und $R_2$ jeweils unabhängig ein Vertreter aus der Gruppe, bestehend aus $C_{1-6}$-Alkyl, $C_{1-6}$-Haloalkyl und $C_{3-6}$-Cycloalkyl sind.

**10.** Herbizid nach Anspruch 7, worin der wirksame Bestandteil 6-[(N-Ethyl-N-methylsulfonyl)amino]-N-[[(4,6-dimethoxypyrimidin-2-yl)amino]carbonyl]-2-pyridinsulfonamid ist.

**11.** Herbizid nach Anspruch 7, worin der wirksame Bestandteil 6-[(N-Ethyl-N-ethylsulfonyl)amino]-N-[[(4,6-dimethoxypyrimidin-2-yl)amino]carbonyl]-2-pyridinsulfonamid ist.

**12.** Herbizid nach Anspruch 7, worin der wirksame Bestandteil 6-[(N-Ethyl-N-isopropylsulfonyl)amino]-N-[[-(4,6-dimethoxypyrimidin-2-yl)amino]carbonyl]-2-pyridinsulfonamid ist.

**13.** Herbizidzusammensetzung, welche umfasst: eine herbizid wirksame Menge mindestens einer Verbindung, ausgewählt aus der Gruppe, bestehend aus substituierten Pyridinsulfonamidverbindungen oder deren Salzen mit der folgenden allgemeinen Formel (I)

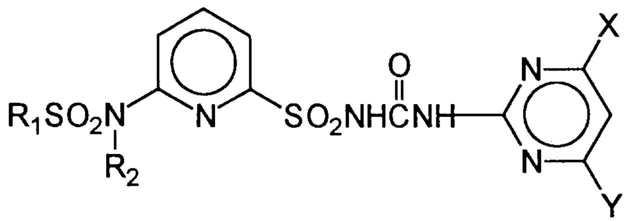

(I)

worin $R_1$ und $R_2$ entweder jeweils unabhängig ein Vertreter, ausgewählt aus der Gruppe, bestehend aus $C_{1-6}$-Alkyl, gegebenenfalls substituiert mit einem oder mehreren Substituenten aus der Gruppe, bestehend aus Halogen und $C_{1-6}$-Alkoxy; $C_{2-6}$-Alkenyl, gegebenenfalls substituiert mit einem oder mehreren Substituenten aus der Gruppe, bestehend aus Halogen und $C_{1-6}$-Alkoxy; $C_{3-6}$-Cycloalkyl, gegebenenfalls substituiert mit einem oder mehreren Substituenten aus der Gruppe bestehend aus Halogen, $C_{1-6}$-Alkyl und $C_{1-6}$-Alkoxy; und Phenyl, gegebenenfalls substituiert mit einem oder mehreren Substituenten aus der Gruppe, bestehend aus Halogen, $C_{1-6}$-Alkyl, $C_{1-6}$-Haloalkyl und einer Nitrogruppe, sind; oder $R_1$ und $R_2$ gegebenenfalls miteinander unter Bildung einer $-(CH_2)_n$-Gruppe kombiniert sind, wobei n eine ganze Zahl von 2 bis 5 ist; und X und Y jeweils unabhängig ein Vertreter aus der Gruppe, bestehend aus $C_{1-6}$-Alkyl und $C_{1-6}$-Alkoxy sind; eine herbizid wirksame Menge wenigstens einer Verbindung aus der Gruppe, bestehend aus:
Ethyl(±)-2-[4-[(6-chlor-2-chinoxalinyl)oxy]phenoxy]propionat, Ethyl(±)-2-[4-[(6-chlor-2-benzoxazolyl)oxy]-phenoxy]propionat, Butyl(±)-2-[4-[[5-(trifluormethyl)-2-pyridinyl]oxy]phenoxy]propionat, Methyl-2-[4-[[3-chlor-5-(trifluormethyl)-2-pyridinyl]oxy]phenoxy]propionat, 2-Ethoxyethyl-2-[4-[[3-chlor-5-(trifluormethyl)-2-pyridinyl]oxy]phenoxy]propionat, (R)-2[[(1-Methylethyliden)amino]oxy]ethyl-2-[4-[6-chlor-2-chinoxalinyl)oxy]phenoxy]propionat, Natrium-5-[2-chlor-4-(trifluormethyl)phenoxy]-2-nitrobenzoat, (±)-2-Ethoxy-1-methyl-2-oxoethyl-5-[2-chlor-4-(trifluormethyl)phenoxy]-2-nitrobenzoat, 5-[2-Chlor-4-(trifluormethyl)-phenoxy]-N-(methylsulfonyl)-2-nitrobenzamid, 2-Chlor-N-(2-ethyl-6-methylphenyl)-N-(2-methoxy-1-methylethyl)acetamid, 2-Chlor-N-(2,6-diethylphenyl)-N-(methoxymethyl)acetamid, (±)-2-[4,5-Dihydro-4-methyl-4-(1-methylethyl)-5-oxo-1H-imidazol-2-yl]-5-ethyl-3-pyridincarbonsäure, 2-[4,5-Dihydro-4-methyl-4-(1-methylethyl)-5-oxo-1H-imidazol-2-yl]-3-chinolincarbonsäure, 2,6-Dinitro-N,N-dipropyl-4-(trifluormethyl)anilin, N-(1-Ethylpropyl)-3,4-dimethyl-2,6-dinitroanilin, 3-[(Methoxycarbonyl)amino]phenyl-(3-methylphenyl)carbamat, Ethyl-[3-[[(phenylamino)carbonyl]oxy]phenyl]carbamat, 2-[1-(Ethoxyimino)butyl]-5-[2-(ethylthio)propyl]-3-hydroxy-2-cyclohexen-1-on, 3-(1-Methylethyl)-(1H)-2,1,3-benzothiadiazin-4(3H)-on-2,2-dioxid, Ethyl-2-[[[[(4-chlor-6-methoxy-2-pyrimidinyl)amino]carbonyl]amino]sulfonyl]benzoat, 4-(2,4-Dichlorphenoxy)butansäure, 3-(3,4-Dichlorphenyl)-1-methoxy-1-methylharnstoff, 4-Amino-6-(1,1-dimethylethyl)-3-(methylthio)-1,2,4-triazin-5(4H)-on, 7-Oxabicyclo[2.2.1]heptan-2,3-dicarbonsäure, S-Ethyldipropylcarbamothioat, (±)-2-Ethoxy-2,3-dihydro-3,3-dimethyl-5-benzofuranylmethansulfonat, 5-Amino-4-chlor-2-phenyl-3(2H)-pyridazinon und einen oder mehrere landwirtschaftliche Hilfsstoffe.

**14.** Herbizidzusammensetzung, welche umfasst:
eine herbizid wirksame Menge wenigstens einer Verbindung, die ausgewählt wird aus der Gruppe, bestehend aus substituierten Pyridinsulfonamidverbindungen oder ihren Salzen mit der folgenden allgemeinen Formel (I)

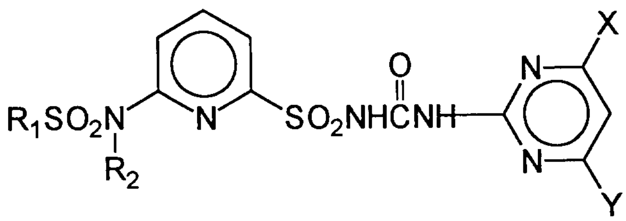

(I)

worin $R_1$ und $R_2$ entweder jeweils unabhängig ein Vertreter, ausgewählt aus der Gruppe, bestehend aus $C_{1-6}$-Alkyl, gegebenenfalls substituiert mit einem oder mehreren Substituenten aus der Gruppe, bestehend aus Halogen und $C_{1-6}$-Alkoxy; $C_{2-6}$-Alkenyl, gegebenenfalls substituiert mit einem oder mehreren Substituenten aus der Gruppe bestehend aus Halogen und $C_{1-6}$-Alkoxy; $C_{3-6}$-Cycloalkyl, gegebenenfalls substituiert mit einem oder mehreren Substituenten aus der Gruppe, bestehend aus Halogen, $C_{1-6}$-Alkyl und $C_{1-6}$-Alkoxy; und Phenyl, gegebenenfalls substituiert mit einem oder mehreren Substituenten aus der Gruppe, bestehend aus Halogen, $C_{1-6}$-Alkyl, $C_{1-6}$-Haloalkyl und einer Nitrogruppe, sind; oder $R_1$ und $R_2$ gegebenenfalls miteinander unter Bildung einer $-(CH_2)_n$-Gruppe kombiniert sind, wobei n eine ganze Zahl von 2 bis 5 ist; und X und Y jeweils unabhängig ein Vertreter aus der Gruppe, bestehend aus $C_{1-6}$-Alkyl und $C_{1-6}$-Alkoxy, sind,
eine herbizid wirksame Menge wenigstens einer Verbindung, die ausgewählt wird aus der Gruppe, bestehend aus Methyl-2-[[[[(4-methoxy-6-methyl-1,3,5-triazin-2-yl)amino]carbonyl]amino]sulfonyl]-benzoat, Methyl-2-[[[[N-(4-methoxy-6-methyl-1,3,5-triazin-2-yl)methylamino]carbonyl]amino]sulfonyl]-benzoat, 2-Chlor-N-[[(4-methoxy-6-methyl-1,3,5-triazin-2-yl]amino]carbonyl]benzolsulfonamid, Methyl-3-[[[[(4-methoxy-6-methyl-1,3,5-triazin-2-yl)amino]carbonyl]amino]sulfonyl]-2-thiophencarboxylat, Methyl-(±)-2-[4-(2,4-dichlorphenoxy)phenoxy]propionat, Methyl-6-(4-isopropyl-4-methyl-5-oxo-2-imidazolin-2-yl)-m-toluat, Methyl-2-(4-isopropyl-4-methyl-5-oxo-2-imidazolin-2-yl)-p-toluat, 1,2-Dimethyl-3,5-diphenyl-1H-pyrazoliummethylsulfat, 3,5-Dibrom-4-hydroxybenzonitril, 4-Hydroxy-3,5-diiodobenzonitril, 2,4-Dichlorphenoxyessigsäure, S-(2,3,3-Trichlor-2-propenyl)-bis(1-methylethyl)carbamothioat, 4-Chlor-2-methylphenoxyessigsäure, 3,6-Dichlor-2-pyridincarbonsäure, O-(6-Chlor-3-phenyl-4-pyridazinyl)-S-octylthiocarbonothioat, 3,6-Dichlor-2-methoxybenzoesäure, N'-(3,4-Dichlorphenyl)-N,N-dimethylharnstoff und 4-Amino-3,5,6-trichlor-2-pyridincarbonsäure; und
einen oder mehrere landwirtschaftliche Hilfsstoffe.

**15.** Herbizidzusammensetzung, welche umfasst: eine herbizid wirksame Menge wenigstens einer Verbindung, die ausgewählt wird aus der Gruppe, bestehend aus substituierten Pyridinsulfonamidverbindungen oder deren Salzen mit der folgenden allgemeinen Formel (I)

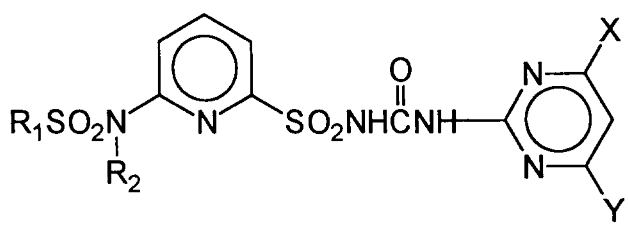

(I)

worin $R_1$ und $R_2$ entweder jeweils unabhängig ein Vertreter, ausgewählt aus der Gruppe bestehend aus

$C_{1-6}$-Alkyl, gegebenenfalls substituiert mit einem oder mehreren Substituenten aus der Gruppe, bestehend aus Halogen und $C_{1-6}$-Alkoxy; $C_{2-6}$-Alkenyl, gegebenenfalls substituiert mit einem oder mehreren Substituenten aus der Gruppe, bestehend aus Halogen und $C_{1-6}$-Alkoxy; $C_{3-6}$-Cycloalkyl, gegebenenfalls substituiert mit einem oder mehreren Substituenten aus der Gruppe, bestehend aus Halogen, $C_{1-6}$-Alkyl und $C_{1-6}$-Alkoxy; und Phenyl, gegebenenfalls substituiert mit einem oder mehreren Substituenten aus der Gruppe, bestehend aus Halogen, $C_{1-6}$-Alkyl, $C_{1-6}$-Haloalkyl und einer Nitrogruppe, sind; oder $R_1$ und $R_2$ gegebenenfalls miteinander unter Bildung einer $-(CH_2)_n$-Gruppe kombiniert sind, wobei n eine ganze Zahl von 2 bis 5 ist; und X und Y jeweils unabhängig ein Vertreter aus der Gruppe, bestehend aus $C_{1-6}$-Alkyl und $C_{1-6}$-Alkoxy sind;
eine herbizid wirksame Menge wenigstens einer Verbindung, die ausgewählt wird aus der Gruppe, bestehend aus N,N-Dimethyl-N'-[3-(trifluormethyl)phenyl]harnstoff, 2-[[4-Chlor-6-(ethylamino)-1,3,5-triazin-2-yl]amino-2-methylpropionitril, N,N'-Bis(1-methylethyl)-6-(methylthio)-1,3,5-triazin-2,4-diamin, N-(3,4-Dichlorphenyl)propanamid, Methylarsonsäure-Dinatriumsalz, Methylarsonsäure-Mononatriumsalz, 2-Chlor-1-(3-ethoxy-4-nitrophenoxy)-4-trifluormethyl)benzol, O,O-Bis(1-methylethyl)-S-[2-[(phenylsulfonyl)-amino]ethyl]phosphordithioat, 2-(3,4-Dichlorphenyl)-4-methyl-1,2,4-oxadiazolidin-3,5-dion, 4-Chlor-5-(methylamino)-2-[3-(trifluormethyl)phenyl]-3(2H)-pyridazinon, 2-Chlor-6-(4,6-dimethoxypyrimidin-2-ylthio)benzoat, Ethyl-2-chlor-6-(4,6-dimethoxypyrimidin-2-ylthio)benzoat und Natrium-2-chlor-6-(4,6-dimethoxypyrimidin-2-ylthio)benzoat; und
einen oder mehrere landwirtschaftliche Hilfsstoffe.

**16.** Unkrautbekämpfungsverfahren, umfassend das Aufbringen auf Pflanzen von einer herbizid wirksamen Menge einer substituierten Pyridinsulfonamidverbindung oder deren Salz mit der folgenden allgemeinen Formel (I)

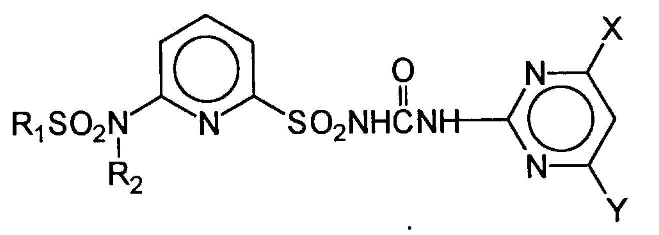

(I)

worin $R_1$ und $R_2$ entweder jeweils unabhängig ein Vertreter, ausgewählt aus der Gruppe, bestehend aus $C_{1-6}$-Alkyl, gegebenenfalls substituiert mit einem oder mehreren Substituenten aus der Gruppe, bestehend aus Halogen und $C_{1-6}$-Alkoxy; $C_{2-6}$-Alkenyl, gegebenenfalls substituiert mit einem oder mehreren Substituenten aus der Gruppe, bestehend aus Halogen und $C_{1-6}$-Alkoxy; $C_{3-6}$-Cycloalkyl, gegebenenfalls substituiert mit einem oder mehreren Substituenten aus der Gruppe, bestehend aus Halogen, $C_{1-6}$-Alkyl und $C_{1-6}$-Alkoxy; und Phenyl, gegebenenfalls substituiert mit einem oder mehreren Substituenten aus der Gruppe, bestehend aus Halogen, $C_{1-6}$-Alkyl, $C_{1-6}$-Haloalkyl und einer Nitrogruppe, sind; oder $R_1$ und $R_2$ gegebenenfalls miteinander unter Bildung einer $-(CH_2)_n$-Gruppe kombiniert sind, wobei n eine ganze Zahl von 2 bis 5 ist; und X und Y jeweils unabhängig ein Vertreter aus der Gruppe, bestehend aus $C_{1-6}$-Alkyl und $C_{1-6}$-Alkoxy, sind.

**17.** Verfahren zur Herstellung einer substituierten Pyridinsulfonamidverbindung oder deren Salz mit der folgenden allgemeinen Formel (I):

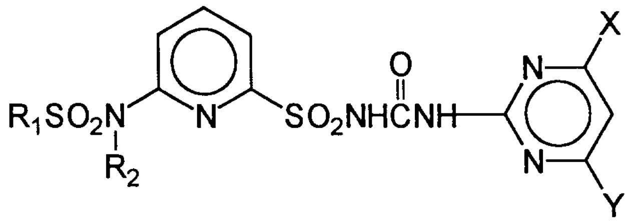

(I)

worin $R_1$ und $R_2$ entweder jeweils unabhängig ein Vertreter, ausgewählt aus der Gruppe, bestehend aus $C_{1-6}$-Alkyl, gegebenenfalls substituiert mit einem oder mehreren Substituenten aus der Gruppe, bestehend aus Halogen und $C_{1-6}$-Alkoxy; $C_{2-6}$-Alkenyl, gegebenenfalls substituiert mit einem oder mehreren Substituenten aus der Gruppe, bestehend aus Halogen und $C_{1-6}$-Alkoxy; $C_{3-6}$-Cycloalkyl, gegebenenfalls substituiert mit einem oder mehreren Substituenten aus der Gruppe, bestehend aus Halogen, $C_{1-6}$-Alkyl und $C_{1-6}$-Alkoxy; und Phenyl, gegebenenfalls substituiert mit einem oder mehreren Substituenten aus der Gruppe, bestehend aus Halogen, $C_{1-6}$-Alkyl, $C_{1-6}$-Haloalkyl und einer Nitrogruppe, sind; oder $R_1$ und $R_2$ gegebenenfalls miteinander unter Bildung einer $-(CH_2)_n$-Gruppe kombiniert sind, wobei n eine ganze Zahl von 2 bis 5 ist; und X und Y jeweils unabhängig ein Vertreter aus der Gruppe, bestehend aus $C_{1-6}$-Alkyl und $C_{1-6}$-Alkoxy sind,
umfassend die Umsetzung einer substituierten Pyridinverbindung mit der folgenden allgemeinen Formel (II)

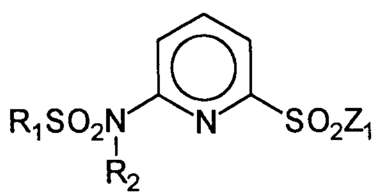

(II)

worin $R_1$ und $R_2$ wie oben definiert sind; und $Z_1$ ein Vertreter aus der Gruppe, bestehend aus einer $-NH_2$-Gruppe, einer -NCO-Gruppe und einer $-NHCO_2R_3$-Gruppe ist, wobei $R_3$ eine Alkyl- oder Arylgruppe ist;
mit einer Pyrimidinverbindung der folgenden allgemeinen Formel (III)

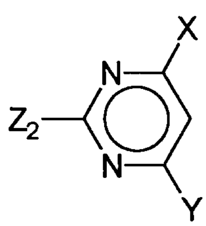

(III)

worin X und Y wie oben definiert sind; und $Z_2$ eine $-NR_2$-Gruppe ist, wenn $Z_1$ eine -NCO- oder eine $-NHCO_2R_3$-Gruppe, ist, und ein Vertreter aus der Gruppe, bestehend aus einer -NCO-Gruppe und einer $-NHCO_2R_3$-Gruppe ist, wobei $R_3$ wie oben definiert ist, wenn $Z_1$ eine $-NR_2$-Gruppe ist.

**18.** Substituierte Pyridinverbindung mit der folgenden allgemeinen Formel (II-1)

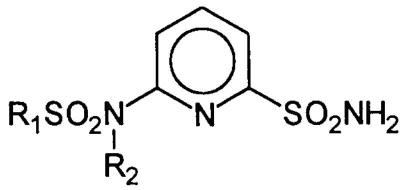

(II-1)

worin $R_1$ und $R_2$ entweder jeweils unabhängig ein Vertreter aus der Gruppe, bestehend aus $C_{1-6}$-Alkyl, gegebenenfalls substituiert mit einem oder mehreren Substituenten aus der Gruppe, bestehend aus Halogen und $C_{1-6}$-Alkoxy; $C_{2-6}$-Alkenyl, gegebenenfalls substituiert mit einem oder mehreren Substituenten aus der Gruppe bestehend aus Halogen und $C_{1-6}$-Alkoxy; $C_{3-6}$-Cycloalkyl, gegebenenfalls substituiert mit einem oder mehreren Substituenten aus der Gruppe, bestehend aus Halogen, $C_{1-6}$-Alkyl und $C_{1-6}$-Alkoxy; und Phenyl, gegebenenfalls substituiert mit einem oder mehreren Substituenten aus der Gruppe, bestehend aus Halogen, $C_{1-6}$-Alkyl, $C_{1-6}$-Haloalkyl und einer Nitrogruppe, sind; oder $R_1$ und $R_2$ gegebenenfalls miteinander unter Bildung einer $-(CH_2)_n$-Gruppe kombiniert sind, wobei n eine ganze Zahl von 2 bis 5 ist.

**Patentansprüche für folgenden Vertragstaat : ES**

**1.** Verfahren zur Herstellung einer substituierten Pyridinsulfonamidverbindung oder deren Salz mit der folgenden allgemeinen Formel (I):

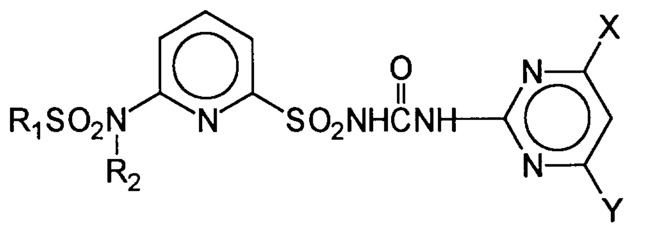

(I)

worin $R_1$ und $R_2$ entweder jeweils unabhängig ein Vertreter, ausgewählt aus der Gruppe, bestehend aus $C_{1-6}$-Alkyl, gegebenenfalls substituiert mit einem oder mehreren Substituenten aus der Gruppe, bestehend aus Halogen und $C_{1-6}$-Alkoxy; $C_{2-6}$-Alkenyl, gegebenenfalls substituiert mit einem oder mehreren Substituenten aus der Gruppe, bestehend aus Halogen und $C_{1-6}$-Alkoxy; $C_{3-6}$-Cycloalkyl, gegebenenfalls substituiert mit einem oder mehreren Substituenten aus der Gruppe, bestehend aus Halogen, $C_{1-6}$-Alkyl und $C_{1-6}$-Alkoxy; und Phenyl, gegebenenfalls substituiert mit einem oder mehreren Substituenten aus der Gruppe, bestehend aus Halogen, $C_{1-6}$-Alkyl, $C_{1-6}$-Haloalkyl und einer Nitrogruppe, sind; oder $R_1$ und $R_2$ gegebenenfalls miteinander unter Bildung einer $-(CH_2)_n$-Gruppe kombiniert sind, wobei n eine ganze Zahl von 2 bis 5 ist; und X und Y jeweils unabhängig ein Vertreter aus der Gruppe, bestehend aus $C_{1-6}$-Alkyl und $C_{1-6}$-Alkoxy sind, umfassend die Umsetzung einer substituierten Pyridinverbindung mit der folgenden allgemeinen Formel (II)

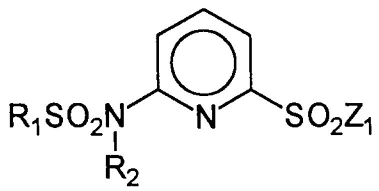

(II)

worin $R_1$ und $R_2$ wie oben definiert sind; und $Z_1$ ein Vertreter aus der Gruppe, bestehend aus einer $-NH_2$-Gruppe, einer -NCO-Gruppe und einer $-NHCO_2R_3$-Gruppe ist, wobei $R_3$ eine Alkyl- oder Arylgruppe ist;
mit einer Pyrimidinverbindung der folgenden allgemeinen Formel (III)

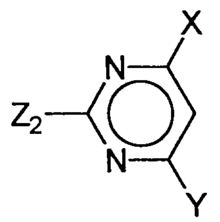

(III)

worin X und Y wie oben definiert sind; und $Z_2$ eine $-NH_2$-Gruppe ist, wenn $Z_1$ eine -NCO- oder eine $-NHCO_2R_3$-Gruppe ist, und ein Vertreter aus der Gruppe, bestehend aus einer -NCO-Gruppe und einer $-NHCO_2R_3$-Gruppe, ist, wobei $R_3$ wie oben definiert ist, wenn $Z_1$ eine $-NH_2$-Gruppe ist.

**2.** Verfahren nach Anspruch 1, wobei $R_1$ und $R_2$ jeweils unabhängig ein Vertreter aus der Gruppe, bestehend aus $C_{1-6}$-Alkyl, gegebenenfalls substituiert mit einem oder mehreren Substituenten aus der Gruppe, bestehend aus Halogen und $C_{1-6}$-Alkoxy; und $C_{3-6}$-Cycloalkyl, gegebenenfalls substituiert mit einem oder mehreren Substituenten aus der Gruppe, bestehend aus Halogen, $C_{1-6}$-Alkyl und $C_{1-6}$-Alkoxy, sind.

**3.** Verfahren gemäss Anspruch 1, worin $R_1$ und $R_2$ jeweils unabhängig ein Vertreter aus der Gruppe, bestehend aus $C_{1-6}$-Alkyl, $C_{1-6}$-Haloalkyl und $C_{3-6}$-Cycloalkyl sind.

**4.** Verfahren gemäss Anspruch 1, worin die Verbindung der Formel (I) 6-[(N-Ethyl-N-methylsulfonyl)-amino]-N-[[(4,6-dimethoxypyrimidin-2-yl)amino]carbonyl]-2-pyridinsulfonamid ist.

**5.** Verfahren gemäss Anspruch 1, worin die Verbindung der Formel (I) 6-[(N-Ethyl-N-ethylsulfonyl)amino]-N-[[(4,6-dimethoxypyrimidin-2-yl)amino]carbonyl]-2-pyridinsulfonamid ist.

**6.** Verfahren gemäss Anspruch 1, worin die Verbindung der Formel (I) 6-[(N-Ethyl-N-isopropylsulfonyl)-amino]-N-[[(4,6-dimethoxypyrimidin-2-yl)amino]carbonyl]-2-pyridinsulfonamid ist.

**7.** Verfahren zur Herstellung einer Herbizidzusammensetzung, umfassend die Mischung einer substituierten Pyridinsulfonamidverbindung oder deren Salz mit der allgemeinen Formel (I) gemäss Anspruch 1 als aktiven Bestandteil mit einem oder mehreren landwirtschaftlichen Hilfsstoffen.

**8.** Verfahren gemäss Anspruch 7, wobei $R_1$ und $R_2$ in der Formel (I) jeweils unabhängig ein Vertreter aus der Gruppe, bestehend aus $C_{1-6}$-Alkyl, gegebenenfalls substituiert mit einem oder mehreren Substituenten aus der Gruppe, bestehend aus Halogen und $C_{1-6}$-Alkoxy; und $C_{3-6}$-Cycloalkyl, gegebenenfalls substituiert mit einem oder mehreren Substituenten aus der Gruppe, bestehend aus Halogen, $C_{1-6}$-Alkyl und $C_{1-6}$-Alkoxy, sind.

9. Verfahren nach Anspruch 7, wobei $R_1$ und $R_2$ jeweils unabhängig ein Vertreter aus der Gruppe, bestehend aus $C_{1-6}$-Alkyl, $C_{1-6}$-Haloalkyl und $C_{3-6}$-Cycloalkyl sind.

10. Verfahren nach Anspruch 7, worin der wirksame Bestandteil 6-[(N-Ethyl-N-methylsulfonyl)amino]-N-[[-(4,6-dimethoxypyrimidin-2-yl)amino]carbonyl]-2-pyridinsulfonamid ist.

11. Verfahren nach Anspruch 7, worin der wirksame Bestandteil 6-[(N-Ethyl-N-ethylsulfonyl)amino]-N-[[(4,6-dimethoxypyrimidin-2-yl)amino]carbonyl]-2-pyridinsulfonamid ist.

12. Verfahren nach Anspruch 7, worin der wirksame Bestandteil 6-[(N-Ethyl-N-isopropylsulfonyl)amino]-N-[[-(4,6-dimethoxypyrimidin-2-yl)amino]carbonyl]-2-pyridinsulfonamid ist.

13. Verfahren gemäss Anspruch 7, worin die Herbizidzusammensetzung ausserdem eine herbizid wirksame Menge wenigstens einer Verbindung umfasst, welche ausgewählt wird aus der Gruppe, bestehend aus Ethyl(±)-2-[4-[(6-chlor-2-chinoxalinyl)oxy]phenoxy]propionat, Ethyl(±)-2-[4-[(6-chlor-2-benzoxazolyl)oxy]-phenoxy]propionat, Butyl(±)-2-[4-[[5-(trifluormethyl)-2-pyridinyl]oxy]phenoxy]propionat, Methyl-2-[4-[[3-chlor-5-(trifluormethyl)-2-pyridinyl]oxy]phenoxy]propionat, 2-Ethoxyethyl-2-[4-[[3-chlor-5-(trifluormethyl)-2-pyridinyl]oxy]phenoxy]propionat, (R)-2[[(1-Methylethyliden)amino]oxy]ethyl-2-[4-[6-chlor-2-chinoxalinyl)oxy]phenoxy]propionat, Natrium-5-[2-chlor-4-(trifluormethyl)phenoxy]-2-nitrobenzoat, (±)-2-Ethoxy-1-methyl-2-oxoethyl-5-[2-chlor-4-(trifluormethyl)phenoxy]-2-nitrobenzoat, 5-[2-Chlor-4-(trifluormethyl)-phenoxy]-N-(methyl-sulfonyl)-2-nitrobenzamid, 2-Chlor-N-(2-ethyl-6-methylphenyl)-N-(2-methoxy-1-methylethyl)acetamid, 2-Chlor-N-(2,6-diethylphenyl)-N-(methoxymethyl)acetamid, (±)-2-[4,5-Dihydro-4-methyl-4-(1-methylethyl)-5-oxo-1H-imidazol-2-yl]-5-ethyl-3-pyridincarbonsäure, 2-[4,5-Dihydro-4-methyl-4-(1-methylethyl)-5-oxo-1H-imidazol-2-yl]-3-chinolincarbonsäure, 2,6-Dinitro-N,N-dipropyl-4-(trifluormethyl)anilin, N-(1-Ethylpropyl)-3,4-dimethyl-2,6-dinitroanilin, 3-[(Methoxycarbonyl)amino]phenyl-(3-methylphenyl)carbamat, Ethyl-[3-[[(phenylamino)carbonyl]oxy]phenyl]carbamat, 2-[1-(Ethoxyimino)butyl]-5-[2-(ethylthio)propyl]-3-hydroxy-2-cyclohexen-1-on, 3-(1-Methylethyl)-(1H)-2,1,3-benzothiadiazin-4(3H)-on-2,2-dioxid, Ethyl-2-[[[[(4-chlor-6-methoxy-2-pyrimidinyl)amino]carbonyl]amino]sulfonyl]benzoat, 4-(2,4-Dichlorphenoxy)butansäure, 3-(3,4-Dichlorphenyl)-1-methoxy-1-methylharnstoff, 4-Amino-6-(1,1-dimethylethyl)-3-(methylthio)-1,2,4-triazin-5(4H)-on, 7-Oxabicyclo[2.2.1]heptan-2,3-dicarbonsäure, S-Ethyldipropylcarbamothioat, (±)-2-Ethoxy-2,3-dihydro-3,3-dimethyl-5-benzofuranylmethansulfonat und 5-Amino-4-chlor-2-phenyl-3(2H)-pyridazinon.

14. Verfahren zur Herstellung einer Herbizidzusammensetzung, umfassend die Mischung einer herbizid wirksamen Menge wenigstens einer Verbindung aus der Gruppe, bestehend aus substituierten Pyridinsulfonamidverbindungen oder ihren Salzen mit der folgenden allgemeinen Formel (I) gemäss Anspruch 1 mit einem oder mehreren landwirtschaftlichen Hilfsstoffen und
einer herbizid wirksamen Menge wenigstens einer Verbindung aus der Gruppe, bestehend aus Methyl-2-[[[[(4-methoxy-6-methyl-1,3,5-triazin-2-yl)amino]carbonyl]amino]sulfonyl]benzoat, Methyl-2-[[[[N-(4-methoxy-6-methyl-1,3,5-triazin-2-yl)methylamino]carbonyl]amino]sulfonyl]benzoat, 2-Chlor-N-[[(4-methoxy-6-methyl-1,3,5-triazin-2-yl]amino]carbonyl]benzolsulfanamid, Methyl-3-[[[[(4-methoxy-6-methyl-1,3,5-triazin-2-yl)amino]carbonyl]amino]sulfonyl]-2-thiophencarboxylat, Methyl-(±)-2-[4-(2,4-dichlorphenoxy)phenoxy]propionat, Methyl-6-(4-isopropyl-4-methyl-5-oxo-2-imidazolin-2-yl)-m-toluat, Methyl-2-(4-isopropyl-4-methyl-5-oxo-2-imidazolin-2-yl)-p-toluat, 1,2-Dimethyl-3,5-diphenyl-1H-pyrazoliummethylsulfat, 3,5-Dibrom-4-hydroxybenzonitril, 4-Hydroxy-3,5-diiodobenzonitril, 2,4-Dichlorphenoxyessigsäure, S-(2,3,3-Trichlor-2-propenyl)-bis(1-methylethyl)carbamothioat, 4-Chlor-2-methylphenoxyessigsäure, 3,6-Dichlor-2-pyridincarbonsäure, O-(6-Chlor-3-phenyl-4-pyridazinyl)-S-octylthiocarbonothioat, 3,6-Dichlor-2-methoxybenzoesäure, N'-(3,4-Dichlorphenyl)-N,N-dimethylharnstoff und 4-Amino-3,5,6-trichlor-2-pyridincarbonsäure; und
einem oder mehreren landwirtschaftlichen Hilfsstoffen.

15. Verfahren gemäss Anspruch 7, wobei die Zusammensetzung ausserdem eine herbizid wirksame Menge wenigstens einer Verbindung aus der Gruppe, bestehend aus
N,N-Dimethyl-N'-[3-(trifluormethyl)phenyl]harnstoff, 2-[[4-Chlor-6-(ethylamino)-1,3,5-triazin-2-yl]amino-2-methylpropionitril, N,N'-Bis(1-methylethyl)-6-(methylthio)-1,3,5-triazin-2,4-diamin, N-(3,4-Dichlorphenyl)-propionamid, Methylarsonsäure-Dinatriumsalz, Methylarsonsäure-Mononatriumsalz, 2-Chlor-1-(3-ethoxy-4-nitrophenoxy)-4-trifluormethyl)benzol, O,O-Bis(1-methylethyl)-S-[2-[(phenylsulfonyl)amino]ethyl]-phosphordithioat, 2-(3,4-Dichlorphenyl)-4-methyl-1,2,4-oxadiazolidin-3,5-dion, 4-Chlor-5-)methylamino)-2-[3-(trifluormethyl)phenyl]-3(2H)-pyridazinon, 2-Chlor-6-(4,6-dimethoxypyrimidin-2-ylthio)benzoat, Ethyl-2-chlor-6-(4,6-dimethoxypyrimidin-2-ylthio)benzoat und Natrium-2-chlor-6-(4,6-dimethoxypyrimidin-2-ylthio)benzoat; und
einen oder mehrere landwirtschaftliche Hilfsstoffe enthält.

**16.** Unkrautbekämpfungsverfahren, umfassend das Aufbringen auf Pflanzen von einer herbizid wirksamen Menge einer substituierten Pyridinsulfonamidverbindung oder deren Salz mit der allgemeinen Formel (I) gemäss Anspruch 1.

## Revendications

**Revendications pour les Etats contractants suivants : AT, BE, CH, DE, DK, FR, GB, GR, IT, LI, LU, NL, MC, PT, SE**

**1.** Un composé pyridine sulfonamide substitué ou son sel représenté par la formule générale suivante (I) :

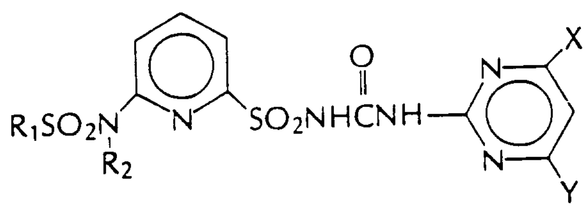

(I)

dans laquelle $R_1$ et $R_2$ peuvent être chacun soit indépendamment un élément choisi dans le groupe constitué de groupes $C_1$-$C_6$ alkyles éventuellement substitués par un ou plusieurs substituants choisis dans le groupe constitué d'halogène et de $C_1$-$C_6$ alcoxy ; de groupes $C_2$-$C_6$ alcényles éventuellement substitués par un ou plusieurs substituants choisis dans le groupe constitué d'halogène et de $C_1$-$C_6$ alcoxy, de groupes $C_3$-$C_6$ cycloalkyles éventuellement substitués par un ou plusieurs substituants choisis dans le groupe constitué d'halogène, de $C_1$-$C_6$ alkyles et de $C_1$-$C_6$ alcoxy ; et de groupes phényles éventuellement substitués par un ou plusieurs substituants choisis dans le groupe constitué d'halogène, de $C_1$-$C_6$ alkyles, de $C_1$-$C_6$ haloalkyles et d'un groupe nitro ; ou bien $R_1$ et $R_2$ sont éventuellement combinés l'un à l'autre pour former un groupe $-(CH_2)_n-$ dans lequel n est un nombre entier de 2 à 5 ; et X et Y désirent chacun indépendamment un élément choisi dans le groupe constitué de $C_1$-$C_6$ alkyles et $C_1$-$C_6$ alcoxy.

**2.** Un composé selon la revendication 1, selon laquelle $R_1$ et $R_2$ désirent chacun indépendamment un élément choisi dans le groupe constitué de groupes $C_1$-$C_6$ alkyles éventuellement substitués par un ou plusieurs substituants choisis dans le groupe constitué d'halogène et de $C_1$-$C_6$ alcoxy ; et de groupes $C_1$-$C_6$ cycloalkyles éventuellement substitués par un ou plusieurs substituants choisis dans le groupe constitué d'halogène, de $C_1$-$C_6$ alkyles et de $C_1$-$C_6$ alcoxy.

**3.** Un composé selon la revendication 1, selon laquelle $R_1$ et $R_2$ désirent chacun indépendamment un élément choisi dans le groupe constitué de groupes $C_1$-$C_6$ alkyles, $C_1$-$C_6$ haloalkyles et $C_3$-$C_6$ cycloalkyles.

**4.** 6-[(N-éthyl-N-méthylsulfonyl)amino]-N-[[-4,6-diméthoxypyrimidine-2-yl)amino]carbonyl]-2-pyridinesulfonamide.

**5.** 6-[(N-éthyl-N-éthylsulfonyl)amino]-N-[[(4,6-diméthoxypyrimidine-2-yl)amino]carbonyl]-2-pyridinesulfonamide.

**6.** 6-[(N-éthyl-N-isopropylsulfonyl)amino]-N-[[(4,6-diméthoxypyrimidine-2-yl)amino]carbonyl]-2-pyridinesulfonamide.

**7.** Un herbicide contenant comme ingrédient efficace un composé pyridinesulfonamide substitué ou son sel représenté par la formule générale suivante (I):

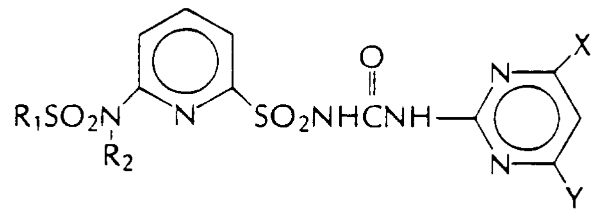

(I)

dans laquelle $R_1$ et $R_2$ peuvent être chacun soit indépendamment un élément choisi dans le groupe constitué de groupes $C_1$-$C_6$ alkyles éventuellement substitués par un ou plusieurs substituants choisis dans le groupe constitué d'halogènes et de $C_1$-$C_6$ alcoxy ; de groupes $C_2$-$C_6$ alcényles éventuellement substitués par un ou plusieurs substituants choisis dans le groupe constitué d'halogènes et de $C_1$-$C_6$ alcoxy ; de groupes $C_3$-$C_6$ cycloalkyles éventuellement substitués par un ou plusieurs substituants choisis dans le groupe constitué d'halogènes, de $C_1$-$C_6$ alkyles et de $C_1$-$C_6$ alcoxy ; et de groupes phényles éventuellement substitués par un ou plusieurs substituants choisis dans le groupe constitué d'halogènes, de $C_1$-$C_6$ alkyles, de $C_1$-$C_6$ haloalkyles et d'un groupe nitro ; soit $R_1$ et $R_2$ sont éventuellement combinés l'un à l'autre pour former un groupe $-(CH_2)_n-$ dans lequel n est un nombre entier de 2 à 5 ; et X et Y désignent chacun indépendamment un élément choisi dans le coupe constitué de $C_1$-$C_6$ alkyles et de $C_1$-$C_6$ alcoxy.

**8.** L'herbicide selon la revendication 7, selon laquelle $R_1$ et $R_2$ désirent chacun indépendamment un élément choisi dans le coupe constitué de groupes $C_1$-$C_6$ alkyles éventuellement substitués par un ou plusieurs substituants choisis dans le coupe constitué d'halogènes et de $C_1$-$C_6$ alcoxy ; et de groupes $C_3$-$C_6$ cycloalkyles éventuellement substitués par un ou plusieurs substituants choisis dans le coupe constitué d'halogènes, de $C_1$-$C_6$ alkyles et de $C_1$-$C_6$ alcoxy.

**9.** L'herbicide selon la revendication 7, selon laquelle $R_1$ et $R_2$ désirent chacun indépendamment un élément choisi dans le coupe constitué de groupes $C_1$-$C_6$ alkyles, $C_1$-$C_6$ haloalkyles et $C_3$-$C_6$ cycloalkyles.

**10.** L'herbicide selon la revendication 7, selon laquelle l'ingrédient efficace est le 6-[(N-éthyl-N-méthylsulfonyl)amino]-N-[[(4,6-diméthoxypyrimidine-2-yl)amino]carbonyl]-2-pyridinesulfonamide.

**11.** L'herbicide selon la revendication 7, selon laquelle ledit ingrédient efficace est le 6-[(N-éthyl-N-éthylsulfonyl)amino]-N-[[(4,6-diméthoxypyrimidine-2-yl)amino]carbonyl]-2-pyridinesulfonamide.

**12.** L'herbicide selon la revendication 7, selon laquelle ledit ingrédient actif est le 6-[(N-éthyl-N-isopropylsulfonyl)amino]-[[(4,6-diméthoxypyrimidine-2-yl)amino]carbonyl]-2-pyridinesulfonamide.

**13.** Une composition herbicide qui comprend :

une quantité herbicide efficace d'au moins un composé choisi dans le groupe constitué de composés pyridinesulfonamides substitués ou leurs sels représentés par la formule générale suivante (I):

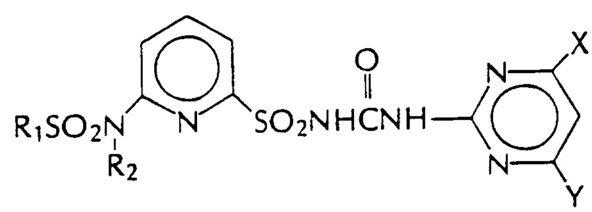

(I)

dans laquelle $R_1$ et $R_2$ peuvent être chacun soit indépendamment un élément choisi dans le groupe constitué de groupes $C_1$-$C_6$ alkyles éventuellement substitués par un ou plusieurs substituants choisis dans le groupe constitué d'halogènes et de $C_1$-$C_6$ alcoxy ; de groupes $C_2$-$C_6$ alcényles éventuellement substitués par un ou plusieurs substituants choisis dans le groupe constitué d'halogènes et de $C_1$-$C_6$

alcoxy ; de groupes $C_3$-$C_6$ cycloalkyles éventuellement substitués par un ou plusieurs substituants choisis dans le groupe constitué d'halogènes, de $C_1$-$C_6$ alkyles et de $C_1$-$C_6$ alcoxy ; et de groupes phényles éventuellement substitués par un ou plusieurs substituants choisis dans le groupe constitué d'halogènes, de $C_1$-$C_6$ alkyles, de $C_1$-$C_6$ haloalkyles et d'un groupe nitro ; soit $R_1$ et $R_2$ sont éventuellement combinés l'un à l'autre pour former un groupe $-(CH_2)_n-$ dans lequel n est un nombre entier de 2 à 5 ; et X et Y désignent chacun indépendamment un élément choisi dans le groupe constitué de $C_1$-$C_6$ alkyles et de $C_1$-$C_6$ alcoxy ;

les quantités herbicides efficaces d'au moins un composé choisi dans le groupe constitué de composé suivants :

(±)-2-[4-[(6-chloro-2-quinoxalinyl)oxy]phénoxy]propionate d'éthyle, (±)-2-[4-[(6-chloro-2-benzoxazolyl)oxy]phénoxy]propionate d'éthyle, (±)-2-[4-[[5-(trifluorométhyl)-2-pyridinyl)oxy]phénoxy]propionate de butyle, 2-[4-[[3-chloro-5-(trifluorométhyl)-2-pyridinyl]oxy]phénoxy]propionate de méthyle, 2-[4-[[3-chloro-5-(trifluorométhyl)-2-pyridinyl]oxy]phénoxy]propionate de 2-éthoxyéthyle, 2-[4-[6-chloro-2-quinoxaline)-oxy]phénoxy]propanoate de (R)-2-[[1-méthyléthylidène)amino]oxy]éthyle, 5-[2-chloro-4-(trifluorométhyl)-phénoxy]-2-nitrobenzoate de sodium, (±)-2-éthoxy-1-méthyl-2-oxoéthyl-5-[2-chloro-4-(trifluorométhyl)-phénoxy]-2-nitrobenzoate, 5-[2-chloro-4-(trifluorométhyl)phénoxy]-N-(méthylsulfonyl)-2-nitrobenzamide, 2-chloro-N-(2-éthyl-6-méthylphényl)-N-(2-méthoxy-1-méthyléthyl)acétamide, 2-chloro-N-(2,6-diéthylphényl)-N-(méthoxyméthyl)acétamide, acide (±)-2-[4,5-dihydro-4-méthyl-4-(1-méthyléthyl)-5-oxo-1H-imidazole-2-yl]-5-éthyl-3-pyridinecarboxylique,acide2-[4,5-dihydro-4-méthyl-4-(1-méthyléthyl)-5-oxo-1H-imidazole-2-yl]-3-quinoléinecar boxylique, 2,6-dinitro-N,N-dipropyl-4-(trifluorométhyl)aniline, N-(1-éthylpropyl)-3,4-diméthyl-2,6-dinitroaniline, (3-méthylphényl)carbamate de 3-[(méthoxycarbonyl)amino]phényle, [3-[-[phénylamino)carbonyl]oxy]phényl]carbamate d'éthyle, 2-[1-(éthoxyimino)butyl]-5-[2-(éthylthio)propyl]-3-hydroxy-2-cyclohexène-1-one, 3-(1-méthyléthyl)-(1H)-2,1,3-benzothiadiazine-4(3H)-one-2,2-dioxyde, 2-[-[[[(4-chloro-6-méthoxy-2-pyrimidinyl)amino]carbonyl]amino]sulfonyl]benzoate d'éthyle, acide 4-(2,4-dichlorophénoxy)butanoïque, 3-(3,4-dichlorophényl)-1-méthoxy-1-méthylurée, 4-amino-6-(1,1-diméthyléthyl)-3-(méthylthio)-1,2,4-triazine-5(4H)-one, acide 7-oxabicyclo[2.2.1]heptane-2,3-dicarboxylique, dipropylcarbamothioate de S-éthyle, méthanesulfonate de (±)-2-éthoxy-2,3-dihydro-3,3-diméthyl-5-benzofuranyle et 5-amino-4-chloro-2-phényl-3(2H)-pyridazinone ;

et un ou plusieurs adjuvants d'agriculture.

**14.** Une composition herbicide qui comprend :

une quantité herbicide efficace d'au moins un composé choisi dans le groupe constitué de composés pyridinesulfonamides substitués ou leurs sels représentés par la formule générale (I) :

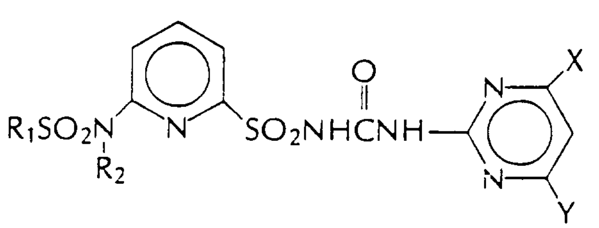

(I)

dans laquelle $R_1$ et $R_2$ peuvent être chacun soit indépendamment un élément choisi dans le groupe constitué de groupes $C_1$-$C_6$ alkyles éventuellement substitués par un ou plusieurs substituants choisis dans le groupe constitué d'halogènes et de $C_1$-$C_6$ alcoxy ; de groupes $C_2$-$C_6$ alcényles éventuellement substitués par un ou plusieurs substituants choisis dans le groupe constitué d'halogènes et de $C_1$-$C_6$ alcoxy ; de groupes $C_3$-$C_6$ cycloalkyles éventuellement substitués par un ou plusieurs substituants choisis dans le groupe constitué d'halogènes, de $C_1$-$C_6$ alkyles et de $C_1$-$C_6$ alcoxy ; et de groupes phényles éventuellement substitués par un ou plusieurs substituants choisis dans le groupe constitué d'halogènes, de $C_1$-$C_6$ alkyles, de $C_1$-$C_6$ haloalkyles et d'un groupe nitro ; soit $R_1$ et $R_2$ sont éventuellement combinés l'un à l'autre pour former un groupe $-(CH_2)_n-$ dans lequel n est un nombre entier de 2 à 5 ; et X et Y désignent chacun indépendamment un élément choisi dans le groupe constitué de $C_1$-$C_6$ alkyles et de $C_1$-$C_6$ alcoxy ;

une quantité herbicide efficace d'au moins un composé choisi dans le groupe constitué de composés suivants :

2-[[[[(4-méthoxy-6-méthyl-1,3,5-triazine-2-yl)amino]carbonyl]amino]sulfonyl]benzoate de méthyle, 2-[[[[N-(4-méthoxy-6-méthyl-1,3,5-triazine-2-yl)méthylamino]carbonyl]amino]sulfonyl]benzoate de méthyle, 2-chloro-N-[[(4-méthoxy-6-méthyl-1,3,5-trizaine-2-yl]-amino]carbonyl]benzènesulfonamide, 3-[[[[(4-méthoxy-6-méthyl-1,3,5-triazine-2-yl]amino]carbonyl]amino]sulfonyl]-2-thiophènecarboxylate de méthyle, (±)-2-[4-(2,4-dichlorophénoxy)phénoxy]propionate de méthyle, 6-(4-isopropyl-4-méthyl-5-oxo-2-imidazoline-2-yl)-m-toluate de méthyle, 2-(4-isopropyl-4-méthyl-5-oxo-2-imidazoline-2-yl)-p-toluate de méthyle, méthylsulfate de 1,2-diméthyl-3,5-diphényl-1H-pyrazolium, 3,5-dibromo-4-hydroxybenzonitrile, 4-hydroxy-3,5-diiodobenzonitrile, acide 2,4-dichlorophénoxyacétique, S-(2,3,3-trichloro-2-propényl)-bis(1-méthyléthyl)-carbamothioate, acide 4-chloro-2-méthylphénoxyacétique, acide 3,6-dichloro-2-pyridinecarboxylique, thiocarbonothioate de O-(6-chloro-3-phényl-4-pyridazinyl)-S-octyle, acide 3,6-dichloro-2-méthoxybenzoïque, N'-(3,4-dichlorophényl)-N,N-diméthylurée et acide 4-amino-3,5,6-trichloro-2-pyridinecarboxylique ; et un ou plusieurs adjuvants d'agriculture.

**15.** Une composition herbicide qui comprend :

une quantité herbicide efficace d'au moins un composé choisi dans le groupe constitué de composés pyridinesulfonamides substitués ou leurs sels représentés par la formule générale suivante (I) :

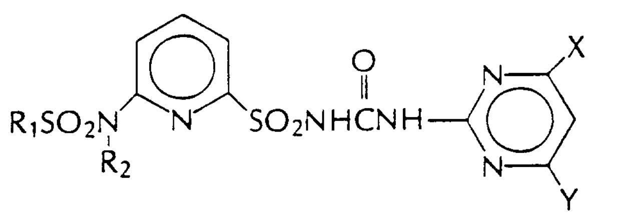

(I)

dans laquelle $R_1$ et $R_2$ peuvent être chacun soit indépendamment un élément choisi dans le groupe constitué de groupes $C_1$-$C_6$ alkyles éventuellement substitués par un ou plusieurs substituants choisis dans le groupe constitué d'halogènes et de $C_1$-$C_6$ alcoxy ; de groupes $C_2$-$C_6$ alcényles éventuellement substitués par un ou plusieurs substituants choisis dans le groupe constitué d'halogènes et de $C_1$-$C_6$ alcoxy ; de groupes $C_3$-$C_6$ cycloalkyles éventuellement substitués par un ou plusieurs substituants choisis dans le groupe constitué d'halogènes, de $C_1$-$C_6$ alkyles et de $C_1$-$C_6$ alcoxy ; et de groupes phényles éventuellement substitués par un ou plusieurs substituants choisis dans le groupe constitué d'halogènes, de $C_1$-$C_6$ alkyles, de $C_1$-$C_6$ haloalkyles et d'un groupe nitro ; soit $R_1$ et $R_2$ sont éventuellement combinés l'un à l'autre pour former un groupe $-(CH_2)_n-$ dans lequel n est un nombre entier de 2 à 5 ; et X et Y désignent chacun indépendamment un élément choisi dans le groupe constitué de $C_1$-$C_6$ alkyles et de $C_1$-$C_6$ alcoxy ;

une quantité herbicide efficace d'au moins un composé choisi dans le groupe constitué de composés suivants :

N,N-diméthyl N'-[3-triflurométhyl)phényl]urée, 2-[[4-chloro-6-(éthylamino)-1,3,5-triazine-2-yl]amino]-2-méthylpropionitrile, N,N'-bis(1-méthyléthyl)-6-(méthylthio)-1,3,5-triazine-2,4-diamine, N-(3,4-dichlorophényl)propanamide, sel disodique de l'acide méthylarsonique, sel monosodique de l'acide méthylarsonique, 2-chloro-1-(3-éthoxy-4-nitrophénoxy)-4-(trifluorométhyl)benzène, O,O-bis(1-méthyléthyl) S-[2-[-(phénylsulfonyl)amino]éthyl]phosphorodithioate, 2-(3,4-dichlorophényl)-4-méthyl-1,2,4-oxadiazolidine-3,5-dione, 4-chloro-5-(méthylamino)-2-[3-(trifluoro méthyl)-phényl]-3(2H)-pyridazinone, 2-choro-6-(4,6-diméthoxypyrimidine-2-ylthio)benzoate, 2-chloro-6-(4,6-diméthoxypyrimidine-2-yltho)-benzoate d'éthyle et 2-chloro-6-(4,6-diméthoxypyrimidine-2-ylthio)benzoate de sodium et un ou plusieurs adjuvants d'agriculture.

**16.** Une méthode herbicide qui consiste à appliquer à des plantes une quantité herbicide efficace d'un composé pyridinesulfonamide substitué ou son sel représenté par la formule générale suivante (I):

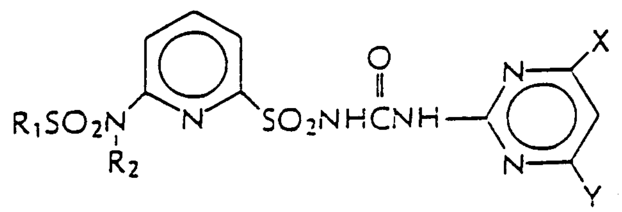

(I)

dans laquelle $R_1$ et $R_2$ peuvent être chacun soit indépendamment un élément choisi dans le groupe constitué de groupes $C_1$-$C_6$ alkyles éventuellement substitués par un ou plusieurs substituants choisis dans le groupe constitué d'halogènes et de $C_1$-$C_6$ alcoxy ; de groupes $C_2$-$C_6$ alcényles éventuellement substitués par un ou plusieurs substituants choisis dans le groupe constitué d'halogènes et de $C_1$-$C_6$ alcoxy ; de groupes $C_3$-$C_6$ cycloalkyles éventuellement substitués par un ou plusieurs substituants choisis dans le groupe constitué d'halogènes, de $C_1$-$C_6$ alkyles et de $C_1$-$C_6$ alcoxy ; et de groupes phényles éventuellement substitués par un ou plusieurs substituants choisis dans le groupe constitué d'halogènes, de $C_1$-$C_6$ alkyles, de $C_1$-$C_6$ haloalkyles et d'un groupe nitro ; soit $R_1$ et $R_2$ sont éventuellement combinés l'un à l'autre pour former un groupe $-(CH_2)_n-$ dans lequel n est un nombre entier de 2 à 5 ; et X et Y désignent chacun indépendamment un élément choisi dans le groupe constitué de $C_1$-$C_6$ alkyles et de $C_1$-$C_6$ alcoxy.

**17.** Un procédé de préparation d'un composé pyridinesulfonamide substitué ou de son sel représenté par la formule générale suivante (I) :

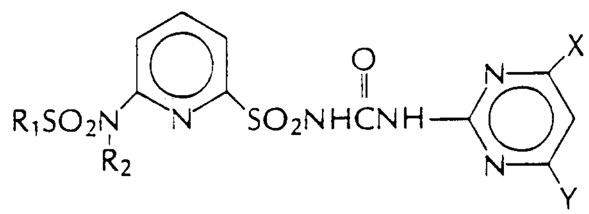

(I)

dans laquelle $R_1$ et $R_2$ peuvent être chacun soit indépendamment un élément choisi dans le groupe constitué de groupes $C_1$-$C_6$ alkyles éventuellement substitués par un ou plusieurs substituants choisis dans le groupe constitué d'halogènes et de $C_1$-$C_6$ alcoxy ; de groupes $C_2$-$C_6$ alcényles éventuellement substitués par un ou plusieurs substituants choisis dans le groupe constitué d'halogènes et de $C_1$-$C_6$ alcoxy ; de groupes $C_3$-$C_6$ cycloalkyles éventuellement substitués par un ou plusieurs substituants choisis dans le groupe constitué d'halogènes, de $C_1$-$C_6$ alkyles et de $C_1$-$C_6$ alcoxy ; et de groupes phényles éventuellement substitués par un ou plusieurs substituants choisis dans le groupe constitué d'halogènes, de $C_1$-$C_6$ alkyles, de $C_1$-$C_6$ haloalkyles et d'un groupe nitro ; soit $R_1$ et $R_2$ sont éventuellement combinés l'un à l'autre pour former un groupe $-(CH_2)_n-$ dans lequel n est un nombre entier de 2 à 5 ; et X et Y désignent chacun indépendamment un élément choisi dans le groupe constitué de $C_1$-$C_6$ alkyles et de $C_1$-$C_6$ alcoxy,

qui comprend la réaction d'un composé pyridine substitué représenté par la formule générale suivante (II) :

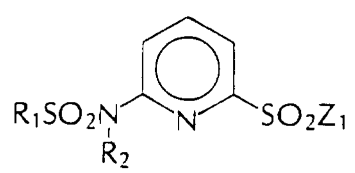

(II)

dans laquelle $R_1$ et $R_2$ sont comme définis précédemment ; et $Z_1$ est un élément choisi dans le groupe constitué d'un groupe $-NH_2$, d'un groupe -NCO, et d'un groupe $-NHCO_2R_3$ où $R_3$ est un groupe alkyle

ou aryle ; avec un composé pyrimidine représenté par la formule générale suivante (III) :

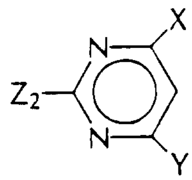

(III)

dans laquelle X et Y sont comme définis précédemment ; et $Z_2$ est un groupe $-NH_2$ lorsque $Z_1$ est un groupe -NCO ou un groupe $-NHCO_2R_3$, et est un élément choisi dans le groupe constitué d'un groupe -NCO et d'un groupe $-NHCO_2R_3$ où $R_3$ est comme défini précédemment, lorsque $Z_1$ est un groupe $-NH_2$.

**18.** Un composé pyridine substitué représenté par la formule générale suivante (II-1) :

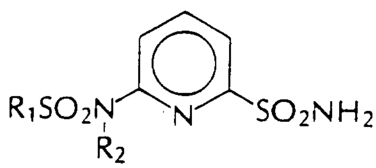

(II-1)

dans laquelle $R_1$ et $R_2$ désignent chacun indépendamment un élément choisi dans le groupe constitué de groupes $C_1$-$C_6$ alkyles éventuellement substitués par un ou plusieurs substituants choisis dans le groupe constitué d'halogènes et de $C_1$-$C_6$ alcoxy ; de groupes $C_2$-$C_6$ alcényles éventuellement substitués par un ou plusieurs substituants choisis dans le groupe constitué d'halogènes et de $C_1$-$C_6$ alcoxy ; de groupes $C_3$-$C_6$ cycloalkyles éventuellement substitués par un ou plusieurs substituants choisis dans le groupe constitué d'halogènes, de $C_1$-$C_6$ alkyles et de $C_1$-$C_6$ alcoxy ; et de groupes phényles éventuellement substitués par un ou plusieurs substituants choisis dans le groupe constitué d'halogènes, de $C_1$-$C_6$ alkyles, de $C_1$-$C_6$ haloalkyles et d'un groupe nitro ; soit $R_1$ et $R_2$ sont éventuellement combinés l'un à l'autre pour former un groupe $-(CH_2)_n-$ dans lequel n est un nombre entier de 2 à 5.

**Revendications pour l'Etat contractant suivant : ES**

**1.** Un procédé de préparation d'un composé pyridinesulfonamide substitué ou de son sel représenté par la formule générale suivante (I) :

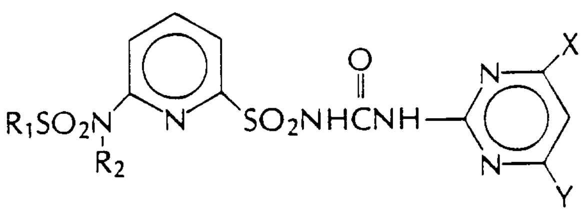

(I)

dans laquelle $R_1$ et $R_2$ peuvent être chacun soit indépendamment un élément choisi dans le groupe constitué de groupes $C_1$-$C_6$ alkyles éventuellement substitués par un ou plusieurs substituants choisis dans le groupe constitué d'halogènes et de $C_1$-$C_6$ alcoxy ; de groupes $C_2$-$C_6$ alcényles éventuellement substitués par un ou plusieurs substituants choisis dans le groupe constitué d'halogènes et de $C_1$-$C_6$ alcoxy ; de groupes $C_3$-$C_6$ cycloalkyles éventuellement substitués par un ou plusieurs substituants choisis dans le groupe constitué d'halogènes, de $C_1$-$C_6$ alkyles et de $C_1$-$C_6$ alcoxy ; et de groupes phényles éventuellement substitués par un ou plusieurs substituants choisis dans le groupe constitué d'halogènes, de $C_1$-$C_6$ alkyles, de $C_1$-$C_6$ haloalkyles et d'un groupe nitro ; soit $R_1$ et $R_2$ sont

éventuellement combinés l'un à l'autre pour former un groupe - $(CH_2)_n$- dans lequel n est un nombre entier de 2 à 5 ; et X et Y désignent chacun indépendamment un élément choisi dans le groupe constitué de $C_1$-$C_6$ alkyles et de $C_1$-$C_6$ alcoxy,

qui comprend la réaction d'un composé pyridine substitué représenté par la formule générale suivante (II) :

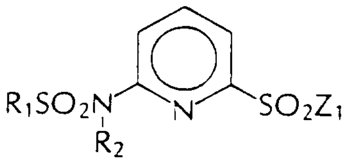

(II)

dans laquelle $R_1$ et $R_2$ sont comme définis précédemment ; et $Z_1$ est un élément choisi dans le groupe constitué d'un groupe -$NH_2$, d'un groupe -NCO, et d'un groupe -$NHCO_2R_3$ où $R_3$ est un groupe alkyle ou aryle ; avec un composé pyrimidine représenté par la formule générale suivante (III) :

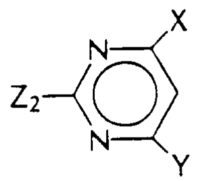

(III)

dans laquelle X et Y sont comme définis précédemment ; et $Z_2$ est un groupe -$NH_2$ lorsque $Z_1$ est un groupe -NCO ou un groupe -$NHCO_2R_3$, et est un élément choisi dans le groupe constitué d'un groupe -NCO et d'un groupe -$NHCO_2R_3$ où $R_3$ est comme défini précédemment, lorsque $Z_1$ est un groupe -$NH_2$.

**2.** Un procédé selon la revendication 1, selon laquelle $R_1$ et $R_2$ désignent chacun indépendamment un élément choisi dans le groupe constitué de groupes $C_1$-$C_6$ alkyles éventuellement substitués par un ou plusieurs substituants choisis dans le groupe constitué d'halogènes et de $C_1$-$C_6$ alcoxy ; et de groupes $C_1$-$C_6$ cycloalkyles éventuellement substitués par un ou plusieurs substituants choisis dans le groupe constitué d'halogènes, de $C_1$-$C_6$ alkyles et de $C_1$-$C_6$ alcoxy.

**3.** Un procédé selon la revendication 1, selon laquelle $R_1$ et $R_2$ désignent chacun indépendamment un élément choisi dans le groupe constitué de groupes $C_1$-$C_6$ alkyles, $C_1$-$C_6$ haloalkyles et $C_3$-$C_6$ cycloalkyles.

**4.** Un procédé selon la revendication 1, selon laquelle le composé de formule (I) est le 6-[(N-éthyl-N-méthylsulfonyl)amino]-N-[[-4,6-diméthoxypyrimidine-2-yl)amino]carbonyl]-2-pyridinesulfonamide.

**5.** Un procédé selon la revendication 1, selon laquelle le composé de formule (I) est le 6-[(N-éthyl-N-éthylsulfonyl)amino]-N-[[(4,6-diméthoxypyrimidine-2-yl)amino]carbonyl]-2-pyridinesulfonamide.

**6.** Un procédé selon la revendication 1, selon laquelle le composé de formule (I) est le 6-[(N-éthyl-N-isopropylsulfonyl)amino]-N-[[(4,6-diméthoxypyrimidine-2-yl)amino]carbonyl]-2-pyridinesulfonamide.

**7.** Une méthode de production d'une composition herbicide consistant à mélanger un composé pyridinesulfonamide substitué ou son sel représenté par la formule générale (I) tel que défini dans la revendication 1, comme ingrédient actif, avec un ou plusieurs adjuvants en agriculture.

**8.** Une méthode selon la revendication 7, selon laquelle $R_1$ et $R_2$ dans la formule (I) désignent chacun indépendamment un élément choisi dans le groupe constitué de groupes $C_1$-$C_6$ alkyles éventuellement substitués par un ou plusieurs substituants choisis dans le groupe constitué d'halogènes et de $C_1$-$C_6$ alcoxy ; et de groupes $C_3$-$C_6$ cycloalkyles éventuellement substitués par un ou plusieurs substiuants

choisis dans le groupe constitué d'halogènes, de $C_1$-$C_6$ alkyles et de $C_1$-$C_6$ alcoxy.

**9.** Une méthode selon la revendication 7, selon laquelle $R_1$ et $R_2$ désignent chacun indépendamment un élément choisi dans le groupe constitué de groupes $C_1$-$C_6$ alkyles, $C_1$-$C_6$ haloalkyles et $C_3$-$C_6$ cycloalkyles.

**10.** Une méthode selon la revendication 7, selon laquelle ledit ingrédient actif est le 6-[(N-éthyl-N-méthylsulfonyl)amino]-N-[[(4,6-diméthoxypyrimidine-2-yl)amino]carbonyl]-2-pyridinesulfonamide.

**11.** Une méthode selon la revendication 7, selon laquelle ledit ingrédient efficace est le 6-[(N-éthyl-N-éthylsulfonyl)amino]-N-[[(4,6-diméthoxypyrimidine-2-yl)amino]carbonyl]-2-pyridinesulfonamide.

**12.** Une méthode selon la revendication 7, selon laquelle ledit ingrédient efficace est le 6-[(N-éthyl-N-isopropylsulfonyl)amino]-N-[[(4,6-diméthoxypyrimidine-2-yl)amino]carbonyl]-2-pyridinesulfonamide.

**13.** Une méthode selon la revendication 7, selon laquelle la composition herbicide comprend encore une quantité herbicide efficace d'au moins un composé choisi dans le groupe constitué de composés suivants :

(±)-2-[4-[(6-chloro-2-quinoxalinyl)oxy]phénoxy]propionate d'éthyle, (±)-2-[4-[(6-chloro-2-benzoxazolyl)oxy]phénoxy]propionate d'éthyle, (±)-2-[4-[[5-(trifluorométhyl)-2-pyridinyl)oxy]phénoxy]propionate de butyle, 2-[4-[[3-chloro-5-(trifluorométhyl)-2-pyridinyl]oxy]phénoxy]propionate de méthyle, 2-[4-[[3-chloro-5-(trifluorométhyl)-2-pyridinyl]oxy]phénoxy]propionate de 2-éthoxyéthyle, 2-[4-[6-chloro-2-quinoxaline)-oxy]phénoxy]propanoate de (R)-2-[[1-méthyléthylidène)amino]oxy]éthyle, 5-[2-chloro-4-(trifluorométhyl)-phénoxy]-2-nitrobenzoate de sodium, (±)-2-éthoxy-1-méthyl-2-oxoéthyl-5-[2-chloro-4-(trifluorométhyl)-phénoxy]-2-nitrobenzoate, 5-[2-chloro-4-(trifluorométhyl)phénoxy]-N-(méthylsulfonyl)-2-nitrobenzamide, 2-chloro-N-(2-éthyl-6-méthylphényl)-N-(2-méthoxy-1-méthyléthyl)acétamide, 2-chloro-N-(2,6-diéthylphényl)-N-(méthoxyméthyl)acétamide, acide (±)-2-[4,5-dihydro-4-méthyl-4-(1-méthyléthyl)-5-oxo-1H-imidazole-2-yl]-5-éthyl-3-pyridinecarboxylique,acide2-[4,5-dihydro-4-méthyl-4-(1-méthyléthyl)-5-oxo-1H-imidazole-2-yl]-3-quinoléinecar boxylique, 2,6-dinitro-N,N-dipropyl-4-(trifluorométhyl)aniline, N-(1-éthylpropyl)-3,4-diméthyl-2,6-dinitroaniline, (3-méthylphényl)carbamate de 3-[(méthoxycarbonyl)amino]phényle, [3-[-[phénylamino)carbonyl]oxy]phényl]carbamate d'éthyle, 2-[1-(éthoxyimino)butyl]-5-[2-(éthylthio)propyl]-3-hydroxy-2-cyclohexène-1-one, 3-(1-méthyléthyl)-(1H)-2,1,3-benzothiadiazine-4(3H)-one-2,2-dioxyde, 2-[-[[[(4-chloro-6-méthoxy-2-pyrimidinyl)amino]carbonyl]amino]sulfonyl]benzoate d'éthyle, acide 4-(2,4-dichlorophénoxy)butanoïque, 3-(3,4-dichlorophényl)-1-méthoxy-1-méthylurée, 4-amino-6-(1,1-diméthyléthyl)-3-(méthylthio)-1,2,4-triazine-5(4H)-one, acide 7-oxabicyclo[2.2.1]heptane-2,3-dicarboxylique, dipropylcarbamothioate de S-éthyle, méthanesulfonate de (±)-2-éthoxy-2,3-dihydro-3,3-diméthyl-5-benzofuranyle et 5-amino-4-chloro-2-phényl-3(2H)-pyridazinone.

**14.** Une méthode de production d'une composition herbicide qui consiste à mélanger une quantité herbicide efficace d'au moins un composé choisi dans le groupe constitué de composés pyridinesulfonamides substitués ou leurs sels représentés par la formule générale suivante (I) telle que défini dans la revendication 1 avec un ou plusieurs adjuvants d'agriculture et

une quantité herbicide efficace d'au moins un composé choisi dans le groupe constitué de composés suivants :

2-[[[[(4-méthoxy-6-méthyl-1,3,5-triazine-2-yl)-amino]carbonyl]amino]sulfonyl]benzoate de méthyle, 2-[[[[N-(4-méthoxy-6-méthyl-1,3,5-triazine-2-yl)méthylamino]carbonyl]amino]sulfonyl]benzoate de méthyle, 2-chloro-N-[[(4-méthoxy-6-méthyl-1,3,5-triazine-2-yl]-amino]carbonyl]benzènesulfonamide, 3-[[[[(4-méthoxy-6-méthyl-1,3,5-triazine-2-yl]amino]carbonyl]amino]sulfonyl]-2-thiophènecarboxylate de méthyle, (±)-2-[4-(2,4-dichlorophénoxy)phénoxy]propionate de méthyle, 6-(4-isopropyl-4-méthyl-5-oxo-2-imidazoline-2-yl)-m-toluate de méthyle, 2-(4-isopropyl-4-méthyl-5-oxo-2-imidazoline-2-yl)-p-toluate de méthyle, méthylsulfate de 1,2-diméthyl-3,5-diphényl-1H-pyrazolium, 3,5-dibromo-4-hydroxybenzonitrile, 4-hydroxy-3,5-diiodobenzonitrile, acide 2,4-dichlorophénoxyacétique, S-(2,3,3-trichloro-2-propényl)-bis(1-méthyléthyl)-carbamothioate, acide 4-chloro-2-méthylphénoxyacétique, acide 3,6-dichloro-2-pyridinecarboxylique, thiocarbonothioate de O-(6-chloro-3-phényl-4-pyridazinyl)-S-octyle, acide 3,6-dichloro-2-méthoxybenzoïque, N'-(3,4-dichlorophényl)-N,N-diméthylurée et acide 4-amino-3,5,6-trichloro-2-pyridinecarboxylique ; et

un ou plusieurs adjuvants d'agriculture.

**15.** Une méthode selon la revendication 7, selon laquelle la composition contient également une quantité herbicide efficace d'au moins un composé choisi dans le groupe constitué de composés suivants :
N,N-diméthyl N'-[3-trifluorométhyl)phényl]urée, 2-[[4-chloro-6-(éthylamino)-1,3,5-triazine-2-yl]-amino]-2-méthylpropionitrile, N,N'-bis(1-méthyléthyl)-6-(méthylthio)-1,3,5-triazine-2,4-diamine, N-(3,4-dichlorophényl)propanamide, sel disodique de l'acide méthylarsonique, sel monosodique de l'acide méthylarsonique, 2-chloro-1-(3-éthoxy-4-nitrophénoxy)-4-(trifluorométhyl)benzène, O,O-bis(1-méthyléthyl) S-[2-[(phénylsulfonyl)amino]éthyl]phosphorodithioate, 2-(3,4-dichlorophényl)-4-méthyl-1,2,4-oxadiazolidine-3,5-dione, 4-chloro-5-(méthylamino)-2-[3-(trifluorométhyl)phényl]-3(2H)-pyridazinone, 2-chloro-6-(4,6-diméthoxypyrimidine-2-ylthio)benzoate, 2-chloro-6-(4,6-diméthoxypyrimidine-2-ylthio)-benzoate d'éthyle et 2-chloro-6-(4,6-diméthoxypyrimidthe-2-ylthio)benzoate de sodium ; et un ou plusieurs adjuvants d'agriculture.

**16.** Une méthode herbicide qui comprend l'application à des plantes d'une quantité herbicide efficace d'un composé pyridinesulfonamide substitué ou de son sel représenté par la formule générale (I) telle que défini dans la revendication 1.